# EUROPEAN PATENT APPLICATION

(11) **EP 4 596 554 A1**
(43) Date of publication of application: **06.08.2025**
(21) Application number: 23870887.9
(22) Date of filing: 27.09.2023
(51) Int. Cl.: C07D 495/04, C07D 519/00, C07D 413/14, A61K 31/573, A61K 31/519, A61K 31/5377, A61P 11/00, A61P 27/02, A61P 25/00, A61P 1/00, A61P 29/00

(54) **AZA-FUSED RING COMPOUND, PREPARATION METHOD THEREFOR, AND USE THEREOF IN MEDICINE**

(30) Priority: 29.09.2022 CN 202211201376
(71) Applicant: Suzhou Arkbiopharmaceutical Co., Ltd., Suzhou, Jiangsu 215000 (CN)
(72) Inventor: PENG, Cheng, Suzhou, Jiangsu 215000 (CN); QIAN, Mengfei, Suzhou, Jiangsu 215000 (CN); LING, Weikang, Suzhou, Jiangsu 215000 (CN); WU, Jin, Suzhou, Jiangsu 215000 (CN); GAO, Fan, Suzhou, Jiangsu 215000 (CN); YUAN, Shuai, Suzhou, Jiangsu 215000 (CN); SONG, Guowei, Suzhou, Jiangsu 215000 (CN); YE, Junmin, Suzhou, Jiangsu 215000 (CN)
(74) Representative: ZBM Patents - Zea, Barlocci & Markvardsen
(86) International application number: PCT/CN2023/121867
(87) International publication number: WO 2024/067660

(57) **Abstract**

The present disclosure relates to an aza-fused ring compound, a preparation method therefor, and a use thereof in medicine. Specifically, the present disclosure relates to an aza-fused ring compound represented by formula (I), a preparation method therefor, a pharmaceutical composition containing said compound, and a use thereof as a phosphodiesterase (PDEs) inhibitor, in particular a use in the preparation of a medicament for the treatment and/or prevention of diseases or conditions mediated by PDE4 enzymes.

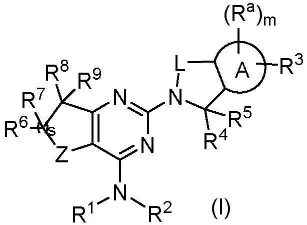

## Description

This disclosure claims the priority of the Chinese patent application No. 202211201376.X, entitled "Aza-fused ring compound, preparation method therefor, and use thereof in medicine" filed to the Chinese Patent Office on September 29, 2022, the entire disclosure of which is incorporated herein by reference in its entirety.

### TECHNICAL FIELD

This disclosure belongs to the field of medicine and relates to aza-fused ring compound, preparation method therefor, and use thereof in medicine. Specifically, this disclosure relates to azacyclic compounds of formula (I), preparation method therefor, pharmaceutical compositions containing the same, and the use thereof as phosphodiesterase (PDE) inhibitors, particularly the use in the preparation of drugs for treating and/or preventing diseases or conditions mediated by PDE4 enzymes.

### BACKGROUND

Phosphodiesterases (PDEs) have the function of hydrolyzing intracellular second messengers (cAMP, cyclic adenosine monophosphate, or cGMP, cyclic guanosine monophosphate) and degrade the intracellular cAMP or cGMP, thereby terminating the biochemical effects transmitted by these second messengers. cAMP and cGMP play important regulatory roles in cellular activities. The regulation on concentrations is primarily determined by the balance between the synthesis of adenylate cyclase and the hydrolytic action of PDEs. PDEs are widely distributed in the human body, and their physiological roles involve multiple research areas.

Among the 11 identified PDEs, PDE4, PDE7, and PDE8 are cAMP-selective. The PDE4 family includes four subtypes (PDE4A, B, C, and D) with more than 20 splice variants, making it one of the largest PDE subfamilies (Bender and Beavo, 2006). These four subtypes are differentially expressed in various tissues and cell types. For example, PDE4B is mainly expressed in monocytes and neutrophils but not in cortical cells and epithelial cells, while PDE4D is expressed in the lungs, cortex, cerebellum, and T cells (C. Kroegel and M. Foerster, Exp. Opinion Investig. Drugs, 16(1), 2007, 109-124). PDE4B primarily hydrolyzes cAMP, with an apparent Km for cAMP substrateof 1-5 µM. PDE4 is the most important regulator of cAMP expressed in immune cells and inflammatory cells such as neutrophils, macrophages, and T-lymphocytes. Since cAMP is a key second messenger in regulations of inflammatory responses, PDE4 has been found to modulate inflammatory responses in inflammatory cells by regulating pro-inflammatory cytokines such as TNFα, IL-2, IFN-y, GM-CSF, and LTB4. Therefore, PDE4 inhibition has become an attractive target for treating inflammatory diseases such as asthma, chronic obstructive pulmonary disease (COPD), rheumatoid arthritis, atopic dermatitis, psoriasis, inflammatory bowel diseases like Crohn's disease, etc. (M.D. Houslay et al., Drug Discovery Today, 2005, 10(22), 1503-1519). Since PDE activity is elevated in patients with atopic dermatitis (AD), PDE4 inhibition also appears to be a viable treatment for AD (Journal of Investigative Dermatology, 1986, 87(3), 372-376).

Relevant patent applications include WO2009053268A1, WO2009050242A2, WO2009050236A1, CN101827852B, CN101163706A, CN101426505A, CN102875556B, CN103497201A, CN103889970B, and CN108299400B, etc.

### SUMMARY

The object of this disclosure is to provide compounds of formula (I) or pharmaceutically acceptable salt thereof: 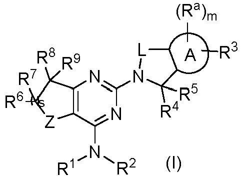 where:
Ring A is selected from heteroaryl, aryl, heterocyclyl, and cycloalkyl;
L is -(CR^{4a} R^{4b} )ₚ(CR^{5a}R⁵⁶ )_{q}-;
Z is selected from -S-, -S(O)-, -S(O)₂-, and -O-;
R¹ and R² are identical or different, and are each independently selected from a hydrogen atom, a deuterium atom, alkyl, alkenyl, alkynyl, cycloalkyl, heterocyclyl, aryl, and heteroaryl; where the alkyl, alkenyl, alkynyl, cycloalkyl, heterocyclyl, aryl, and heteroaryl are each independently optionally substituted by one or more substituents selected from a group consisting of a deuterium atom, alkyl, halogen, oxo, haloalkyl, -OR^{c1} , cyano, -(CH₂)ᵤ₁NR^{d1}R^{d2} , -C(O)NR^{d3}R^{d4} , -S(O)₂NR^{d3}R^{d4} , -(CH₂)ᵥ₁NR^{d5}C(O)R^{e1} , - (CH₂)ᵥ₁NR^{d5}S(O)₂R^{e1}, -C(O)R^{e2}, -OC(O)R^{e3}, -S(O)ᵣR^{e4}, -(CH₂)_{w1}C(O)OR^{c2}, -(CH₂)ᵤR, hydroxyalkyl, cycloalkyl, heterocyclyl, aryl, and heteroaryl;
R³ is selected from a group consisting of a hydrogen atom, a deuterium atom, alkyl, alkenyl, alkynyl, halogen, cyano, alkylcyano, oxo, -OR^{c3}, -(CH₂)ᵤ₂NR^{d6}R^{d7}, -C(O)NR^{d8}R^{d9}, -(CH₂)ᵥ₂NR^{d10}C(O)R^{e5}, -C(O)R^{e6}, -OC(O)R^{e7}, -S(O)ₜR^{e8}, -(CH₂)_{w2}C(O)OR^{c4} , and 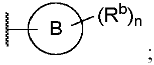 where the alkyl, alkenyl, and alkynyl are each independently optionally substituted by one or more substituents selected from a deuterium atom, halogen, haloalkyl, -OR^{c1},cyano,-(CH₂)ᵤ₁NR^{d1}R^{d2}, hydroxyalkyl, cycloalkyl, heterocyclyl, aryl, and heteroaryl;
Ring B is selected from heteroaryl, aryl, heterocyclyl, and cycloalkyl;
Each R^{b} is identical or different, and is each independently selected from a group consisting of a deuterium atom, halogen, cyano, alkylcyano, oxo, alkyl, haloalkyl, -OR^{c3}, -(CH₂)ᵤ₂NR^{d6}R^{d7}, -C(O)NR^{d8}R^{d9}, -(CH₂)ᵥ₂NR^{d10}C(O)R^{e5}, -C(O)R^{e6}, -OC(O)R^{e7}, -S(O)ₜR^{e8}, -(CH₂)_{w2}C(O)OR^{c4} , hydroxyalkyl, cycloalkyl, heterocyclyl, aryl, and heteroaryl;
R⁴, R^{4a}, R^{4b}, R⁵, R^{5a} and R^{5b} are identical or different, and are each independently selected from a hydrogen atom, a deuterium atom, alkyl, halogen, alkenyl, alkynyl, cyano, alkylcyano, hydroxyalkyl, haloalkyl, -OR^{c3}, -(CH₂)ᵤ₂NR^{d6}R^{d7}, -C(O)NR^{d8}R^{d9}, -(CH₂)ᵥ₂NR^{d10}C(O)R^{e5}, -C(O)R^{e6}, -OC(O)R^{e7}, -S(O)ₜR^{e8}, -(CH₂)_{w2}C(O)OR^{c4} , cycloalkyl, heterocyclyl, aryl, and heteroaryl; or each of R⁴ and R⁵ , R^{4a} and R^{4b} , R^{5a} and R^{5b} together with the carbon atom to which they are attached form an oxo;
R⁶, R⁷, R⁸ and R⁹ are identical or different, and are each independently selected from a hydrogen atom, a deuterium atom, alkyl, alkenyl, alkynyl, halogen, cyano, alkylcyano, haloalkyl, hydroxyalkyl, -OR^{c3}, - (CH₂)ᵤ₂NR^{d6}R^{d7}, cycloalkyl, heterocyclyl, aryl, and heteroaryl; where the alkyl, alkenyl, alkynyl, cycloalkyl, heterocyclyl, aryl, and heteroaryl are each independently optionally substituted by one or more substituents selected from a group consisting of halogen, oxo, alkyl, haloalkyl, -OR^{c1}, cyano, -(CH₂)ᵤ₁NR^{d1}R^{d2}, hydroxyalkyl, cycloalkyl, heterocyclyl, aryl, and heteroaryl;
Each R^{a} is identical or different, and is each independently selected from a deuterium atom, alkyl, halogen, oxo, alkenyl, alkynyl, cyano, alkylcyano, hydroxyalkyl, haloalkyl, -OR^{c3}, -(CH₂)ᵤ₂NR^{d6}R^{d7}, cycloalkyl, heterocyclyl, aryl, and heteroaryl;
R is selected from a group consisting of cycloalkyl, heterocyclyl, aryl, and heteroaryl; where the cycloalkyl, heterocyclyl, aryl, and heteroaryl are each independently optionally substituted by one or more substituents selected from a group consisting of a deuterium atom, halogen, oxo, alkyl, haloalkyl, -OR^{c1}, cyano, alkylcyano, -(CH₂)ᵤ₁NR^{d1}R^{d2}, hydroxyalkyl, cycloalkyl, heterocyclyl, aryl, and heteroaryl;
at each occurrence, R^{c1}, R^{c2}, R^{c3}, and R^{c4} are identical or different, and are each independently selected from a group consisting of a hydrogen atom, a deuterium atom, alkyl, hydroxyalkyl, cycloalkyl, heterocyclyl, aryl, and heteroaryl, where the alkyl, cycloalkyl, heterocyclyl, aryl, and heteroaryl are each independently optionally substituted by one or more substituents selected from a deuterium atom, halogen, oxo, alkyl, haloalkyl, alkoxy, haloalkoxy, cyano, alkylcyano, -(CH₂)ᵤ₁NR^{d1}R^{d2}, hydroxy, hydroxyalkyl, cycloalkyl, heterocyclyl, aryl, and heteroaryl;
at each occurrence, R^{d1}, R^{d2}, R^{d3}, R^{d4}, R^{d5}, R^{d6}, R^{d7}, R^{a8}, R^{d9} and R^{d10} are identical or different, and are each independently selected from a hydrogen atom, a deuterium atom, alkyl, haloalkyl, hydroxyalkyl, cycloalkyl, heterocyclyl, aryl, and heteroaryl;
Or each of R^{d1} and R^{d2} , R^{d3} and R^{d4}, R^{d6} and R^{d7}, and R^{d8} and R^{d9} forms a heterocyclyl together with the nitrogen atom to which they are attached, where the heterocyclyl is each independently optionally substituted by one or more substituents selected from a deuterium atom, halogen, oxo, alkyl, alkoxy, haloalkyl, haloalkoxy, cyano, alkylcyano, amino, alkylamino, hydroxy, hydroxyalkyl, cycloalkyl, heterocyclyl, aryl, and heteroaryl;
at each occurrence, R^{e1}, R^{e2}, R^{e3}, R^{e4}, R^{e5}, R^{e6}, R^{e7}, and R^{e8} are identical or different, and are each independently selected from alkyl, hydroxyalkyl, cycloalkyl, heterocyclyl, aryl, and heteroaryl, where the alkyl, cycloalkyl, heterocyclyl, aryl, and heteroaryl are each independently optionally substituted by one or more substituents selected from a deuterium atom, halogen, oxo, alkyl, haloalkyl, alkoxy, haloalkoxy, cyano, alkylcyano, -(CH₂)ᵤ₁NR^{d1}R^{d2}, hydroxy, hydroxyalkyl, cycloalkyl, heterocyclyl, aryl, and heteroaryl;
m is 0, 1, 2, or 3;
n is 0, 1, 2, 3, 4, 5, or 6;
p is 0, 1, or 2;
q is 0, 1, or 2;
r is 0, 1, or 2;
s is 1 or 2;
t is 0, 1, or 2;
u is 0, 1, 2, or 3;
u1 is 0, 1, 2, or 3;
u2 is 0, 1, 2, or 3;
v1 is 0, 1, 2, or 3;
v2 is 0, 1, 2, or 3;
w1 is 0, 1, 2, or 3; and
w2 is 0, 1, 2, or 3.

In some embodiments of this disclosure, for the compounds of formula (I) or pharmaceutically acceptable salts thereof , s is 1 or 2; preferably, s is 1.

In some embodiments of this disclosure, for the compounds of formula (I) or pharmaceutically acceptable salts thereof , R³ is 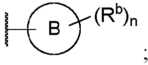 Ring B, R^{b} , and n are as defined in formula (I).

In some embodiments of this disclosure, for the compounds of formula (I) or pharmaceutically acceptable salts thereof, Z is selected from -S-, -S(O)-, and -S(O)₂-; preferably, Z is -S(O)- or -S(O)₂-; more preferably, Z is -S(O)-.

In some embodiments of this disclosure, for the compounds of formula (I) or pharmaceutically acceptable salts thereof, R⁶, R⁷, R⁸ and R⁹ are identical or different and are each independently selected from a hydrogen atom, a deuterium atom, C₁₋₆ alkyl, C₁₋₆ alkoxy, hydroxy C₁₋₆ alkyl, halo C₁₋₆ alkyl, halo C₁₋₆ alkoxy, amino, 3-to 8-membered cycloalkyl, 3- to 8-membered heterocyclyl, 6- to 10-membered aryl, and 5-to 10-membered heteroaryl; preferably, R⁶, R⁷, R⁸ and R⁹ are identical or different and are each independently selected from a hydrogen atom, a deuterium atom, C₁₋₆ alkyl, C₁₋₆ alkoxy, hydroxy C₁₋₆ alkyl, halo C₁₋₆ alkyl, halo C₁₋₆ alkoxy, and amino; more preferably, R⁶, R⁷, R⁸ and R⁹ are identical or different and are each independently selected from a hydrogen atom or C₁₋₆ alkyl; most preferably, R⁶, R⁷, R⁸ and R⁹ are all hydrogen atoms.

In some embodiments of this disclosure, the compounds of formula (I) or pharmaceutically acceptable salts thereof are compounds of formula (II) or pharmaceutically acceptable salts thereof: 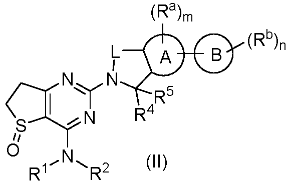 where:
Ring A, Ring B, L, R^{a}, R^{b}, R¹, R², R⁴, R⁵, n and m are as defined in formula (I).

In some embodiments of this disclosure, the compounds of formula (I) or pharmaceutically acceptable salt thereof are compounds of formula (II-1) or formula (II-2) or pharmaceutically acceptable salts thereof: 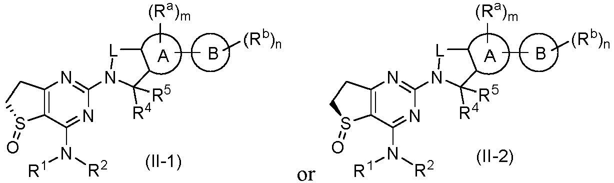 where:
Ring A, Ring B, L, R^{a}, R^{b}, R¹, R², R⁴, R⁵, n and m are as defined in formula (I).

In some embodiments of this disclosure, for the compound of formula (I), formula (II), formula (II-1), or formula (II-2) or the pharmaceutically acceptable salts thereof , L is -(CR^{4a}R^{4b})ₚ(CR^{5a}R⁵⁶)_{q}-; p is 1 or 2; q is 0, 1, or 2; and R^{4a}, R^{4b}, R^{5a} and R^{5b} are as defined in formula (I); preferably, L is -(CR^{4a}R^{4b})ₚ(CR^{5a}R^{5b})_{q}-; p is 1; q is 0 or 1, and R^{4a}, R^{4b}, R^{5a} and R^{5b} are as defined in formula (I).

In some embodiments of this disclosure, the compound of formula (I) or formula (II) or pharmaceutically acceptable salts thereof is a compound of formula (III) or pharmaceutically acceptable salts thereof: 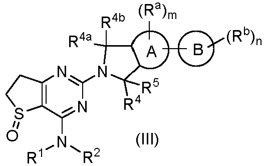 where:
Ring A, Ring B, R^{a}, R^{b}, R¹, R², R⁴, R⁵, R^{4a}, R^{4b}, n and m are as defined in formula (I).

In some embodiments of this disclosure, the compound of formula (I), formula (II) or formula (III) or pharmaceutically acceptable salts thereof are compounds of formula (III-1) or formula (III-2) or pharmaceutically acceptable salts thereof: 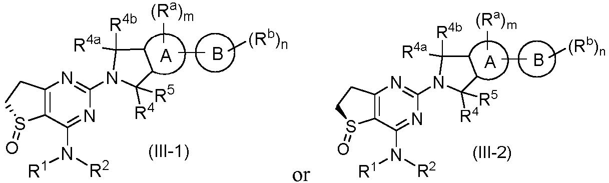 where:
Ring A, Ring B, R^{a}, R^{b}, R¹, R², R⁴, R⁵, R^{4a}, R^{4b}, n and m are as defined in formula (I).

In some embodiments of this disclosure, the compound of formula (I) or formula (II) or pharmaceutically acceptable salts thereof are compounds of formula (IV) or pharmaceutically acceptable salts thereof: 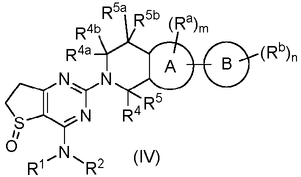 where:
Ring A, Ring B, R^{a}, R^{b}, R¹, R², R⁴, R⁵, R^{4a}, R^{4b}, R^{5a}, R^{5b}, n and m are as defined in formula (I).

In some embodiments of this disclosure, the compound of formula (I), formula (II) or formula (IV) or pharmaceutically acceptable salts thereof are compounds of formula (IV-1) or formula (IV-2) or pharmaceutically acceptable salts thereof: 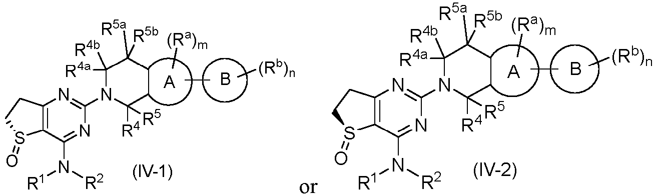 where:
Ring A, Ring B, R^{a}, R^{b}, R¹, R², R⁴, R⁵, R^{4a}, R^{4b}, R^{5a}, R^{5b}, n and m are as defined in formula (I).

In some embodiments of this disclosure, for the compound of formula (I), formula (II), formula (II-1), formula (II-2), formula (III), formula (III-1), formula (III-2), formula (IV), formula (IV-1) or formula (IV-2) or pharmaceutically acceptable salts thereof, ring A is selected from 5- to 10-membered heteroaryl, 6- to 10-membered aryl, 3- to 8-membered heterocyclyl and 3- to 8-membered cycloalkyl; preferably, ring A is 5- to 10-membered heteroaryl or 6- to 10-membered aryl; more preferably, ring A is 5- to 6-membered heteroaryl or phenyl.

In some embodiments of this disclosure, for the compound of formula (I), formula (II), formula (II-1), formula (II-2), formula (III), formula (III-1), formula (III-2), formula (IV), formula (IV-1) or formula (IV-2) or pharmaceutically acceptable salts thereof, ring B is selected from 5- to 10-membered heteroaryl, 6- to 10-membered aryl, 3- to 8-membered heterocyclyl and 3- to 8-membered cycloalkyl; preferably, ring B is selected from 5- to 6-membered heteroaryl, phenyl, 3- to 6-membered heterocyclyl and 3- to 6-membered cycloalkyl.

In some embodiments of this disclosure, for the compound of formula (I), formula (II), formula (II-1), formula (II-2), formula (III), formula (III-1), formula (III-2), formula (IV), formula (IV-1) or formula (IV-2) or pharmaceutically acceptable salts thereof, n is 0, 1, 2 or 3; and each R^{b} is identical or different and independently selected from deuterium atom, halogen, C₁₋₆ alkyl, cyano, C₁₋₆ alkoxyl, C₁₋₆ alkoxy - C₁₋₆ alkoxyl, amino, C₁₋₆ alkylamino, halogenated C₁₋₆ alkyl and halogenated C₁₋₆ alkoxyl; preferably, n is 0 or 1; and R^{b} is selected from halogen, C₁₋₆ alkyl, cyano, C₁₋₆ alkoxyl, amino, C₁₋₆ alkylamino, C₁₋₆ alkoxy - C₁₋₆ alkoxyl, halogenated C₁₋₆ alkyl and halogenated C₁₋₆ alkoxyl.

In some embodiments of this disclosure, for the compound of formula (I), formula (II), formula (II-1), formula (II-2), formula (III), formula (III-1), formula (III-2), formula (IV), formula (IV-1) or formula (IV-2) or pharmaceutically acceptable salts thereof, m is 0, 1, 2 or 3; and each R^{a} is identical or different and independently selected from deuterium atom, halogen, C₁₋₆ alkyl, cyano, C₁₋₆ alkoxyl, amino, C₁₋₆ alkylamino, halogenated C₁₋₆ alkyl and halogenated C₁₋₆ alkoxyl; preferably, m is 0.

In some embodiments of this disclosure, for the compound of formula (I), formula (II), formula (II-1), formula (II-2), formula (III), formula (III-1), formula (III-2), formula (IV), formula (IV-1) or formula (IV-2) or pharmaceutically acceptable salts thereof, 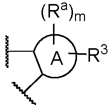 is selected from 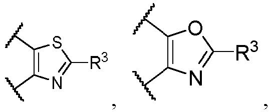 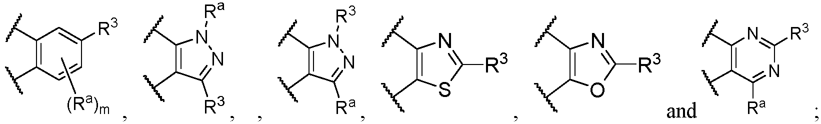 where R³, R^{a} and m are as defined in formula (I).

In some embodiments of this disclosure, for the compound of formula (I), formula (II), formula (II-1), formula (II-2), formula (III), formula (III-1), formula (III-2), formula (IV), formula (IV-1) or formula (IV-2) or pharmaceutically acceptable salts thereof, R¹ and R² are identical or different and each is independently selected from hydrogen atom, deuterium atom, C₁₋₆ alkyl, C₂₋₆ alkenyl, C₂₋₆ alkynyl, 5- to 10-membered heteroaryl, 6- to 10-membered aryl, 3- to 8-membered cycloalkyl and 3- to 8-membered heterocyclyl; where the C₁₋₆ alkyl, C₂₋₆ alkenyl, C₂₋₆ alkynyl, 5- to 10-membered heteroaryl, 6- to 10-membered aryl, 3- to 8-membered cycloalkyl and 3- to 8-membered heterocyclyl are each independently optionally substituted by one or more substituents selected from deuterium atom, C₁₋₆ alkyl, halogen, oxo, halogenated C₁₋₆ alkyl, - OR^{c1}, nitro group, cyano, -(CH₂)ᵤ₁NR^{d1}R^{d2}, -C(O)NR^{d3}R^{d4}, -(CH₂)ᵥ₁NR^{d5}C(O)R^{e1}, -C(O)R^{e2}, -OC(O)R^{e3}, - S(O)ᵣR^{e4}, -(CH₂)_{w1}C(O)OR^{c2}, -(CH₂)ᵤR, hydroxy-C₁₋₆ alkyl, 3- to 8-membered cycloalkyl, 3- to 8-membered heterocyclyl, 6- to 10-membered aryl and 5- to 10-membered heteroaryl; R^{c1}, R^{c2}, R^{d1} to R^{d5}, R^{e1} to R^{e4}, R, u, u1, v1, w1 and r are as defined in the formula (I); preferably, R¹ and R² are identical or different and each is independently selected from hydrogen atom, deuterium atom, C₁₋₆ alkyl, 5- to 10-membered heteroaryl, 6-to 10-membered aryl, 3- to 8-membered cycloalkyl and 3- to 8-membered heterocyclyl; where the C₁₋₆ alkyl, 5- to 10-membered heteroaryl, 6- to 10-membered aryl, 3- to 8-membered cycloalkyl and 3- to 8-membered heterocyclyl are each independently optionally substituted by one or more substituents selected from deuterium atom, halogen, oxo, C₁₋₆ alkyl, halogenated C₁₋₆ alkyl, hydroxyl, C₁₋₆ alkoxyl, -(CH₂)ᵤ₁NR^{d1}R^{d2}, - (CH₂)_{w1}C(O)OR^{c2}, -(CH₂)ᵤR, hydroxy- C₁₋₆ alkyl, 3- to 8-membered cycloalkyl, 3- to 8-membered heterocyclyl, 6- to 10-membered aryl and 5- to 10-membered heteroaryl; R^{c2}, R^{d1}, R^{d2}, R, u, u1 and w1 are as defined in the formula (I); more preferably, R¹ and R² are identical or different and each is independently selected from hydrogen atom, deuterium atom, C₁₋₆ alkyl, 5- to 6-membered heteroaryl, phenyl, 3- to 6-membered cycloalkyl and 3- to 6-membered heterocyclyl; where the C₁₋₆ alkyl, 5- to 6-membered heteroaryl, phenyl, 3- to 6-membered cycloalkyl and 3- to 6-membered heterocyclyl are each independently optionally substituted by one or more substituents selected from deuterium atom, halogen, oxo, C₁₋₆ alkyl, halogenated C₁₋₆ alkyl, hydroxyl, C₁₋₆ alkoxyl, amino, -(CH₂)_{w1}C(O)OR^{c2}, -(CH₂)ᵤR and hydroxy-C₁₋₆ alkyl; R^{c2}, R, u and w1 are as defined in the formula (I); most preferably, one of R¹ and R² is a hydrogen atom, and the other is selected from deuterium atom, C₁₋₆ alkyl, 5- to 6-membered heteroaryl, phenyl, 3- to 6-membered cycloalkyl and 3- to 6-membered heterocyclyl; where the C₁₋₆ alkyl, 5- to 6-membered heteroaryl, phenyl, 3- to 6-membered cycloalkyl and 3- to 6-membered heterocyclyl are each independently optionally substituted by one or more substituents selected from deuterium atom, halogen, oxo, C₁₋₆ alkyl, halogenated C₁₋₆ alkyl, hydroxyl, C₁₋₆ alkoxyl, amino, -(CH₂)_{w1}C(O)OR^{c2}, -(CH₂)ᵤR and hydroxy-C₁₋₆ alkyl; R^{c2}, R, u and w1 are as defined in the formula (I).

In some embodiments of this disclosure, for the compound of formula (I), formula (II), formula (II-1), formula (II-2), formula (III), formula (III-1), formula (III-2), formula (IV), formula (IV-1) or formula (IV-2) or pharmaceutically acceptable salts thereof, one of R¹ and R² is a hydrogen atom, and the other is selected from C₁₋₆ alkyl, 5- to 6-membered heteroaryl, phenyl, 3- to 6-membered cycloalkyl and 3- to 6-membered heterocyclyl; where the C₁₋₆ alkyl, 5- to 6-membered heteroaryl, phenyl, 3- to 6-membered cycloalkyl and 3-to 6-membered heterocyclyl are each independently optionally substituted by one or more substituents selected from halogen, oxo, C₁₋₆ alkyl, halogenated C₁₋₆ alkyl, hydroxyl, C₁₋₆ alkoxyl, amino, - (CH₂)_{w1}C(O)OR^{c2}, and hydroxy-C₁₋₆ alkyl; R^{c2} is a hydrogen atom or C₁₋₆ alkyl; w1 is 0 or 1; preferably, one of R¹ and R² is a hydrogen atom, and the other is phenyl or 3- to 6-membered cycloalkyl; where the phenyl or 3- to 6-membered cycloalkyl is each independently optionally substituted by one or more substituents selected from halogen, -(CH₂)_{w1}C(O)OR^{c2} and hydroxy-C₁₋₆ alkyl; R^{c2} is a hydrogen atom or C₁₋₆ alkyl; w1 is 0 or 1; most preferably, one of R¹ and R² is a hydrogen atom, and the other is selected from

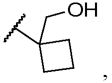

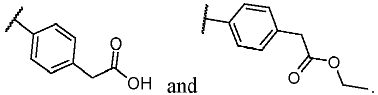

In some embodiments of this disclosure, for the compound of formula (I), formula (II), formula (II-1), formula (II-2), formula (III), formula (III-1), formula (III-2), formula (IV), formula (IV-1) or formula (IV-2) or pharmaceutically acceptable salts thereof, R⁴, R^{4a}, R^{4b} and R⁵ are identical or different and each independently selected from hydrogen atom, deuterium atom, C₁₋₆ alkyl, C₁₋₆ alkoxyl, halogen, cyano, nitro group, amino, hydroxy-C₁₋₆ alkyl, halogenated C₁₋₆ alkyl and halogenated C₁₋₆ alkoxyl; preferably, R⁴, R^{4a}, R^{4b} and R⁵ are identical or different and each independently a hydrogen atom or C₁₋₆ alkyl; more preferably, R⁴, R^{4a}, R^{4b} and R⁵ are all hydrogen atoms.

In some embodiments of this disclosure, for the compound of formula (I), formula (II), formula (II-1), formula (II-2), formula (IV), formula (IV-1) or formula (IV-2) or pharmaceutically acceptable salts thereof, R^{5a} and R^{5b} are identical or different and each independently selected from hydrogen atom, deuterium atom, C₁₋₆ alkyl, C₁₋₆ alkoxyl, halogen, cyano, nitro group, amino, hydroxy-C₁₋₆ alkyl, halogenated alkyl and halogenated C₁₋₆ alkoxyl; preferably, R^{5a} and R^{5b} are identical or different and each is independently a hydrogen atom or C₁₋₆ alkyl; more preferably, R^{5a} and R^{5b} are both hydrogen atoms.

In some embodiments of this disclosure, for the compound of formula (I), formula (II), formula (II-1), formula (II-2), formula (III), formula (III-1), formula (III-2), formula (IV), formula (IV-1) or formula (IV-2) or pharmaceutically acceptable salts thereof, R^{c1}, R^{c2}, R^{c3} and R^{c4} are identical or different at each occurrence and each is independently selected from hydrogen atom, deuterium atom, C₁₋₆ alkyl, hydroxy -C₁₋₆ alkyl, 3-to 8-membered cycloalkyl, 3- to 8-membered heterocyclyl, 6- to 10-membered aryl and 5- to 10-membered heteroaryl, where the C₁₋₆ alkyl, 3- to 8-membered cycloalkyl, 3- to 8-membered heterocyclyl, 6- to 10-membered aryl and 5- to 10-membered heteroaryl are each independently optionally substituted by one or more substituents selected from deuterium atom, halogen, oxo, C₁₋₆ alkyl, halogenated C₁₋₆ alkyl, C₁₋₆ alkoxyl, halogenated C₁₋₆ alkoxyl, cyano, C₁₋₆ alkylcyano, amino, C₁₋₆ alkylamino, hydroxyl and hydroxy-C₁₋₆ alkyl; preferably, R^{c1}, R^{c2}, R^{c3} and R^{c4} are identical or different at each occurrence and each independently selected from hydrogen atom, deuterium atom, C₁₋₆ alkyl and hydroxy-C₁₋₆ alkyl; where the C₁₋₆ alkyl is optionally substituted by one or more substituents selected from deuterium atom, halogen, halogenated C₁₋₆ alkyl, C₁₋₆ alkoxyl, halogenated C₁₋₆ alkoxyl, cyano, C₁₋₆ alkylcyano, amino, C₁₋₆ alkylamino, hydroxyl and hydroxy-C₁₋₆ alkyl;
R^{d1}, R^{d2}, R^{d3}, R^{d4}, R^{d5}, R^{d6}, R^{d7}, R^{d8}, R^{d9} and R^{d10} are identical or different at each occurrence and each independently selected from hydrogen atom, deuterium atom, C₁₋₆ alkyl, halogenated C₁₋₆ alkyl and hydroxy-C₁₋₆ alkyl.

R^{e1}, R^{e2}, R^{e3}, R^{e4}, R^{e5}, R^{e6}, R^{e7} and R^{e8} are identical or different at each occurrence and each independently selected from C₁₋₆ alkyl, hydroxy-C₁₋₆ alkyl, 3- to 8-membered cycloalkyl, 3- to 8-membered heterocyclyl, 6- to 10-membered aryl and 5- to 10-membered heteroaryl.

**Table A. Typical compounds of present disclosure include, but are not limited to:**

| Example No. | Compound Structure and Name |
|---|---|
| 1 | 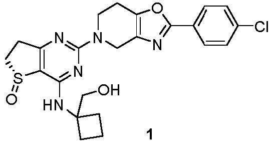 |
| | *(R)*-2-(2-(4-chlorophenyl)-6,7-dihydrooxazolo[4,5-*c*]pyridin-5(4H)-yl)-4-((1-(hydroxymethyl)cyclobutyl)amino)-6,7-dihydrothieno[3,2-*d*]pyrimidine 5-oxide **1** |
| 2 | 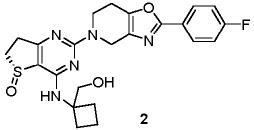 |
| | *(R)*-2-(2-(4-fluorophenyl)-6,7-dihydrooxazolo[4,5-*c*]pyridin-5(4H)-yl)-4-((1-(hydroxymethyl)cyclobutyl)amino)-6,7-dihydrothieno[3,2-*d*]pyrimidine 5-oxide **2** |
| 3 | 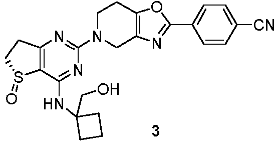 |
| | *(R)*-4-(5-(4-((1-(Hydroxymethyl)cyclobutyl)amino)-5-oxo-6,7-dihydrothieno[3,2-d]pyrimidin-2-yl)-4,5,6,7-tetrahydrooxazole[4,5-*c*]pyridin-2-yl)benzonitrile **3** |
| 4 | 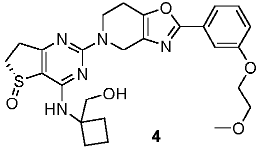 |
| | *(R)*-4-((1-(hydroxymethyl)cyclobutyl)amino)-2-(2-(3-(2-methoxyethoxy)phenyl)-6,7-dihydrooxazole[4,5-c]pyridin-5(4*H*)-yl)-6,7-dihydrothieno[3,2-*d*]pyrimidine 5-oxide **4** |
| 5 | 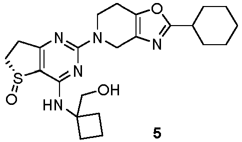 |
| | *(R)*-2-(2-cyclohexyl-6,7-dihydrooxazolo[4,5-*c*]pyridin-5(4*H*)-yl)-4-((1-(hydroxymethyl)cyclobutyl)amino)-6,7-dihydrothieno[3,2-*d*]pyrimidine 5-oxide **5** |
| 6 | 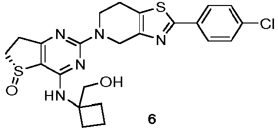 |
| | *(R)*-2-(2-(4-chlorophenyl)-6,7-dihydrothiazolo[4,5-*c*]pyridin-5(4*H*)-yl)-4-((1-(hydroxymethyl)cyclobutyl)amino)-6,7-dihydrothieno[3,2-*d*]pyrimidine 5-oxide **6** |
| 7 | 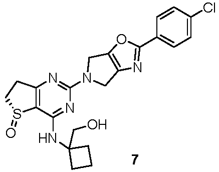 |
| | *(R)*-2-(2-(4-chlorophenyl)-4,6-dihydro-5*H*-pyrrolo[3,4-*d*]oxazol-5-yl)-4-((1-(hydroxymethyl)cyclobutyl)amino)-6,7-dihydrothieno[3,2-*d*]pyrimidine 5-oxide **7** |
| 8 | 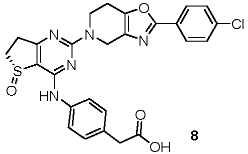 |
| | *(R)-*2-(4-((2-(2-(4-chlorophenyl)-6,7-dihydrooxazolo[4,5-*c*]pyridin-5(4*H*)-yl)-5-oxo-6,7-dihydrothieno[3,2-*d*]pyrimidin-4-yl)amino)phenyl)acetic acid **8** |
| 8d | 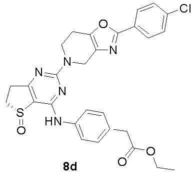 |
| | Ethyl *(R*)-2-(4-((2-(2-(4-chlorophenyl)-6,7-dihydrooxazolo[4,5-*c*]pyridin-5(4*H*)-yl)-5-oxo-6,7-dihydrothieno[3,2-*d*]pyrimidin-4-yl)amino)phenyl)acetate **8d** |
| 9 | 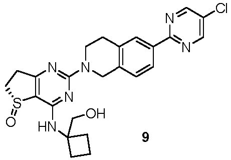 |
| | *(R)*-2-(6-(5-chloropyrimidin-2-yl)-3,4-dihydroisoquinolin-2(1*H*)-yl)-4-((1-(hydroxymethyl)cyclobutyl)amino)-6,7-dihydrothieno[3,2-*d*]pyrimidine 5-oxide **9** |
| 10 | 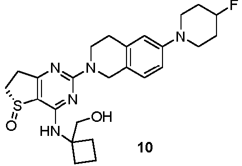 |
| | *(R)*-2-(6-(4-Fluoropiperidin-1-yl)-3,4-dihydroisoquinolin-2(1*H*-yl)-4-((1-(hydroxymethyl)cyclobutyl)amino)-6,7-dihydrothieno[3,2-*d*]pyrimidine 5-oxide **10** |
| 11 | 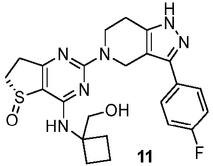 |
| | *(R)*-2-(3-(4-Fluorophenyl)-1,4,6,7-tetrahydro-5*H*-pyrazolo[4,3-*c*]pyridin-5-yl)-4-((1-(hydroxymethyl)cyclobutyl)amino)-6,7-dihydrothieno[3,2-*d*]pyrimidine 5-oxide **11** |
| 12 | 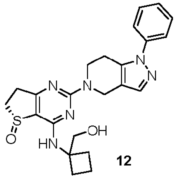 |
| | *(R)*-4-((1-(hydroxymethyl)cyclobutyl)amino)-2-(1-phenyl-1,4,6,7-tetrahydro-5*H*-pyrazolo[4,3-*c*]pyridin-5-yl)-6,7-dihydrothieno[3,2-*d*]pyrimidine-5-oxide **12** |
| 13 | 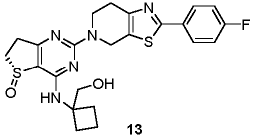 |
| | *(R)*-2-(2-(4-fluorophenyl)-6,7-dihydrothiazolo[5,4-*c*]pyridin-5(4*H*)-yl)-4-((1-(hydroxymethyl)cyclobutyl)amino)-6,7-dihydrothieno[3,2-d]pyrimidine 5-oxide **13** |
| 14 | 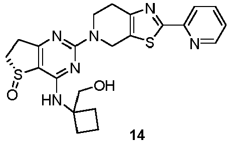 |
| | *(R)*-4-((1-(hydroxymethyl) cyclobutyl) amino)-2-(2-(pyridin-2-yl)-6,7-dihydrothiazolo [5,4*-c*] pyridin-5 (4*H*)-yl)-6,7-dihydrothieno [3,2-*d*] pyrimidine 5-oxide **14** |
| *15* | 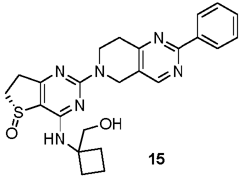 |
| | *(R)*-4-((1-(hydroxymethyl)cyclobutyl)amino)-2-(2-phenyl-7,8-dihydropyrimido[4,3-*d*]pyrimidin-6(5*H*)-yl)-6,7-dihydrothieno[3,2-*d*]pyrimidine 5-oxide **15** |
| 16 | 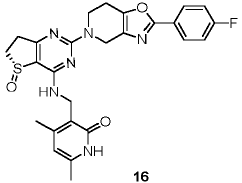 |
| | *(R)-*3-(((2-(2-(4-fluorophenyl)-6,7-dihydrooxazolo[4,5-*c*]pyridin-5(4*H*)-yl)-5-oxo-6,7-dihydrothieno[3,2-*d*]pyrimidin-4-yl)amino)methyl)-4,6-dimethylpyridin-2(1*H*)-one **16** |
| 17 | 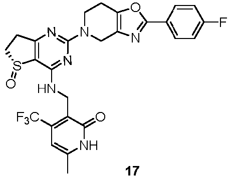 |
| | *(R)*-3-(((2-(2-(4-fluorophenyl)-6,7-dihydrooxazolo[4,5-*c*]pyridin-5(4*H*)-yl)-5-oxo-6,7-dihydrothieno[3,2-*d*]pyrimidin-4-yl)amino)methyl)-6-methyl-4-(trifluoromethyl)pyridin-2(1*H*)-one **17** |
| 18 | 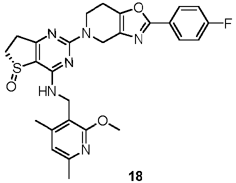 |
| | *(R)-*2-(2-(4-fluorophenyl)-6,7-dihydrooxazolo[4,5-*c]*pyridin-5(4*H*)-yl)-4-(((2-methoxy-4,6-dimethylpyridin-3-yl)methyl)amino)-6,7-dihydrothieno[3,2-*d*]pyrimidine 5-oxide **18** |
| 19 | 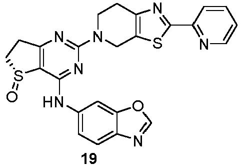 |
| | *(R)*-4-(benzo[d]oxazol-6-ylamino)-2-(2-(pyridin-2-yl)-6,7-dihydrothiazolo[5,4-*c*]pyridin-5(4*H*)-yl)-6,7-dihydrothieno[3,2-d]pyrimidine 5-oxide **19** |
| 20 | 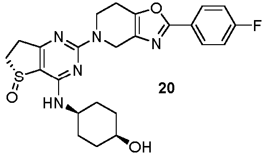 |
| | *(R)*-2-(2-(4-fluorophenyl)-6,7-dihydrooxazolo[4,5-*c*]pyridin-5(4*H*)-yl)-4-(((1*S*,4*S*)-4-hydroxycyclohexyl)amino)-6,7-dihydrothieno[3,2-*d*]pyrimidine 5-oxide **20** |
| 21 | 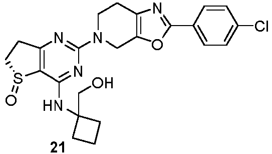 |
| | *(R)*-2-(2-(4-chlorophenyl)-6,7-dihydrooxazolo[5,4-*c*]pyridin-5(4*H*)-yl)-4-((1-(hydroxymethyl)cyclobutyl)amino)-6,7-dihydrothieno[3,2-d]pyrimidine 5-oxide **21** |
| 22 | 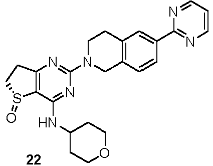 |
| | *(R)*-2-(6-(pyrimidin-2-yl)-3,4-dihydroisoquinolin-2(1H)-yl)-4-((tetrahydro-2H-pyran-4-yl)amino)-6,7-dihydrothieno[3,2-d]pyrimidine 5-oxide **22** |
| 23 | 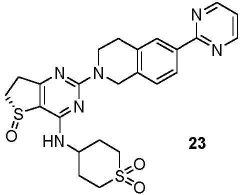 |
| | *(R)*-2-(2-(4-fluorophenyl)-6,7-dihydrooxazolo[4,5-c]pyridin-5(4H)-yl)-4-(((2-methoxy-4,6-dimethylpyridin-3-yl)methyl)amino)-6,7-dihydrothieno[3,2-d]pyrimidine 5-oxide b |
| 24 | 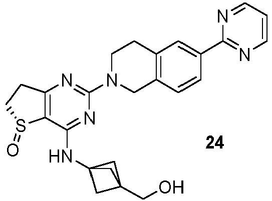 |
| | *(R)*-4-((3-(Hydroxymethyl)bicyclo[1.1.1]pentan-1-yl)amino)-2-(6-(pyrimidin-2-yl)-3,4-dihydroisoquinolin-2(1H)-yl)-6,7-dihydrothieno[3,2-d]pyrimidine 5-oxide **24** |
| 25 | 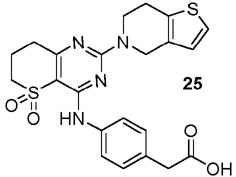 |
| | 2-(4-((2-(6,7-dihydrothieno[3,2-c]pyridin-5(4H)-yl)-5,5-dioxido-7,8-dihydro-6H-thiopyrano[3,2-d]pyrimidin-4-yl)amino)phenyl)acetic acid **25** |
| 25f | 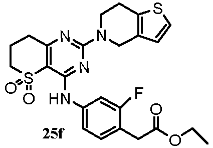 |
| | Ethyl 2-(4-((2-(6,7-dihydrothieno[3,2-c]pyridin-5(4H)-yl)-5,5-dioxido-7,8-dihydro-6H-thiopyrano[3,2-d]pyrimidin-4-yl)amino)phenyl)acetate 25f |
| 26 | 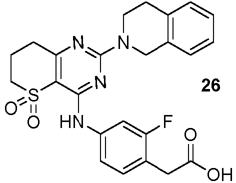 |
| | 2-(4-((2-(3,4-dihydroisoquinolin-2(1H)-yl)-5,5-dioxido-7,8-dihydro-6H-thiopyrano[3,2-d]pyrimidin-4-yl)amino)-2-fluorophenyl)acetic acid 26 |
| 26d | 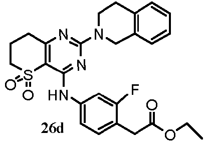 |
| | Ethyl 2-(4-((2-(3,4-dihydroisoquinolin-2(1H)-yl)-5,5- dioxide- 7,8-dihydro-6H-thiopyrano[3,2-d]pyrimidin-4-yl)amino)-2-fluorophenyl)acetate 26d |

**Table B. Typical intermediate compounds of this disclosure include but are not limited to:**

| Example No. | Compound structure and Name |
|---|---|
| 1i | 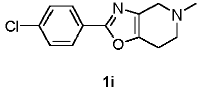 |
| | 2-(4-chlorophenyl)-5-methyl-4,5,6,7-tetrahydrooxazolo[4,5-c]pyridine **1i** |
| | 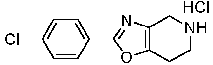 |
| 2e | 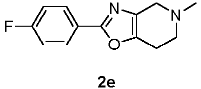 |
| | 2-(4-fluorophenyl)-5-methyl-4,5,6,7-tetrahydrooxazolo[4,5-c]pyridine **2e** |
| 3e | 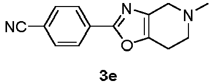 |
| | 2-(4-cyanophenyl)-5-methyl-4,5,6,7-tetrahydrooxazolo[4,5-c]pyridine **3e** |
| 3f | 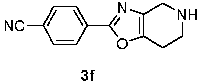 |
| | 2-(4-cyanophenyl)-4,5,6,7-tetrahydrooxazole[4,5-c]pyridine **3f** |
| | 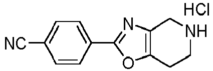 |
| 4f | 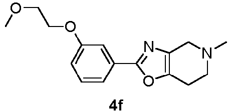 |
| | 2-(3-(2-methoxyethoxy)phenyl)-5-methyl-4,5,6,7-tetrahydrooxazolo[4,5-c]pyridine **4f** |
| 4g | 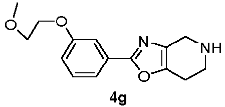 |
| | 2-(3-(2-methoxyethoxy)phenyl)-4,5,6,7-tetrahydrooxazolo[4,5-c]pyridine **4g** |
| | 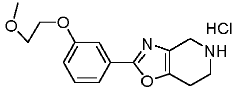 |
| 5e | 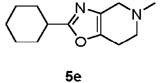 |
| | 2-cyclohexyl-5-methyl-4,5,6,7-tetrahydrooxazolo[4,5-c]pyridine **5e** |
| | 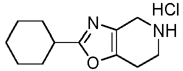 |
| 6e | 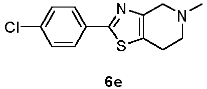 |
| | 2-(4-chlorophenyl)-5-methyl-4,5,6,7-tetrahydrothiazolo[4,5-c]pyridine **6e** |
| | 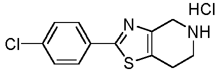 |
| 7e | 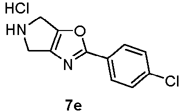 |
| | 2-(4-chlorophenyl)-5,6-dihydro-4*H*-pyrrolo[3,4-d]oxazole hydrochloride **7e** |
| 8b | 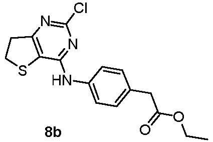 |
| | ethyl 2-(4-((2-chloro-6,7-dihydrothieno[3,2-d]pyrimidin-4-yl)amino)phenyl)acetate **8b** |
| 8c | 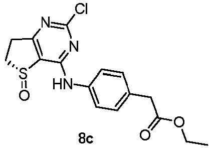 |
| | ethyl *(R)*-2-(4-((2-chloro-5-oxido-6,7-dihydrothieno[3,2-d]pyrimidin-4-yl)amino)phenyl)acetate **8c** |
| 9c | 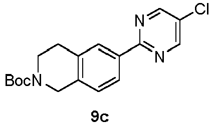 |
| | tert-butyl 6-(5-chloropyrimidin-2-yl)-3,4-dihydroisoquinoline-2(1H)-carboxylate **9c** |
| 9d | 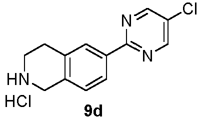 |
| | 6-(5-chloropyrimidin-2-yl)-1,2,3,4-tetrahydroisoquinoline hydrochloride **9d** |
| | 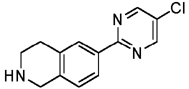 |
| 10a | 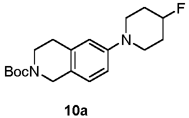 |
| | tert-butyl 6-(4-fluoropiperidin-1-yl)-3,4-dihydroisoquinoline-2(1H)-carboxylate **10a** |
| 10b | 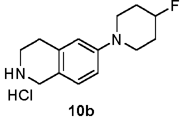 |
| | 6-(4-fluoropiperidin-1-yl)-1,2,3,4-tetrahydroisoquinoline hydrochloride **10b** |
| | 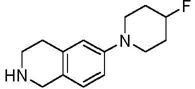 |
| 13d | 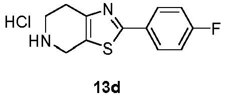 |
| | 2-(4-fluorophenyl)-4,5,6,7-tetrahydrothiazolo[5,4-c]pyridine hydrochloride **13d** |
| 14b | 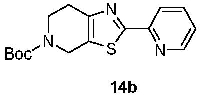 |
| | tert-butyl 2-(pyridin-2-yl)-6,7-dihydrothiazolo[5,4-c]pyridine-5(4H)-carboxylate **14b** |
| 14c | |
| | 2-(pyridin-2-yl)-4,5,6,7-tetrahydrothiazolo[5,4-c]pyridine hydrochloride **14c** |
| 21f | 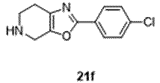 |
| | 2-(4-chlorophenyl)-4,5,6,7-tetrahydrooxazolo[5,4-c]pyridine |
| 22e | 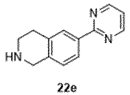 |
| | 6-(pyrimidin-2-yl)-1,2,3,4-tetrahydroisoquinoline |

Another aspect of this disclosure relates to a method for preparing a compound of formula (I) or a pharmaceutically acceptable salt thereof, the method including the following steps:

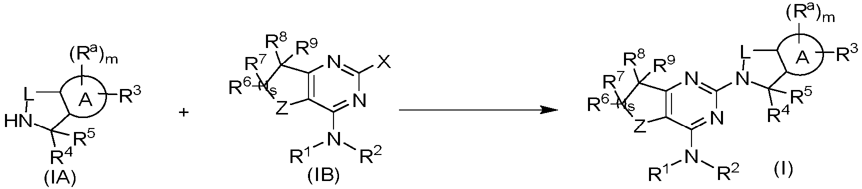

Subjecting a compound of formula (IA) or a salt thereof to a nucleophilic substitution reaction with a compound of formula (IB) or a salt thereof to obtain a compound of formula (I) or a pharmaceutically acceptable salt thereof;
where:
X is a halogen, preferably Cl;
Ring A, L, Z, R^{a}, R¹ to R⁹, m and s are as defined in the formula (I).

Another aspect of the present disclosure relates to a method for preparing a compound of formula (II) or a pharmaceutically acceptable salt thereof, whichincludes the following steps:

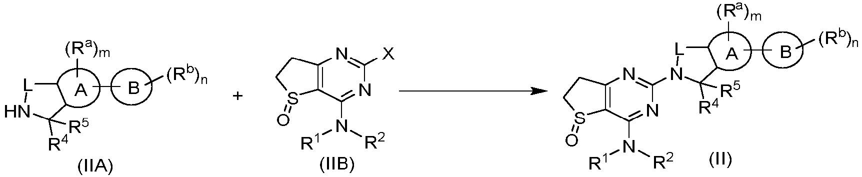

Subjecting a compound of formula (IIA) or a salt thereof to a nucleophilic substitution reaction with a compound of formula (IIB) or a salt thereof to obtain a compound of formula (II) or a pharmaceutically acceptable salt thereof;
Where:
X is a halogen, preferably Cl;
Ring A, Ring B, L, R^{a}, R^{b}, R¹, R², R⁴, R⁵, m and n are as defined in formula (II).

Another aspect of the present disclosure relates to a method for preparing the compound of formula (II-1) or its salt, which includes the following steps:

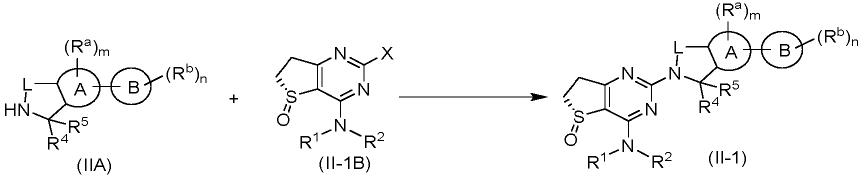

Subjecting a compound of formula (IIA) or a salt thereof to a nucleophilic substitution reaction with a compound of formula (II-1B) or a salt thereof to obtain a compound of formula (II-1) or a pharmaceutically acceptable salt thereof;
where, X represents a halogen, preferably Cl;
Ring A, Ring B, L, R^{a}, R^{b}, R¹, R², R⁴, R⁵, m and n are as defined in formula (II-1).

Another aspect of the present disclosure relates to a method for preparing the compound of formula (II-2) or a pharmaceutically acceptable salt thereof, which includes the following steps:

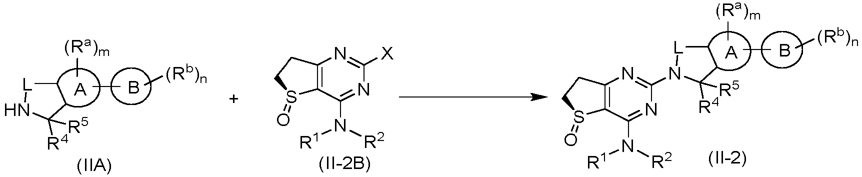

Subjecting a compound of formula (IIA) or a salt thereof to a nucleophilic substitution reaction with a compound of formula (II-2B) or a salt thereof to obtain a compound of formula (II-2) or a pharmaceutically acceptable salt thereof;
where, X is a halogen, preferably Cl.

Ring A, Ring B, L, R^{a}, R^{b}, R¹, R², R⁴, R⁵, m and n are as defined in formula (II-2).

Another aspect of this disclosure relates to a method for preparing a compound of formula (III) or a pharmaceutically acceptable salt thereof, which includes the following steps:

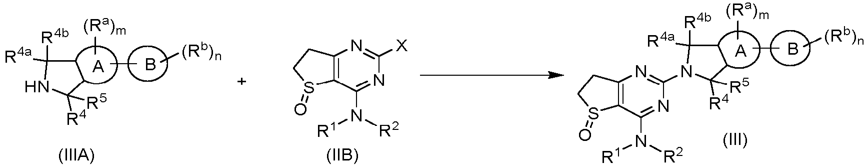

Subjecting a compound of formula (IIIA) or a salt thereof to a nucleophilic substitution reaction with a compound of formula (IIB) or a salt thereof to obtain a compound of formula (III) or a pharmaceutically acceptable salt thereof;
where, X represents a halogen, preferably Cl.

Ring A, Ring B, R^{a}, R^{b}, R¹, R², R⁴, R⁵, R^{4a}, R^{4b}, m and n are as defined in formula (III).

Another aspect of the present disclosure relates to a method for preparing the compound of formula (III-1) or a pharmaceutically acceptable salt thereof, which includes the following steps:

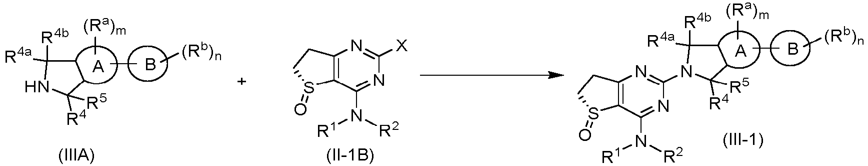

Subjecting a compound of formula (IIIA) or a salt thereof to a nucleophilic substitution reaction with a compound of formula (II-1B) or a salt thereof to obtain a compound of formula (III-1) or a pharmaceutically acceptable salt thereof;
where, X is a halogen, preferably Cl.

Ring A, Ring B, R^{a}, R^{b}, R¹, R², R⁴, R⁵, R^{4a}, R^{4b}, m and n are defined as in formula (III-1).

Another aspect of the present disclosure relates to a method for preparing the compound of formula (III-2) or a pharmaceutically acceptable salt thereof, which includes the following steps:

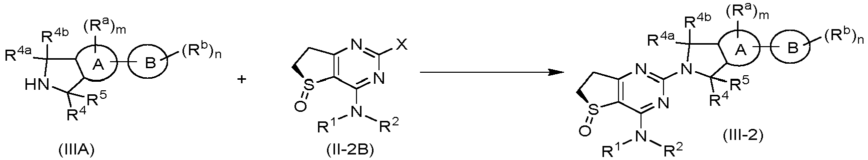

Subjecting a compound of formula (IIIA) or a salt thereof to a nucleophilic substitution reaction with a compound of formula (II-2B) or a salt thereof to obtain a compound of formula (III-2) or a pharmaceutically acceptable salt thereof;
where, X is a halogen, preferably Cl.

Ring A, Ring B, R^{a}, R^{b}, R¹, R², R⁴, R⁵, R^{4a}, R^{4b}, m and n are defined as in formula (III-2).

Another aspect of the present disclosure relates to a method for preparing a compound of formula (IV) or a pharmaceutically acceptable salt thereof, which includes the following steps:

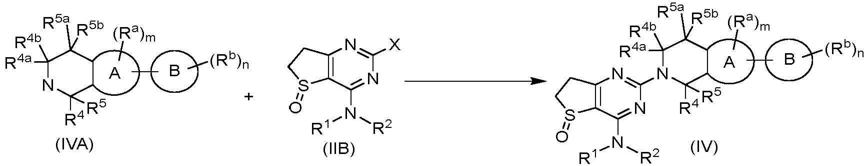

Subjecting a compound of formula (IVA) or a salt thereof to a nucleophilic substitution reaction with a compound of formula (IIB) or a salt thereof to obtain a compound of formula (IV) or a pharmaceutically acceptable salt thereof;
where, X is a halogen, preferably Cl.

Ring A, Ring B, R^{a}, R^{b}, R¹, R², R⁴, R⁵, R^{4a}, R^{4b}, R^{5a}, R^{5b}, m and n are defined as in formula (IV).

Another aspect of the present disclosure relates to a method for preparing the compound of formula (IV-1) or a pharmaceutically acceptable salt thereof, which includes the following steps:

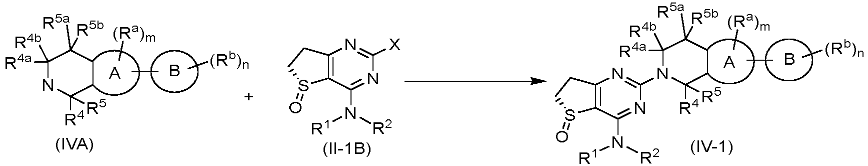

Subjecting a compound of formula (IVA) or a salt thereof to a nucleophilic substitution reaction with a compound of formula (II-1B) or a salt thereof to obtain a compound of formula (IV-1) or a pharmaceutically acceptable salt thereof;
where, X is a halogen, preferably Cl.

Ring A, Ring B, R^{a}, R^{b}, R¹, R², R⁴, R⁵, R^{4a}, R^{4b}, R^{5a}, R^{5b}, m and n are defined as in formula (IV-1).

Another aspect of this disclosure relates to a method for preparing the compound of formula (IV-2) or its pharmaceutically acceptable salt, which includes the following steps:

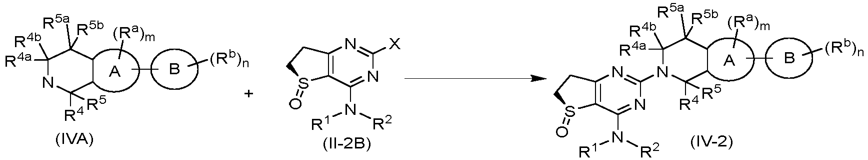

Subjecting a compound of formula (IVA) or a salt thereof to a nucleophilic substitution reaction with a compound of formula (II-2B) or a salt thereof to obtain a compound of formula (IV-2) or a pharmaceutically acceptable salt thereof;
where, X represents a halogen, preferably Cl.

Ring A, Ring B, R^{a}, R^{b}, R¹, R², R⁴, R⁵, R^{4a}, R^{4b}, R^{5a}, R^{5b}, m and n are defined as in formula (IV-2).

Another aspect of the present disclosure relates to a pharmaceutical composition, which contains a therapeutically effective amount of a compound of formula (I), formula (II), formula (II-1), formula (II-2), formula (III), formula (III-1), formula (III-2), formula (IV), formula (IV-1), formula (IV-2) in present disclosure or a compound shown in Table A, or a pharmaceutically acceptable salt thereof, and one or more pharmaceutically acceptable carriers, diluents or excipients.

The present disclosure further relates to the use of a compound of formula (I), formula (II), formula (II-1), formula (II-2), formula (III), formula (III-1), formula (III-2), formula (IV), formula (IV-1), formula (IV-2) or as shown in Table A, or a pharmaceutically acceptable salt thereof, or a pharmaceutical composition containing the same in the preparation of a medicament for inhibiting PDEs enzymes, preferably PDE4 enzymes, more preferably PDE4B enzymes.

The present disclosure further relates to the used a compounds of formula (I), formula (II), formula (II-1), formula (II-2), formula (III), formula (III-1), formula (III-2), formula (IV), formula (IV-1), formula (IV-2) or as shown in Table A, or the pharmaceutically acceptable salts thereof, or the pharmaceutical compositions containing the same, in the preparation of a medicament for treating and/or preventing diseases or disorders mediated by PDEs enzymes, preferably PDE4 enzymes, more preferably PDE4B enzymes.

The present disclosure further relates to the use of the compounds of formula (I), formula (II), formula (II-1), formula (II-2), formula (III), formula (III-1), formula (III-2), formula (IV), formula (IV-1), formula (IV-2) or as shown in Table A, or pharmaceutically acceptable salts thereof, or pharmaceutical compositions containing the same, in the preparation of a medicament for treating and/or preventing diseases or disorders mediated by PDEs enzymes, preferably PDE4 enzymes, more preferably PDE4B enzymes, where the diseases or disorders are selected from respiratory diseases, pulmonary diseases, gastrointestinal diseases, inflammatory diseases, cancers, and peripheral or central nervous system diseases.

The present disclosure further relates to a method for inhibiting PDEs enzymes, which includes administering to a patient in need a therapeutically effective amount of a compound represented by formula (I), formula (II), formula (II-1), formula (II-2), formula (III), formula (III-1), formula (III-2), formula (IV), formula (IV-1), formula (IV-2) or as shown in Table A, or a pharmaceutically acceptable salt thereof, or a pharmaceutical composition including the same. Preferably, the present disclosure further relates to a method for inhibiting PDE4 enzymes, which includes administering to a patient in need a therapeutically effective amount of a compound represented by formula (I), formula (II), formula (II-1), formula (II-2), formula (III), formula (III-1), formula (III-2), formula (IV), formula (IV-1), formula (IV-2) or as shown in Table A, or a pharmaceutically acceptable salt thereof, or a pharmaceutical composition including the same. More preferably, the present disclosure further relates to a method for inhibiting PDE4B enzymes, which includes administering to a patient in need a therapeutically effective amount of a compound represented by formula (I), formula (II), formula (II-1), formula (II-2), formula (III), formula (III-1), formula (III-2), formula (IV), formula (IV-1), formula (IV-2) or as shown in Table A, or a pharmaceutically acceptable salt thereof, or a pharmaceutical composition including the same.

The present disclosure further relates to a method for treating and/or preventing diseases or disorders mediated by PDEs enzymes, which includes administering to a patient in need a therapeutically effective amount of a compound represented by formula (I), formula (II), formula (II-1), formula (II-2), formula (III), formula (III-1), formula (III-2), formula (IV), formula (IV-1), formula (IV-2) or as shown in Table A, or a pharmaceutically acceptable salt thereof, or a pharmaceutical composition including the same. Preferably, the present disclosure further relates to a method for treating and/or preventing diseases or disorders mediated by PDE4 enzymes, which includes administering to a patient in need a therapeutically effective amount of a compound represented by formula (I), formula (II), formula (II-1), formula (II-2), formula (III), formula (III-1), formula (III-2), formula (IV), formula (IV-1), formula (IV-2) or as shown in Table A, or a pharmaceutically acceptable salt thereof, or a pharmaceutical composition including the same. More preferably, the present disclosure further relates to a method for treating and/or preventing diseases or disorders mediated by PDE4B enzymes, which includes administering to a patient in need a therapeutically effective amount of a compound represented by formula (I), formula (II), formula (II-1), formula (II-2), formula (III), formula (III-1), formula (III-2), formula (IV), formula (IV-1), formula (IV-2) or as shown in Table A, or a pharmaceutically acceptable salt thereof, or a pharmaceutical composition including the same.

The present disclosure further relates to a method for treating and/or preventing diseases or disorders mediated by PDEs enzymes, preferably PDE4 enzymes, more preferably PDE4B enzymes, which includes administering to a patient in need a therapeutically effective amount of a compound represented by formula (I), formula (II), formula (II-1), formula (II-2), formula (III), formula (III-1), formula (III-2), formula (IV), formula (IV-1), formula (IV-2) or as shown in Table A, or a pharmaceutically acceptable salt thereof, or a pharmaceutical composition including the same, where the diseases or disorders are selected from respiratory diseases, pulmonary diseases, gastrointestinal diseases, inflammatory diseases, cancers, and peripheral or central nervous system diseases.

The present disclosure further relates to a compound of formula (I), formula (II), formula (II-1), formula (II-2), formula (III), formula (III-1), formula (III-2), formula (IV), formula (IV-1), formula (IV-2) or as shown in Table A, or a pharmaceutically acceptable salt thereof, or a pharmaceutical composition including the same, for use as a medicament.

The present disclosure further relates to a compound of formula (I), formula (II), formula (II-1), formula (II-2), formula (III), formula (III-1), formula (III-2), formula (IV), formula (IV-1), formula (IV-2) or as shown in Table A, or a pharmaceutically acceptable salt thereof, or a pharmaceutical composition including the same, for use as a PDEs enzyme inhibitor, preferably a PDE4 enzyme inhibitor, more preferably a PDE4B enzyme inhibitor.

The present disclosure further relates to a compound of formula (I), formula (II), formula (II-1), formula (II-2), formula (III), formula (III-1), formula (III-2), formula (IV), formula (IV-1), formula (IV-2) or as shown in Table A, or a pharmaceutically acceptable salt thereof, or a pharmaceutical composition including the same, for use in treating and/or preventing diseases or disorders mediated by PDEs enzymes, preferably PDE4 enzymes, more preferably PDE4B enzymes.

The present disclosure further relates to a compound of formula (I), formula (II), formula (II-1), formula (II-2), formula (III), formula (III-1), formula (III-2), formula (IV), formula (IV-1), formula (IV-2) or as shown in Table A, or a pharmaceutically acceptable salt thereof, or a pharmaceutical composition including the same, for use in treating and/or preventing diseases or disorders mediated by PDEs enzymes, preferably PDE4 enzymes, more preferably PDE4B enzymes, where the diseases or disorders are selected from respiratory diseases, pulmonary diseases, gastrointestinal diseases, inflammatory diseases, cancers, and peripheral or central nervous system diseases.

Preferably, the respiratory and pulmonary diseases described in present disclosure are selected from the respiratory and pulmonary diseases, obstructive pulmonary diseases and airway diseases, which are accompanied by increased mucus production. Preferably, the respiratory and pulmonary diseases are selected from COPD (chronic obstructive pulmonary disease), asthma, interstitial lung disease, pulmonary fibrosis, idiopathic pulmonary fibrosis(IPF), α1-antitrypsin deficiency, chronic sinusitis, chronic bronchitis and pulmonary arterial hypertension.

Preferably, the gastrointestinal diseases described in the present disclosure are selected from regional enteritis, ulcerative colitis, and Crohn's disease.

Preferably, the inflammatory diseases described in the present disclosure are selected from hyperplastic and inflammatory skin diseases, arthritic diseases, and ocular inflammatory diseases; where, the inflammatory skin diseases are preferably selected from atopic dermatitis, seborrheic dermatitis, contact dermatitis, epidermal inflammation, alopecia, alopecia areata, rosacea, SAPHO syndrome, skin atrophy, skin photoaging, acne vulgaris, hidradenitis suppurativa, urticaria, pruritus, eczema hand dermatitis, and psoriasis; the arthritic diseases are preferably selected from rheumatoid arthritis, psoriatic arthritis, and spondyloarthritis; the ocular inflammatory disease is preferably glaucoma or dry eye syndrome; the contact dermatitis includes irritant contact dermatitis and allergic contact dermatitis; the psoriasis includes psoriasis vulgaris and inverse psoriasis; the SAPHO syndrome includes synovitis, acne, pustulosis, hyperostosis, and osteitis.

Preferably, the peripheral or central nervous system diseases described in present disclosure are selected from Alzheimer's disease, age-associated memory impairment (AAMI), age-related cognitive decline, vascular dementia, delirium, Parkinson's disease, Huntington's disease, Pick's disease, mental retardation, cerebrovascular diseases, depression, schizophrenia, stroke, neurasthenic disorders, attention deficit disorder, subdural hematoma, normal-pressure hydrocephalus, brain tumors, cerebral apoplexy, cognitive impairment caused by sleep deprivation, intellectual and developmental disabilities, and multiple sclerosis.

Preferably, the cancers described in the present disclosure are selected from leukemia, lymphoma, macroglobulinemia, heavy chain disease, sarcoma, carcinoma, pancreatic cancer, breast cancer, ovarian cancer, prostate cancer, squamous cell carcinoma, sweat gland carcinoma, sebaceous gland carcinoma, papillary carcinoma, cystadenocarcinoma, medullary carcinoma, bronchial carcinoma, liver cancer, cholangiocarcinoma, choriocarcinoma, seminoma, embryonal carcinoma, Wilms' tumor, cervical cancer, endometrial cancer, testicular cancer, lung cancer, bladder cancer, glioma, medulloblastoma, craniopharyngioma, ependymoma, pinealoma, hemangioblastoma, acoustic neuroma, neurilemmoma, neurofibroma, retinoblastoma, melanoma, skin cancer, kidney cancer, nasopharyngeal cancer, gastric cancer, esophageal cancer, head and neck cancer, colorectal cancer, small intestinal cancer, gallbladder cancer, pediatric tumors, urothelial cancer, ureteral tumor, thyroid cancer, osteoma, neuroblastoma, brain tumor and myeloma.

In some embodiments, the unit dose of the pharmaceutical composition described in the present disclosure is 0.001 mg-1000 mg.

In some embodiments, based on the total weight of the composition, the pharmaceutical composition contains 0.01% - 99.99% of the compound of the formula (I), formula (II), formula (II-1), formula (II-2), formula (III), formula (III-1), formula (III-2), formula (IV), formula (IV-1), formula (IV-2) or as shown in Table A, or a pharmaceutically acceptable salt thereof, or a pharmaceutical composition including the same. In some embodiments, the pharmaceutical composition contains 0.1% - 99.9% of the compound of the formula (I), formula (II), formula (II-1), formula (II-2), formula (III), formula (III-1), formula (III-2), formula (IV), formula (IV-1), formula (IV-2) or as shown in Table A, or a pharmaceutically acceptable salt thereof, or a pharmaceutical composition including the same. In some embodiments, the pharmaceutical composition contains 0.5%-99.5% of the compound of the formula (I), formula (II), formula (II-1), formula (II-2), formula (III), formula (III-1), formula (III-2), formula (IV), formula (IV-1), formula (IV-2) or as shown in Table A, or a pharmaceutically acceptable salt thereof, or a pharmaceutical composition including the same. In some embodiments, the pharmaceutical composition contains 1%-99% of the compound of the formula (I), formula (II), formula (II-1), formula (II-2), formula (III), formula (III-1), formula (III-2), formula (IV), formula (IV-1), formula (IV-2) or as shown in Table A of the present disclosure, or a pharmaceutically acceptable salt thereof, or a pharmaceutical composition including the same. In some embodiments, the pharmaceutical composition contains 2%-98% of the compound of the formula (I), formula (II), formula (II-1), formula (II-2), formula (III), formula (III-1), formula (III-2), formula (IV), formula (IV-1), formula (IV-2) or as shown in Table A, or a pharmaceutically acceptable salt thereof, or a pharmaceutical composition including the same.

In some embodiments, based on the total weight of the composition, the pharmaceutical composition described in present disclosure contains 0.01% - 99.99% of a pharmaceutically acceptable carrier, diluent or excipient. In some embodiments, the pharmaceutical composition described in present disclosure contains 0.1% - 99.9% of a pharmaceutically acceptable carrier, diluent or excipient. In some embodiments, the pharmaceutical composition described in present disclosure contains 0.5% - 99.5% of a pharmaceutically acceptable carrier, diluent or excipient. In some embodiments, the pharmaceutical composition described in present disclosure contains 1% - 99% of a pharmaceutically acceptable carrier, diluent or excipient. In some embodiments, the pharmaceutical composition described in present disclosure contains 2% - 98% of a pharmaceutically acceptable carrier, diluent or excipient.

The compounds of formula (I), formula (II), formula (II-1), formula (II-2), formula (III), formula (III-1), formula (III-2), formula (IV), formula (IV-1), formula (IV-2) or as shown in Table A of the present disclosure, their pharmaceutically acceptable salts, or pharmaceutical compositions containing them, etc., can be administered into an organism via any administration route. The administration routes can include oral administration, intravenous injection, intramuscular injection, subcutaneous injection, rectal administration, vaginal administration, sublingual administration, nasal inhalation, oral inhalation, eye drops, as well as local or systemic transdermal administration.

The compounds of formula (I), formula (II), formula (II-1), formula (II-2), formula (III), formula (III-1), formula (III-2), formula (IV), formula (IV-1), formula (IV-2) or as shown in Table A of the present disclosure, their pharmaceutically acceptable salts, etc., or pharmaceutical compositions containing them can be formulated into a single dose, which contains the active compounds in present disclosure, as well as carriers, excipients, etc. The dosage forms for administration can be tablets, capsules, injections, granules, powders, suppositories, pills, creams, pastes, gels, powders, oral solutions, inhalants, suspensions, dry suspensions, patches, lotions, etc. These dosage forms can contain components commonly used in pharmaceutical preparations, such as diluents, absorbents, wetting agents, binders, disintegrants, colorants, pH regulators, antioxidants, bacteriostats, isotonic regulators, anti-sticking agents, etc.

Appropriate formulations for the above-mentioned various dosage forms can be obtained from public sources, such as Remington: The Science and Practice of Pharmacy, 21st edition, published by Lippincott Williams & Wilkins in 2006, and Rowe, Raymond C. Handbook of Pharmaceutical Excipients, Chicago, published in 2005 by Pharmaceutical Press. Therefore, those skilled in the art can easily prepare them.

As well-known to those skilled in the art, the dosage of a drug depends on various factors, including but not limited to the following: the activity of the specific compound used, the age of the patient, the weight of the patient, the health condition of the patient, the behavior of the patient, the diet of the patient, the administration time, the administration method, the excretion rate, the combination of drugs, the severity of the disease, etc. In addition, the optimal treatment method, such as the treatment mode, the daily dosage of the compound, or the type of pharmaceutically acceptable salt, can be verified according to traditional treatment protocols.

### Explanation of Terms

Unless stated to the contrary, the terms used in the specification and claims have the following meanings.

The term "alkyl" refers to a saturated straight-chain or branched-chain aliphatic hydrocarbon group having 1 to 20 (e.g., 1, 2, 3, 4, 5, 6, 7, 8, 9, 10, 11, 12, 13, 14, 15, 16, 17, 18, 19, or 20) carbon atoms (i.e., C₁₋₂₀ alkyl). Preferably, the alkyl is an alkyl having 1 to 12 carbon atoms (i.e., C₁₋₁₂ alkyl), and more preferably 1 to 6 carbon atoms (i.e., C₁₋₆ alkyl). Non-limiting examples include: methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3- methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3- dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3- dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and their various branched isomers, etc. The alkyl can be substituted or unsubstituted. When substituted, it can be substituted at any available attachment point, and the substituents are preferably selected from one or more of a D atom, halogen, alkoxyl, haloalkyl, haloalkoxyl, cycloalkyloxyl, heterocyclic oxy groups, hydroxyl, hydroxyalkyl, cyano, amino, nitro group, cycloalkyl, heterocyclyl, aryl, and heteroaryl.

The term "alkylene" refers to a divalent alkyl group, where the alkyl is defined as above, having 1 to 20 (e.g., 1, 2, 3, 4, 5, 6, 7, 8, 9, 10, 11, 12, 13, 14, 15, 16, 17, 18, 19, or 20) carbon atoms (i.e., C_{1 - 20} alkylene), preferably, 1 to 12 carbon atoms (i.e., C₁₋₁₂ alkylene), more preferably 1 to 6 carbon atoms (i.e., C₁₋₆ alkylene). Non-limiting examples include: -CH₂-, -CH(CH₃)-, -C(CH₃)₂-, -CH₂CH₂-, -CH(CH₂CH₃)-, -CH₂CH(CH₃)-, -CH₂C(CH₃)₂-, -CH₂CH₂CH₂-, -CH₂CH₂CH₂CH₂-, etc. The alkylene can be substituted or unsubstituted. When substituted, it can be substituted at any available attachment point, and the substituents are preferably selected from one or more of a D atom, halogen, alkoxyl, haloalkyls, haloalkoxyl, cycloalkyloxyl, heterocyclic oxyl, hydroxyl, hydroxyalkyl, cyano, amino, nitro group, cycloalkyl, heterocyclyl, aryl group, and heteroaryl.

The term "alkenyl" refers to an alkyl group containing at least one carbon-carbon double bond in the molecule, where the alkyl is defined as above, having 2 to 12 (e.g., 2, 3, 4, 5, 6, 7, 8, 9, 10, 11, or 12) carbon atoms (i.e., C₂₋₁₂ alkenyl), preferably 2 to 6 carbon atoms (i.e., C₂₋₆ alkenyl). Non-limiting examples include: vinyl, propenyl, isopropenyl, butenyl, etc. The alkenyl can be substituted or unsubstituted. When substituted, it can be substituted at any available attachment point, and the substituents are preferably selected from one or more of a D atom, alkoxyl, halogen, haloalkyl, haloalkoxyl, cycloalkyloxy, heterocyclic oxyl, hydroxyl, hydroxyalkyl, cyano, amino, nitro group, cycloalkyl, heterocyclyl, aryl, and heteroaryl.

The term "alkynyl" refers to an alkyl group containing at least one carbon-carbon triple bond in the molecule, where the alkyl is defined as above, having 2 to 12 (e.g., 2, 3, 4, 5, 6, 7, 8, 9, 10, 11, or 12) carbon atoms (i.e., C₂₋₁₂ alkynyl), preferably 2 to 6 carbon atoms (i.e., C₂₋₆ alkynyl). Non-limiting examples include: ethynyl, propynyl, butynyl, pentynyl, hexynyl, etc. The alkynyl can be substituted or unsubstituted. When substituted, it can be substituted at any available attachment point, and the substituents are preferably selected from one or more of a D atom, alkoxyl, halogen, haloalkyl, haloalkoxyl, cycloalkyloxyl, heterocyclic oxyl, hydroxyl, hydroxyalkyl, cyano, amino, nitro group, cycloalkyl, heterocyclyl, aryl, and heteroaryl.

The term "alkoxy" refers to -O-(alkyl), where the alkyl is defined as above. Non-limiting examples include: methoxy, ethoxy, propoxy, and butoxy, etc. The alkoxyl can be substituted or unsubstituted. When substituted, it can be substituted at any available attachment point, and the substituents are preferably selected from one or more of a D atom, alkoxyl, halogen, haloalkyl, haloalkoxyl, cycloalkyloxyl, heterocyclic oxyl, hydroxyl, hydroxyalkyl, cyano, amino, nitro group, cycloalkyl, heterocyclyl, aryl, and heteroaryl.

The term "cycloalkyl" refers to a saturated or partially unsaturated monocyclic all-carbon ring (i.e., monocyclic cycloalkyl) or a polycyclic system (i.e., polycyclic cycloalkyl), having 3 to 20 (e.g., 3, 4, 5, 6, 7, 8, 9, 10, 11, 12, 13, 14, 15, 16, 17, 18, 19, or 20) ring atoms (i.e., 3- to 20-membered cycloalkyl), preferably 3 to 12 ring atoms (i.e., 3- to 12-membered cycloalkyl), more preferably 3 to 8 ring atoms (i.e., 3- to 8-membered cycloalkyl), or preferably 3 to 6 ring atoms (i.e., 3- to 6-membered cycloalkyl).

Non-limiting examples of the monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, etc.

The polycyclic cycloalkyl includes spirocycloalkyl, fused-ring cycloalkyl and bridged-ring cycloalkyl.

The term "spirocycloalkyl" refers to a polycyclic system in which a carbon atom (referred to as the spiro atom) is shared between rings, and may contain one or more double bonds, or one or more heteroatoms selected from nitrogen, oxygen and sulfur (the nitrogen may be optionally oxidized, i.e., forming an N-oxide; the sulfur may be optionally oxidized, i.e., forming a sulfoxide or sulfone, but excluding -OO -, -O-S- and - S-S-), provided that at least one all-carbon ring is present with the attachment point thereon, and the polycyclic system has 5 to 20 (e.g., 5, 6, 7, 8, 9, 10, 11, 12, 13, 14, 15, 16, 17, 18, 19 or 20) ring atoms (i.e., 5- to 20-membered spirocycloalkyl), preferably 6 to 14 ring atoms (i.e., 6- to 14-membered spirocycloalkyl), more preferably 7 to 10 ring atoms (i.e., 7- to 10-membered spirocycloalkyl). The spirocycloalkyl includes mono-spirocycloalkyl and poly-spirocycloalkyl (such as bis-spirocycloalkyl, etc.), preferably monospirocycloalkyl or bis-spirocycloalkyl, more preferably 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered or 7-membered/6-membered monospirocycloalkyl. Non-limiting examples include: 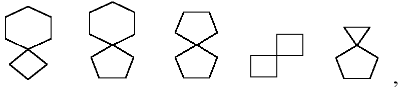 and the attachment point can be at any position;

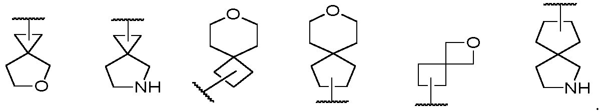

The term "fused cycloalkyl" refers to a polycyclic system in which rings share two adjacent carbon atoms, i.e., formed by the fusion of a monocyclic cycloalkyl with one or more monocyclic cycloalkyls, or the fusion of a monocyclic cycloalkyl with one or more of heterocyclyls, aryls or heteroaryls, where the attachment point is on the monocyclic cycloalkyl. The fused-ring may contain one or more double bonds and has 5 to 20 (e.g., 5, 6, 7, 8, 9, 10, 11, 12, 13, 14, 15, 16, 17, 18, 19 or 20) ring atoms (i.e., 5- to 20-membered fused cycloalkyl), preferably 6 to 14 ring atoms (i.e., 6- to 14-membered fused-ring cycloalkyl), more preferably 7 to 10 ring atoms (i.e., 7- to 10-membered fused-ring cycloalkyl). The fused cycloalkyl includes bicyclic fused cycloalkyl and polycyclic fused cycloalkyl (such as tricyclic fused cycloalkyl, tetracyclic fused cycloalkyl, etc.), preferably bicyclic fused cycloalkyl or tricyclic fused cycloalkyl, more preferably 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered or 7-membered/6-membered bicyclic fused cycloalkyl. Non-limiting examples include: 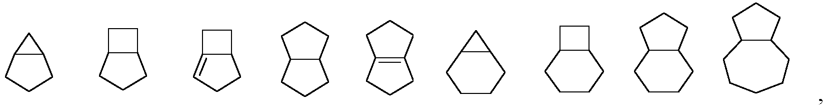 and the attachment point can be at any position;

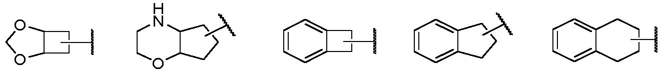

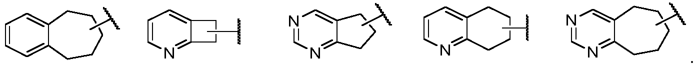

The term "bridged cycloalkyl" refers to an all-carbon polycyclic system in which rings share two non-directly connected carbon atoms, and may contain one or more double bonds, having 5 to 20 (e.g., 5, 6, 7, 8, 9, 10, 11, 12, 13, 14, 15, 16, 17, 18, 19, or 20) carbon atoms (i.e., 5- to 20-membered bridged cycloalkyl), preferably 6 to 14 carbon atoms (i.e., 6- to 14-membered bridged cycloalkyl), more preferably 7 to 10 carbon atoms (i.e., 7 - to 10-membered bridged cycloalkyl). The bridged cycloalkyl includes bicyclic bridged cycloalkyl and polycyclic bridged cycloalkyl (such as tricyclic bridged cycloalkyl, tetracyclic bridged cycloalkyl, etc.), preferably bicyclic bridged cycloalkyl or tricyclic bridged cycloalkyl. Non-limiting examples include: 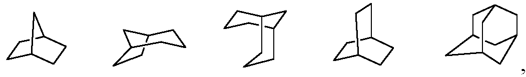 and the attachment point can be at any position.

The cycloalkyl can be substituted or unsubstituted. When substituted, it can be substituted at any available attachment point and the substituents are preferably selected from one or more of a D atom, halogen, alkyl, alkoxyl, haloalkyl, haloalkoxyl, cycloalkyloxyl, heterocyclic oxyl, hydroxyl, hydroxyalkyl, oxo, cyano, amino, nitro group, cycloalkyl, heterocyclyl, aryl, and heteroaryl.

The term "heterocyclyl" refers to a saturated or partially unsaturated monocyclic heterocycle (i.e., monocyclic heterocyclyl) or a polycyclic heterocyclic system (i.e., polycyclic heterocyclyl), which contains at least one (e.g., 1, 2, 3, or 4) heteroatom selected from nitrogen, oxygen, and sulfur in the ring (the nitrogen can be optionally oxidized to form an N-oxide; the sulfur can be optionally oxidized to form a sulfoxide or sulfone, excluding -O-O-, -O-S-, and -S-S-), and has 3 to 20 (e.g., 3, 4, 5, 6, 7, 8, 9, 10, 11, 12, 13, 14, 15, 16, 17, 18, 19, or 20) ring atoms (i.e., 3- to 20 - membered heterocyclyl), preferably 3 to 12 ring atoms (i.e., 3- to 12-membered heterocyclyl), further preferably 3 to 8 ring atoms (i.e., 3- to 8-membered heterocyclyl), more preferably 3 to 6 ring atoms (i.e., 3- to 6-membered heterocyclyl) or preferably 5 or 6 ring atoms (i.e., 5- or 6-membered heterocyclyl).

Non-limiting examples of the monocyclic heterocyclyl include pyrrolidinyl, tetrahydropyranyl, 1,2,3,6-tetrahydropyridinyl, piperidinyl, piperazinyl, morpholinyl, thiomorpholinyl, and homopiperazinyl, etc.

The polycyclic heterocyclyl includes spiroheterocyclyl, fused heterocyclyl, and bridged heterocyclyl.

The term "spiroheterocyclyl" refers to a polycyclic heterocyclic system in which rings share one atom (referred to as the spiro atom), and may contain one or more double bonds, and at least one (e.g., 1, 2, 3, or 4) heteroatom selected from nitrogen, oxygen, and sulfur (the nitrogen can be optionally oxidized to form an N-oxide; the sulfur can be optionally oxidized to form a sulfoxide or sulfone, excluding -O-O-, -O-S-, and - S-S-), provided that it contains at least one monocyclic heterocyclyl with the attachment point thereon, and has 5 to 20 (e.g., 5, 6, 7, 8, 9, 10, 11, 12, 13, 14, 15, 16, 17, 18, 19, or 20) ring atoms (i.e., 5- to 20 - membered spiroheterocyclyl), preferably 6 to 14 ring atoms (i.e., 6- to 14-membered spiroheterocyclyl), more preferably 7 to 10 ring atoms (i.e., 7- to 10-membered spiroheterocyclyl). The spiroheterocyclyl includes monospiroheterocyclyl and polyspiroheterocyclyl (such as bis-spiroheterocyclyl, etc.), preferably monospiroheterocyclyl or bis-spiroheterocyclyl, more preferably 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered or 7-membered/6-membered monospiroheterocyclyl. Non-limiting examples include: 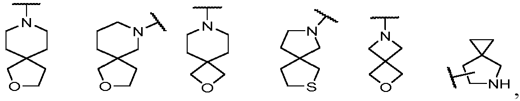 etc.

The term "fused heterocyclyl" refers to a polycyclic heterocyclic system in which rings share two adjacent atoms, and may contain one or more double bonds, and at least one (e.g., 1, 2, 3 or 4) heteroatom selected from nitrogen, oxygen and sulfur (the nitrogen can be optionally oxidized to form nitrogen oxides; the sulfur can be optionally oxygenated to form sulfoxides or sulfones, excluding -O-O-, -O-S-, or -S-S-) in the ring, i.e., formed by the fusion of a monocyclic heterocyclyl with one or more monocyclic heterocyclyls, or the fusion of a monocyclic heterocyclyl with one or more of cycloalkyl, aryl or heteroaryl, where the point of attachment is on the monocyclic heterocyclyl and the fused heterocyclyl has 5 to 20 (e.g., 5, 6, 7, 8, 9, 10, 11, 12, 13, 14, 15, 16, 17, 18, 19 or 20) ring atoms (i.e., 5- to 20-membered fused heterocyclyl), preferably 6 to 14 ring atoms (i.e., 6- to 14-membered fused heterocyclyl), more preferably 7 to 10 ring atoms (i.e., 7- to 10-membered fused heterocyclyl). The fused heterocyclyl includes bicyclic and polycyclic fused heterocyclyls (such as tricyclic fused heterocyclyl, tetracyclic fused heterocyclyl, etc.), preferably bicyclic or tricyclic fused heterocyclyl, more preferably 3/4-membered, 3/5-membered, 3/6-membered, 4/4-membered, 4/5-membered, 4/6-membered, 5/3-membered, 5/4-membered, 5/5-membered, 5/6-membered, 5/7-membered, 6/3-membered, 6/4-membered, 6/5-membered, 6/6-membered, 6/7-membered, 7/5-membered or 7/6-membered bicyclic fused heterocyclyl. Non-limiting examples include: 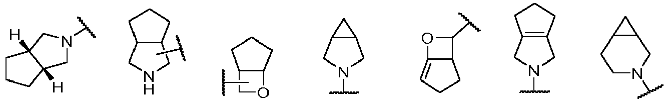 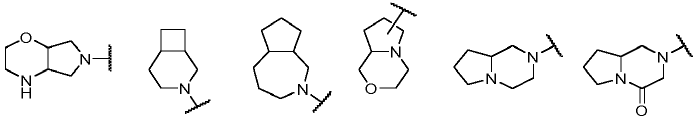 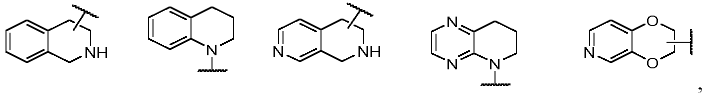 etc.

The term "bridged heterocyclyl" refers to a polycyclic heterocyclic system in which the rings share two non-directly connected atoms, and may contain one or more double bonds and at least one (e.g., 1, 2, 3 or 4) heteroatoms selected from nitrogen, oxygen and sulfur (the nitrogen can be optionally oxidized to form nitrogen oxides; the sulfur can be optionally oxidized to form sulfoxides or sulfones, excluding -O-O-, -O-S-, and -S-S-) in the ring, having 5 to 20 (e.g., 5, 6, 7, 8, 9, 10, 11, 12, 13, 14, 15, 16, 17, 18, 19 or 20) ring atoms (i.e., 5- to 20-membered bridged heterocyclyl). Preferably, the bridged heterocyclyl is a bridged heterocyclyl having 6 to 14 ring atoms (i.e., 6- to 14-membered bridged heterocyclyl), more preferably 7 to 10 ring atoms (i.e., 7- to 10-membered bridged heterocyclyl). According to the number of rings, it can be divided into bicyclic bridged heterocyclyl and polycyclic bridged heterocyclyl (such as tricyclic bridged heterocyclyl, tetracyclic bridged heterocyclyl, etc.), preferably bicyclic bridged heterocyclyl or tricyclic bridged heterocyclyl. Non-limiting examples include: 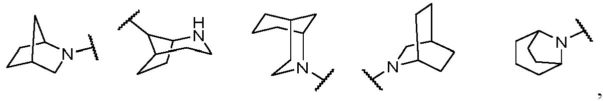 etc.

Heterocyclyl can be substituted or unsubstituted. When substituted, it can be substituted at any available point of attachment, and the substituents are preferably selected from one or more of a deuterium atom, halogen, alkyl, alkoxy, haloalkyl, haloalkoxy, cycloalkyloxyl, heterocyclyloxyl, hydroxyl, hydroxyalkyl, oxo, cyano, amino, nitro, cycloalkyl, heterocyclyl, aryl and heteroaryl.

The term "aryl" refers to a monocyclic all-carbon aromatic ring (i.e., monocyclic aryl) or a polycyclic aromatic ring system (i.e., polycyclic aryl) having a conjugated π-electron system and having 6 to 14 (e.g., 6, 7, 8, 9, 10, 11, 12, 13 or 14) ring atoms (i.e., 6- to 14-membered aryl). The aryl is preferably an aryl having 6 to 10 ring atoms (i.e., 6- to 10-membered aryl), more preferably 8 to 10 ring atoms (i.e., 8- to 10-membered polycyclic aryl). Examples of the monocyclic aryl include phenyl. Non-limiting examples of the polycyclic aryl include naphthyl, anthryl, phenanthryl, etc. The polycyclic aryl also includes phenyl fused with one or more of heterocyclyl or cycloalkyl, or naphthyl fused with one or more of heterocyclyl or cycloalkyl, where the point of attachment is on the phenyl or naphthyl. And in such cases, the number of ring atoms still represents the number of ring atoms in the polycyclic aromatic ring system. Non-limiting examples include: 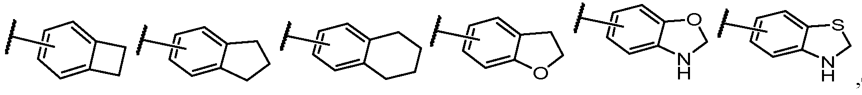 etc.

Aryl can be substituted or unsubstituted. When substituted, it can be substituted at any available attachment point, and the substituents are preferably selected from one or more of a D atom, halogen, alkyl, alkoxyl, haloalkyl, haloalkoxyl, cycloalkyloxyl, heterocyclic oxyl, hydroxyll, hydroxyalkyl l, oxosl, cyanosl, amino, nitro, cycloalkyl, heterocyclyl, aryl, and heteroaryl.

The term "heteroaryl" refers to a monocyclic heteroaromatic ring (i.e., monocyclic heteroaryl) or a polycyclic heteroaromatic ring system (i.e., polycyclic heteroaryl) having a conjugated π-electron system, containing at least one (e.g., 1, 2, 3, or 4) heteroatoms selected from nitrogen, oxygen, and sulfur (the nitrogen can be optionally oxidized to form nitrogen oxides; the sulfur can be optionally oxidized to form sulfoxides or sulfones, excluding -O-O -, -O-S-, and -S-S-), and having 5 to 14 (e.g., 5, 6, 7, 8, 9, 10, 11, 12, 13, or 14) ring atoms (i.e., 5- to 14-membered heteroaryl) in the ring. Preferably, the heteroaryl is a heteroaryl having 5 to 10 ring atoms (i.e., 5- to 10-membered heteroaryl), more preferably 5 or 6 ring atoms (i.e., 5- or 6-membered monocyclic heteroaryl) or 8 to 10 ring atoms (i.e., 8-to 10-membered polycyclic heteroaryl).

Non-limiting examples of the monocyclic heteroaryl include: furyl, thienyl, thiazolyl, isothiazolyl, oxazolyl, isoxazolyl, oxadiazolyl, thiadiazolyl, imidazolyl, pyrazolyl, triazolyl, tetrazolyl, furazanyl, pyrrolyl, N-alkylpyrrolyl, pyridinyl, pyrimidinyl, pyridoneyl, N-alkylpyridone (such as 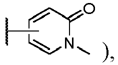 pyrazinyl, pyridazinyl, etc.

Non-limiting examples of the polycyclic heteroaryl groups include: indolyl, indazolyl, quinolinyl, isoquinolinyl, quinoxalinyl, phthalazinyl, benzimidazolyl, benzothiophenyl, benzofuranyl, quinazolinyl, carbazolyl, pyrrolotriazinyl, 5,6,7,8-tetrahydro-triazolo-pyrazinyl, imidazopyridazinyl and [1,2,4]triazolo[1,5-a]pyridinyl, etc. The polycyclic heteroaryl also include those formed by the fusion of a monocyclic heteroaryl group with one or more aryls, where the point of attachment is on the aromatic ring, and in this case, the number of ring atoms still represents the number of ring atoms in the polycyclic heteroaromatic ring system. The polycyclic heteroaryl groups also include those formed by the fusion of a monocyclic heteroaryl group with one or more cycloalkyls or heterocyclyls, where the point of attachment is on the monocyclic heteroaromatic ring, and in this case, the number of ring atoms still represents the number of ring atoms in the polycyclic heteroaromatic ring system. Non-limiting examples include: 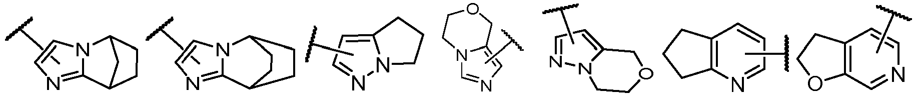 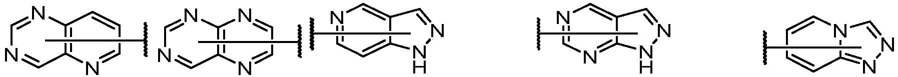 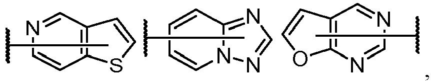 etc.

Heteroaryl can be substituted or unsubstituted. When substituted, it can be substituted at any available point of attachment, and the substituents are preferably selected from one or more of a D atom, halogen, alkyl, alkoxy, haloalkyl, haloalkoxy, cycloalkyloxy, heterocyclyloxyl, hydroxyl, hydroxyalkyl, cyano, amino, nitro, cycloalkyl, heterocyclyl, aryl and heteroaryl.

The term "amino protecting group" refers to a removable group introduced onto an amino to keep the amino intact during reactions at other sites of the molecule. Non-limiting examples include: (trimethylsilyl)ethoxymethyl, tetrahydropyranyl, tert-butoxycarbonyl (Boc), benzyloxycarbonyl (Cbz), fluorenylmethyloxycarbonyl (Fmoc), allyloxycarbonyl (Alloc), trimethylsilylethoxycarbonyl (Teoc), methoxycarbonyl, ethoxycarbonyl, phthaloyl (Pht), tosyl (Tos), trifluoroacetyl (Tfa), trityl (Trt), 2,4-dimethoxybenzyl (DMB), acetyl, benzyl, allyl, p-methoxybenzyl, etc.

The term "hydroxy protecting group" refers to a removable group introduced onto a hydroxyl group to block or protect the hydroxyl group during reactions on other functional groups of the compound. Non-limiting examples include: trimethylsilyl (TMS), triethylsilyl (TES), triisopropylsilyl (TIPS), tertbutyldimethylsilyl (TBS), tert-butyldiphenylsilyl (TBDPS), methyl, tert-butyl, allyl, benzyl, methoxymethyl (MOM), ethoxyethyl, 2-tetrahydropyranyl (THP), formyl, acetyl, benzoyl, p-nitrobenzoyl, etc.

The term "haloalkyl" refers to an alkyl group substituted by one or more halogens, where the alkyl group is as defined above.

The term "haloalkoxyl" refers to an alkoxyl substituted by one or more halogens, where the alkoxyl is as defined above.

The term "hydroxyalkyl" refers to an alkyl group substituted by one or more hydroxyl groups, where the alkyl group is as defined above.

The term "halogen" refers to fluorine, chlorine, bromine or iodine.

The term "hydroxyl" refers to -OH.

The term "amino" refers to -NH₂.

The term "cyano" refers to -CN.

The term "nitro" refers to -NO₂.

The term "oxo" refers to "=O".

The term "carbonyl" refers to C=O.

The term "carboxyl" refers to -C(O)OH.

The term "carboxylate" refers to -C(O)O(alkyl), -C(O)O(cycloalkyl), (alkyl)C(O)O- or (cycloalkyl)C(O)O-, where the alkyl and cycloalkyl are as defined above.

The compounds of the present disclosure can exist in specific stereoisomeric forms. The term "stereoisomer" encompasses all isomeric forms, including enantiomers, diastereomers and geometric isomers including cis-trans isomers. Therefore, individual stereochemical isomers, enantiomers, diastereomers, or geometric isomers (or cis-trans isomers), as well as mixtures thereof, for the compounds designed in the present disclosure, all fall within the scope of the present disclosure. An isomer of a compound described in present disclosure can be prepared by asymmetric synthesis or using a chiral auxiliary. Alternatively, when the molecule contains a basic functional group (such as an amino) or an acidic functional group (such as a carboxyl group), it can form diastereomeric salts with an appropriate optically active acid or base, and then the diastereomers can be separated by conventional methods known in the art to obtain the pure isomers. In addition, the separation of enantiomers and diastereomers is usually accomplished by chromatography.

In the chemical structure of the compounds described in present disclosure, the bond " 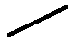 ''' indicates an unspecified configuration, that is, if there are chiral isomers in the chemical structure, the bond " 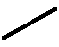 " can be " 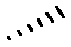 " or " 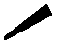 ", or contain both " 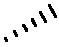 " and " 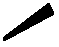 " configurations.

The compounds of the present disclosure include all suitable isotopic derivatives of the compounds. The term "isotopic derivative" refers to a compound in which at least one atom is replaced by an atom with the same atomic number but a different atomic mass. Examples of isotopes that can be introduced into the compounds of the present disclosure include stable and radioactive isotopes of hydrogen, carbon, nitrogen, oxygen, phosphorus, sulfur, fluorine, chlorine, bromine and iodine, etc., such as ²H (deuterium, D) ,³H (tritium, T), ¹¹C, ¹³C, ¹⁴C, ¹⁵N, ¹⁷O, ¹⁸O, ³²p, ³³p, ³³S, ³⁴S, ³⁵S, ³⁶S, ¹⁸F, ³⁶Cl, ⁸²Br, ¹²³I, ¹²⁴I, ¹²⁵I, ¹²⁹I and ¹³¹I, preferably deuterium .

Compared with undeuterated drugs, deuterated drugs have advantages of such as reduced toxicity and side effects, increased drug stability, enhanced efficacy, and prolonged biological half-life. All variations of isotope replacement for the compounds of the present disclosure, whether radioactive or not, are included within the scope of the present disclosure. Each available hydrogen atom attached to a carbon atom can be independently replaced by a deuterium atom, and the deuterium substitution can be partial or complete. Partial deuterium substitution means that at least one hydrogen is replaced by at least one deuterium.

"Optionally" or "optional" means that the subsequently described event or circumstance may occur and not must occur, and it includes both the occurrence and non-occurrence of the event or circumstance. For example, "alkyl optionally substituted with halogen or cyano" includes the case where the alkyl is substituted with halogen or cyano and the case where the alkyl is not substituted with halogen or cyano.

"Substituted" or "substitution" means that one or more hydrogen atoms in a group, preferably 1 to 6, more preferably 1 to 3 hydrogen atoms, are independently replaced by the corresponding number of substituents. Those skilled in the art can determine possible or impossible substitutions without undue effort (through experiments or theory). For example, an amino or a hydroxyl group with a free hydrogen may be unstable when combined with a carbon atom having an unsaturated bond (such as an alkene).

"Pharmaceutical composition" means a mixture containing one or more of the compounds described herein or the pharmaceutically acceptable salts thereof and other chemical components, and other components such as pharmaceutically acceptable carriers and excipients. The purpose of the pharmaceutical composition is to facilitate the administration to organisms, facilitating the absorption of the active ingredient to exert its biological activity.

"Pharmaceutically acceptable salt" refers to a salt of the compounds of the present disclosure, which can be selected from inorganic salts or organic salts. Such salts are safe and effective when used in mammals and have the desired biological activity. They can be prepared separately during the final separation and purification of the compound, or by reacting a suitable group with a suitable base or acid. Bases commonly used to form pharmaceutically acceptable salts include inorganic bases such as sodium hydroxide and potassium hydroxide, and organic bases such as ammonia. Acids commonly used to form pharmaceutically acceptable salts include both inorganic and organic acids.

In the context of a drug or a pharmacological active agent, the term "therapeutically effective amount" refers to the amount of the drug or agent sufficient to achieve or at least partially achieve the desired effect. The determination of the therapeutically effective amount varies from person to person, depending on the age and general condition of the recipient, as well as the specific active substance. The appropriate therapeutically effective amount in a particular case can be determined by those skilled in the art through routine experiments.

The term "pharmaceutically acceptable" as used herein means that these compounds, materials, compositions and/or dosage forms, within the scope of reasonable medical judgment, are suitable for contact with the tissues of patients without excessive toxicity, irritation, allergic reaction or other problems or complications, have a reasonable benefit/risk ratio, and are effective for the intended use.

As used herein, the singular forms "a", "an" and "the" include plural references and vice versa, unless the context clearly indicates otherwise.

When the term "about" is applied to parameters such as pH, concentration, temperature, etc., it indicates that the parameter can vary by ±10%, and sometimes more preferably within ±5%. As those skilled in the art will understand, when the parameters are not critical, numbers are usually given for illustrative purposes only and are not for limitation.

### The synthetic methods of the compounds disclosed in present disclosure

To achieve the objectives of the present disclosure, the following technical solutions are adopted in the present disclosure:

### Scheme 1

A method for preparing the compound of formula (I) of the present disclosure or pharmaceutically acceptable salt thereof includes the following steps:

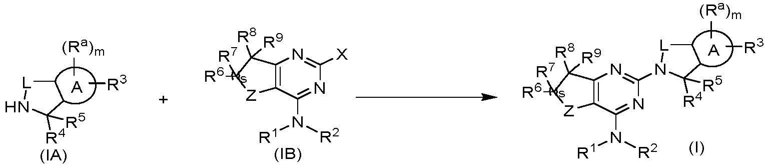

Subjecting a compound of formula (IA) or a salt thereof to a nucleophilic substitution reaction under microwave conditions with a compound of formula (IB) or a salt thereof, to obtain a compound of formula (I) or a pharmaceutically acceptable salt thereof,
where:
X is a halogen, preferably Cl;
ring A, L, Z, R^{a}, R¹ to R⁹, m and s are defined as in the formula (I).

### Scheme 2

A method for preparing the compound of formula (II) of present disclosure or pharmaceutically acceptable salt thereof includes the following steps:

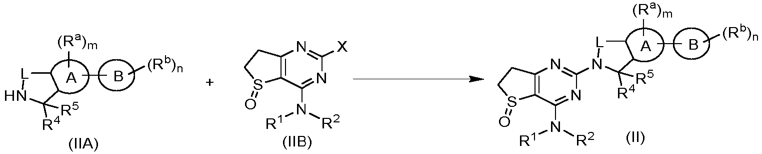

Subjecting a compound of formula (IIA) or a salt thereof to a nucleophilic substitution reaction under microwave conditions with a compound of formula (IIB) or a salt thereof, to obtain a compound of formula (II) or a pharmaceutically acceptable salt thereof;
where:
X is a halogen, preferably Cl;
Ring A, Ring B, L, R^{a}, R^{b}, R¹, R², R⁴, R⁵, m and n are defined as in formula (II).

### Scheme 3

A method for preparing the compound of formula (II-1) of present disclosure or pharmaceutically acceptable salt thereof includes the following steps:

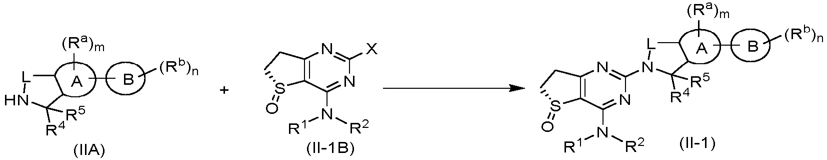

Subjecting a compound of formula (IIA) or a salt thereof to a nucleophilic substitution reaction under microwave conditions with a compound of formula (II-1B) or a salt thereof, to obtain a compound of formula (II-1) or a pharmaceutically acceptable salt thereof;
X represents a halogen, preferably Cl.

Ring A, Ring B, L, R^{a}, R^{b}, R¹, R², R⁴, R⁵, m and n are defined as in formula (II-1).

### Scheme 4

A method for preparing the compound of formula (II-2) of present disclosure or pharmaceutically acceptable salt thereof includes the following steps:

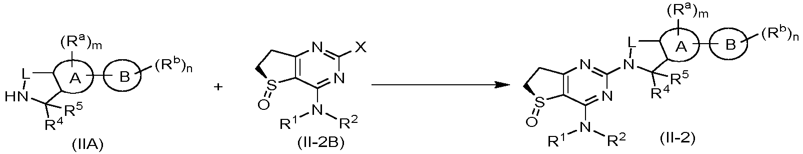

Subjecting a compound of formula (IIA) or a salt thereof to a nucleophilic substitution reaction under microwave conditions with a compound of formula (II-2B) or a salt thereof, to obtain a compound of formula (II-2) or a pharmaceutically acceptable salt thereof;
X is a halogen, preferably Cl.

Ring A, Ring B, L, R^{a}, R^{b}, R¹, R², R⁴, R⁵, m and n are defined as in formula (II-2).

### Scheme 5

A method for preparing the compound of formula (III) of present disclosure or pharmaceutically acceptable salt thereof includes the following steps:

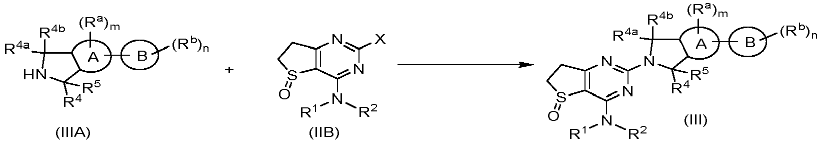

Subjecting a compound of formula (IIIA) or a salt thereof to a nucleophilic substitution reaction under microwave conditions with a compound of formula (IIB) or a salt thereof, to obtain a compound of formula (III) or a pharmaceutically acceptable salt thereof;
X is a halogen, preferably Cl.

Ring A, Ring B, R^{a}, R^{b}, R¹, R², R⁴, R⁵, R^{4a}, R^{4b}, m and n are defined as in formula (III).

### Scheme 6

A method for preparing the compound of formula (III-1) of present disclosure or pharmaceutically acceptable salt thereof includes the following steps:

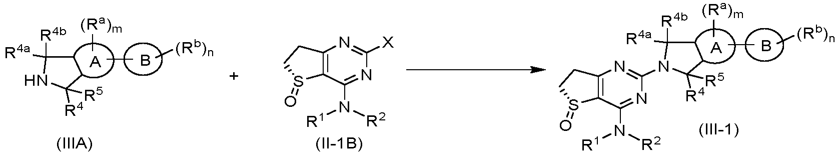

Subjecting a compound of formula (IIIA) or a salt thereof to a nucleophilic substitution reaction under microwave conditions with a compound of formula (II-1B) or a salt thereof, to obtain a compound of formula (III-1) or a pharmaceutically acceptable salt thereof;
X is a halogen, preferably Cl.

Ring A, Ring B, R^{a}, R^{b}, R¹, R², R⁴, R⁵, R^{4a}, R^{4b}, m and n are defined as in formula (III-1).

### Scheme 7

A method for preparing the compound of formula (III-2) of present disclosure or pharmaceutically acceptable salt thereof includes the following steps:

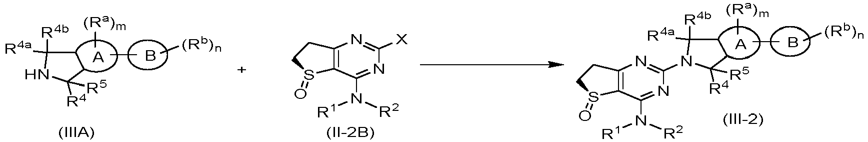

Subjecting a compound of formula (IIIA) or a salt thereof to a nucleophilic substitution reaction under microwave conditions with a compound of formula (II-2B) or a salt thereof, to obtain a compound of formula (III-2) or a pharmaceutically acceptable salt thereof;
X is a halogen, preferably Cl.

Ring A, Ring B, R^{a}, R^{b}, R¹, R², R⁴, R⁵, R^{4a}, R^{4b}, m and n are defined as in formula (III-2).

### Scheme 8

A method for preparing the compound of formula (IV) of present disclosure or pharmaceutically acceptable salt thereof includes the following steps:

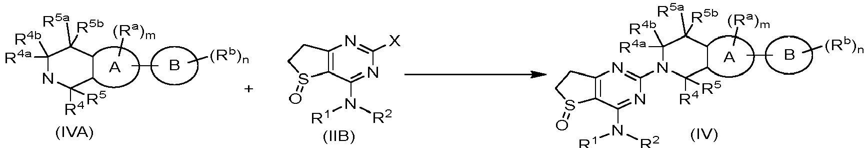

Subjecting a compound of formula (IVA) or a salt thereof to a nucleophilic substitution reaction under microwave conditions with a compound of formula (IIB) or a salt thereof, to obtain a compound of formula (IV) or a pharmaceutically acceptable salt thereof;
X is a halogen, preferably Cl.

Ring A, Ring B, R^{a}, R^{b}, R¹, R², R⁴, R⁵, R^{4a}, R^{4b}, R^{5a}, R^{5b}, m and n are defined as in formula (IV).

### Scheme 9

A method for preparing the compound of formula (IV-1) of present disclosure or pharmaceutically acceptable salt thereof includes the following steps:

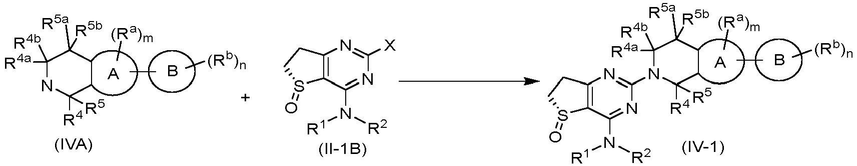

Subjecting a compound of formula (IVA) or a salt thereof to a nucleophilic substitution reaction under microwave conditions with a compound of formula (II-1B) or a salt thereof, to obtain a compound of formula (IV-1) or a pharmaceutically acceptable salt thereof;
X is a halogen, preferably Cl.

Ring A, Ring B, R^{a}, R^{b}, R¹, R², R⁴, R⁵, R^{4a}, R^{4b}, R^{5a}, R^{5b}, m and n are defined as in formula (IV-1).

### Scheme 10

A method for preparing the compound of formula (IV-2) of present disclosure or pharmaceutically acceptable salt thereof includes the following steps:

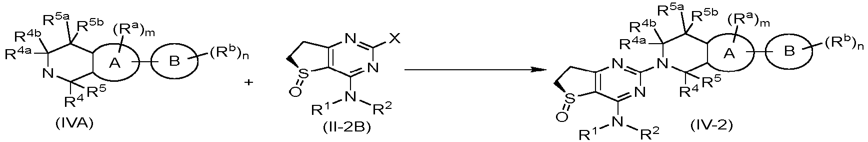

Subjecting a compound of formula (IVA) or a salt thereof to a nucleophilic substitution reaction under microwave conditions with a compound of formula (II-2B) or a salt thereof, to obtain a compound of formula (IV-2) or a pharmaceutically acceptable salt thereof;
X is a halogen, preferably Cl.

Ring A, Ring B, R^{a}, R^{b}, R¹, R², R⁴, R⁵, R^{4a}, R^{4b}, R^{5a}, R^{5b}, m and n are defined as in formula (IV-2).

In the above synthetic schemes, the reagents providing the basic conditions include organic bases and inorganic bases. The organic bases include, but are not limited to, triethylamine, pyridine, N,N - diisopropylethylamine, n-butyllithium, lithium diisopropylamide, sodium acetate, potassium acetate, sodium tert-butoxide, potassium tert-butoxide or 1,8-diazabicycloundec-7-ene. The inorganic bases include, but are not limited to, sodium hydride, potassium phosphate, sodium carbonate, potassium carbonate, cesium carbonate, cadmium carbonate, sodium hydroxide, lithium hydroxide monohydrate, lithium hydroxide and potassium hydroxide. Preferably, the reagent for the basic conditions is N,N - diisopropylethylamine.

The reaction in the above steps is preferably carried out in a solvent. The solvents used include, but are not limited to: pyridine, ethylene glycol dimethyl ether, acetic acid, methanol, ethanol, acetonitrile, n - butanol, toluene, tetrahydrofuran, dichloromethane, petroleum ether, ethyl acetate, n-hexane, dimethyl sulfoxide, 1,4-dioxane, water, N,N-dimethylformamide, N,N-dimethylacetamide, 1,2-dibromoethane and mixtures thereof.

### DETAILED DESCRIPTION

The following examples are provided to further describe the present disclosure, but these examples are not intended to limit the scope of present disclosure.

### EMBODIMENTS

The structure of the compound was determined by NMR spectra obtained using a Varian 400MHz nuclear magnetic resonance spectrometer. Commonly, CDCl₃ or DMSO-d₆ was used as the solvent, and the chemical shifts were reported in ppm. The descriptions of various peaks are as follows: s (singlet), d (doublet), t (triplet), q (quartet), m (multiplet), dd (doublet of doublets). The coupling constant is expressed in Hz.

The LC-MS (liquid chromatography-mass spectrometry) was performed using a Waters ACQUITY Arc equipped with a QDa Detector. The mass spectrometry (MS) employed an ESI source, which only indicates the molecular weight M of the parent molecule, and usually reported as [M + H]⁺. The injection volume was determined according to the sample concentration. The flow rate was 0.8 mL/min. The HPLC peaks were recorded and read at UV-Vis wavelengths of 220 nm and 254 nm. The mobile phases were an ultrapure aqueous solution of 0.01% formic acid (mobile phase A) and an acetonitrile solution of 0.01% formic acid (mobile phase B).

Chiral HPLC analysis was carried out using a SHIMADZU LC-30AD SFC high-performance liquid chromatograph.

For thin-layer chromatography, Yantai Huanghai HSGF254 or Qingdao GF254 silica gel plates were used. The silica gel plates for thin-layer chromatography (TLC) had a thickness of 0.15 mm-0.2mm, and those for separating and purifying products by TLC had a thickness of 0.4 mm-0.5 mm.

For silica gel column chromatography, 200-300 mesh silica gel from Yantai Huanghai was generally used as the carrier.

The known starting materials of the present disclosure can be synthesized by methods known in the art or purchased from companies such as ABCR GmbH & Co. KG, Acros Organics, Aldrich Chemical Company, Accela ChemBio Inc (Shaoyuan Technology (Shanghai) Co., Ltd.), and Darui Chemicals.

Unless otherwise specified in the examples, the reactions were carried out under an argon or nitrogen atmosphere.

An argon or nitrogen atmosphere means that the reaction flask was connected to an argon or nitrogen balloon with a volume of approximately 1 L.

A hydrogen atmosphere means that the reaction flask was connected to a hydrogen balloon with a volume of approximately 1 L.

For hydrogenation reactions, the system was usually evacuated and filled with hydrogen, and this operation was repeated three times.

The microwave reactions were carried out using a Biotage microwave reactor.

Unless otherwise specified in the examples, "solution" refers to an aqueous solution.

Unless otherwise specified in the examples, the reaction temperature was room temperature, which is 20°C - 30°C.

The progress of the reactions in the examples was monitored by thin-layer chromatography (TLC). The developing agents used for the reactions, the eluent systems for column chromatography in purifying compounds, and the developing agent systems for TLC included: A: n - hexane/ethyl acetate system, B: dichloromethane/methanol system, in which the volume ratio of the solvents was adjusted according to the polarity of the compounds, and a small amount of basic or acidic reagents such as triethylamine and acetic acid could also be added for adjustment.

The abbreviations used in present disclosure are as follows:
EDCI: 1-ethyl-3-(3-dimethylaminopropyl)carbodiimide hydrochloride
HOBT: 1-hydroxybenzotriazole
TEA: Triethylamine
DCM: Dichloromethane
DMF: N,N-Dimethylformamide
DMF-DMA: N,N-dimethylformamide dimethyl acetal
LHMDS: Lithium bis(trimethylsilyl)amide
TMSI: Trimethylsilyl iodide
THF: Tetrahydrofuran

### Example 1

### (R)-2-(2-(4-chlorophenyl)-6,7-dihydrooxazolo[4,5-c]pyridin-5(4H)-yl)-4-((1-(hydroxymethyl) cyclobutyl)amino) -6,7-dihydrothieno[3,2-d]pyrimidine 5-oxide 1

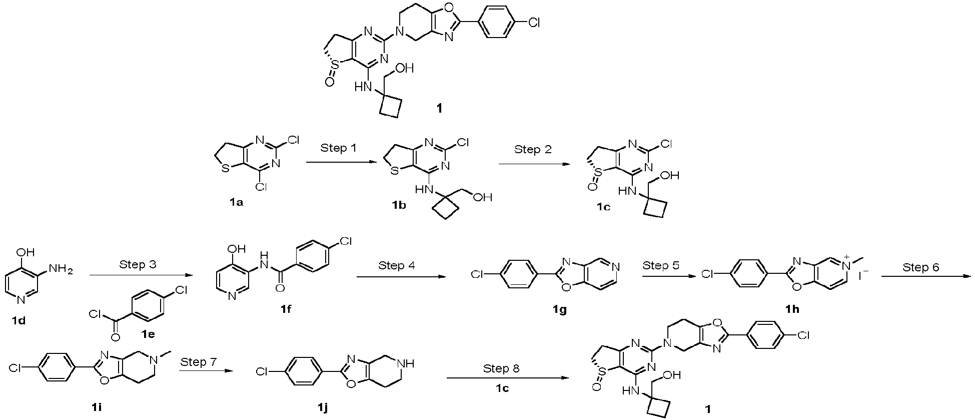

### Step 1

### (1-((2-chloro-6,7-dihydrothieno[3,2-d]pyrimidin-4-yl)amino)cyclobutyl)methanol 1b

2,4-dichloro-6,7-dihydrothieno[3,2-d] 1a (15.0g, 72.8mmol), 1-aminocyclobutanemethylamine hydrochloride (11.0g, 80.1mmol) and triethylamine (22.1g, 218.4 mmol) were mixed in 1,4-dioxane (100mL) at room temperature. The reaction mixture was stirred at 80 °C for 8 hours. After the reaction was completed, the solvent was removed by concentration under reduced pressure. Subsequently, water (100mL) was added, and the mixture was extracted with dichloromethane (200 mL×2). The combined organic phases were concentrated under reduced pressure to obtain the crude product that was purified by flash column chromatography to obtain the title product 1b (15.2 g).

### Step 2

### (R)-2-chloro-4-((1-(hydroxymethyl)cyclobutyl)amino)-6,7-dihydrothieno[3,2-d]pyrimidine 5-oxide 1c

The mixture of compound 1b (15.2g, 58.1mmol), S-1,1'-bi-2-naphthol (1.66g,5.81mmol), titanium tetraisopropoxide (0.82g, 2.9mmol) and water (1.05g, 58.1 mmol) mixed in dichloromethane (100 mL) under nitrogen protection was stirred at room temperature for 1 hour. Subsequently, tert-butyl hydroperoxide (5.75 g, 63.9 mmol) was added and the reaction mixture was heated to 40 °C and stirred for 5 hours. After the reaction was completed, the solid was obtained by filtration and triturated with methanol to obtain the title product 1c (16.7 g, ee value: 97.4%).

### Step 3

### 4-chloro-N-(4-hydroxypyridin-3-yl)benzamide 1f

Under nitrogen protection, triethylamine (2.75 g, 27.3 mmol) was added to a dichloromethane (30 mL) solution of 3-aminopyridin-4-ol 1d (2.0 g, 18.2 mmol). The mixture was cooled to 0 °C in an ice bath. Then, a dichloromethane (5 mL) solution of 4-chlorobenzoyl chloride 1e (3.3 g, 19.1 mmol) was slowly added dropwise. After stirring at room temperature for two hours, the mixture was filtered. The filter cake was washed with water and dried to obtain the title product 1f (2.5 g).

### Step 4

### 2-(4-chlorophenyl)oxazolo[4,5-c]pyridine 1g

At room temperature, triphenylphosphine (6.7 g, 24.3 mmol) and triethylamine (6.5 g, 64.8 mmol) were added to a dichloromethane solution (30 mL) of hexachloroethane (4.7 g, 20.2 mmol). The mixture was stirred at room temperature for 10 minutes, and then compound 1f (2 g, 8.1 mmol) was added in portions. The mixture was stirred at room temperature for 5 hours. The reaction was quenched with ammonium chloride solution and extracted with dichloromethane (100 mL×2). The organic phase was washed with saturated brine (100 mL), dried over anhydrous sodium sulfate, filtered, and concentrated under reduced pressure to obtain the crude product. The crude product was purified by flash column chromatography to obtain the title product 1g (1.3 g).

### Step 5

### 2-(4-chlorophenyl)-5-methyl-oxazolo[4,5-c]pyridin-5-ium 1h

At room temperature, iodomethane (0.9 g, 6.5 mmol) was added to a DMF (10 mL) solution of compound 1g (1.0 g, 4.3 mmol). The mixture was stirred at room temperature for 5 h, and the reaction solution was concentrated to obtain the title product **1h** (1.1 g).

### Step 6

### 2-(4-chlorophenyl)-5-methyl-4,5,6,7-tetrahydrooxazolo[4,5-c]pyridine 1i

Under an ice bath, sodium borohydride (225 mg, 5.9 mmol) was added in portions to a methanol (30 mL) solution of compound **1h** (1.1 g, 2.9 mmol). After the reaction solution was stirred at room temperature for 3 hours, it was quenched with water (20 mL), concentrated until methanol was completely removed, and then extracted with ethyl acetate (30 mL×3). The combined organic phases were washed with saturated brine (50 mL), dried over anhydrous sodium sulfate, filtered, and concentrated under reduced pressure to obtain the crude product. The crude product was purified by flash column chromatography to obtain the title product **1i** (700 mg, yield 21.7%).

### Step 7

### 2-(4-chlorophenyl)-4,5,6,7-tetrahydrooxazolo[4,5-c]pyridine 1j

At 0°C, under a nitrogen atmosphere, 1-chloroethyl chloroformate (343.5 mg, 2.4 mmol) was added to a 1,2-dichloroethane solution (8 mL) of compound **1i** (400 mg, 1.6 mmol). The reaction solution was stirred at 80 °C for 2 hours. Subsequently, the mixture was cooled to room temperature and concentrated to dryness. Then, a methanol (6 mL) solution was added to the mixture and stirred for another 2 h. The mixture was concentrated under reduced pressure to obtain the crude product. The crude product was purified by flash column chromatography to obtain the title product **1j** (350 mg).

### Step 8

### (R)-2-(2-(4-chlorophenyl)-6,7-dihydrooxazolo[4,5-c]pyridin-5(4H)-yl)-4-((1-(hydroxymethyl)cyclobutyl)amino)-6,7-dihydrothieno[3,2-d]pyrimidine 5-oxide 1

Diisopropylethylamine (108 mg, 0.84 mmol) was added to a 1,4-dioxane solution (4 mL) of compound **1j** (50 mg, 0.21 mmol) and compound **1c** (55 mg, 0.19 mmol). The reaction solution was stirred at 120 °C for 40 min under microwave conditions. Ethyl acetate (30 mL) was added to the reaction solution. The organic phase was washed successively with saturated sodium chloride (20 mL) and saturated brine (20 mL), dried over anhydrous sodium sulfate, filtered, and concentrated under reduced pressure to obtain the crude product. The crude product was purified by flash column chromatography to obtain the title product 1 (20 mg, yield: 21.7%).

¹H NMR (400 MHz, DMSO-*d₆*): δ 7.98 (d, *J* = 8.0 Hz, 2 H), 7.61 (d, *J* = 8.0 Hz, 2 H), 7.49 (s, 1 H), 4.87 (t, *J* = 8.0 Hz, 1 H), 4.79 (br. s, 2 H), 4.18 (br. s, 2 H), 3.78 (d, *J* = 4.0 Hz, 2 H), 3.43-3.51 (m, 1 H), 3.22-3.29 (m, 1 H), 2.96-3.02 (m, 2 H), 2.89 (t, *J* = 7.6 Hz, 2 H), 2.31-2.44 (m, 2 H), 2.22-2.27 (m, 2 H), 1.78-1.86 (m, 2 H).

MS m/z (ESI): 486 [M+1].

### Example 2

### (R)-2-(2-(4-Fluorophenyl)-6,7-dihydrooxazolo[4,5-c]pyridin-5(4H)-yl)-4-((1-(hydroxymethyl)cyclobutyl)amino)-6,7-dihydrothieno[3,2-d]pyrimidine 5-oxide 2

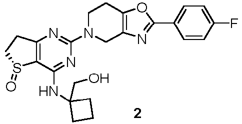

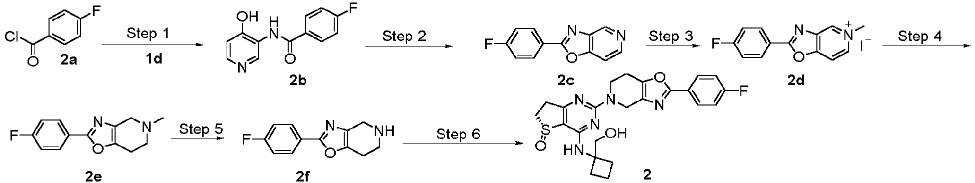

### Step 1

### 4-fluoro-N-(4-hydroxypyridin-3-yl)benzamide 2b

Under nitrogen protection, triethylamine (2.75 g, 27.3 mmol) was added to a solution of 3-aminopyridin-4-ol **1d** (2.0 g, 18.2 mmol) in dichloromethane (30 mL). The mixture was cooled to 0 °C in an ice bath. Then, a solution of 4-fluorobenzoyl chloride **2a** (3.0 g, 19.1 mmol) in dichloromethane (5 mL) was slowly added dropwise. After stirring at room temperature for two hours, the reaction was quenched with ammonium chloride solution and extracted with dichloromethane (100 mL×2). The organic phase was washed with saturated brine (100 mL), dried over anhydrous sodium sulfate, filtered, and concentrated under reduced pressure to obtain the crude product. The crude product was purified by flash column chromatography to obtain the title product **2b** (3.7 g).

### Step 2

### 2-(4-fluorophenyl)oxazolo[4,5-c]pyridine 2c

At room temperature, triphenylphosphine (6.8 g, 25.8 mmol) and triethylamine (6.9 g, 68.8 mmol) were added to a solution of hexachloroethane (5.1 g, 20.2 mmol) in dichloromethane (30 mL). The mixture was stirred at room temperature for 10 minutes. Then, compound **2b** (2.0 g, 8.6 mmol) was added in portions, and the mixture was stirred at room temperature for 5 hours. The reaction was quenched with ammonium chloride solution and extracted with dichloromethane (100 mL×2). The organic phase was washed with saturated brine (100 mL), dried over anhydrous sodium sulfate, filtered, and concentrated under reduced pressure to obtain the crude product. The crude product was purified by flash column chromatography to obtain the title product **2c** (1.2 g).

### Step 3

### 2-(4-fluorophenyl)-5-methyloxazolo[4,5-c]pyridin-5-ium 2d

At room temperature, methyl iodide (1.0 g, 7.0 mmol) was added to a solution of compound **2c** (1.0 g, 4.7 mmol) in DMF (10 mL). The mixture was stirred at room temperature for 5 h, and the reaction solution was concentrated to obtain the title product **2d** (1.1 g).

### Step 4

### 2-(4-fluorophenyl)-5-methyl-4,5,6,7-tetrahydrooxazolo[4,5-c]pyridine 2e

Under an ice bath, sodium borohydride (230 mg, 6.2 mmol) was added in portions to a solution of compound **2d** (1.1 g, 3.1 mmol) in methanol (30 mL). After the reaction solution was stirred at room temperature for 3 hours, it was quenched with water (20 mL). The solution was concentrated until the methanol was removed, and then extracted with ethyl acetate (30 mL×3). The combined organic phases were washed with saturated brine (50 mL), dried over anhydrous sodium sulfate, filtered, and concentrated under reduced pressure to obtain the crude product. The crude product was purified by flash column chromatography to give the title product **2e** (930 mg).

### Step 5

### 2-(4-fluorophenyl)-4,5,6,7-tetrahydrooxazolo[4,5-c]pyridine 2f

At 0 °C, under a nitrogen atmosphere, 1-chloroethyl chloroformate (370 mg, 2.6 mmol) was added to a solution of compound **2e** (400 mg, 1.7 mmol) in dichloroethane (8 mL). The reaction solution was stirred at 80 °C for 2 hours. Then, the mixture was cooled to room temperature and concentrated to dryness. Methanol (6 mL) was added to the mixture and stirring was continued for 2 h. The mixture was concentrated under reduced pressure to obtain the crude product. The crude product was purified by flash column chromatography to give the title product **2f** (380 mg).

### Step 6

### (R)-2-(2-(4-fluorophenyl)-6,7-dihydrooxazolo[4,5-c]pyridin-5(4H)-yl)-4-((1-(hydroxymethyl)cyclobutyl)amino)-6,7-dihydrothieno[3,2-d]pyrimidine 5-oxide 2

Diisopropylethylamine (108 mg, 0.84 mmol) was added to a solution of compound **2f** (45 mg, 0.21 mmol) and compound **1c** (55 mg, 0.19 mmol) in 1,4-dioxane (4 mL). The reaction solution was stirred under microwave conditions at 120°C for 40 min. Ethyl acetate (30 mL) was added to the reaction solution. The organic phase was washed successively with saturated sodium chloride solution (20 mL) and saturated brine (20 mL), dried over anhydrous sodium sulfate, filtered, and concentrated under reduced pressure to obtain the crude product. The crude product was purified by flash column chromatography to give the title product **2** (25 mg, yield: 28.1%).

¹H NMR (400 MHz, DMSO-*d₆*): δ 8.03 (dd, *J* = 16.0, 8.0 Hz, 2 H), 7.54 (s, 1 H), 7.39 (t, *J* = 8.0 Hz, 2 H), 4.89 (t, *J* = 4.0 Hz, 1 H), 4.79 (br. s, 2 H), 4.18 (br. s, 2 H), 3.78 (d, *J =* 4.0 Hz, 2 H), 3.42-3.53 (m, 1 H), 3.21-3.30 (m, 1 H), 2.95-3.04 (m, 1 H), 2.89 (br. s, 3 H), 2.30-2.43 (m, 2 H), 2.22-2.29 (m, 2 H), 1.76-1.89 (m, 2 H).
MS m/z (ESI): 470 [M+1].

### Example 3

### (R)-4-(5-(4-((1-(hydroxymethyl)cyclobutyl)amino)-5-oxido-6,7-dihydrothieno[3,2-d]pyrimidin-2-yl)-4,5,6,7-tetrahydrooxazolo[4,5-c]pyridin-2-yl)benzonitrile 3

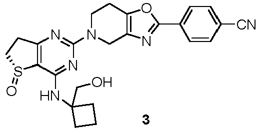

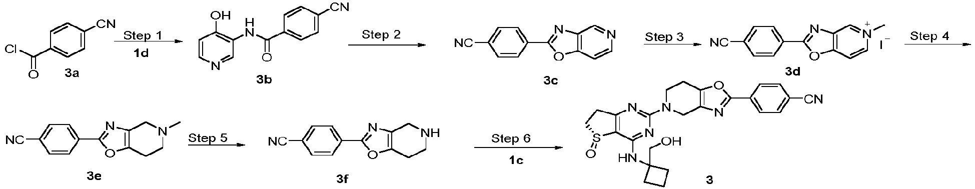

### Step 1

### 4-cyano-N-(4-hydroxypyridin-3-yl)benzamide 3b

Under nitrogen protection, triethylamine (2.75 g, 27.3 mmol) was added to a solution of 3-aminopyridin-4-ol **1d** (2.0 g, 18.2 mmol) in dichloromethane (30 mL). The mixture was cooled to 0 °C in an ice bath. Then, a solution of 4-cyanobenzoyl chloride **3a** (3.2 g, 19.1 mmol) in dichloromethane (5 mL) was slowly added dropwise. After stirring at room temperature for two hours, the reaction was quenched with ammonium chloride solution and extracted with dichloromethane (100 mL × 2). The organic phase was washed with saturated brine (100 mL), dried over anhydrous sodium sulfate, filtered, and concentrated under reduced pressure to obtain the crude product. The crude product was purified by flash column chromatography to give the title product **3b** (2.8 g).

### Step 2

### 4-(oxazolo[4,5-c]pyridin-2-yl)benzonitrile 3c

At room temperature, triphenylphosphine (6.8 g, 25.8 mmol) and triethylamine (6.9 g, 68.8 mmol) were added to a solution of hexachloroethane (5.1 g, 20.2 mmol) in dichloromethane (30 mL). The mixture was stirred at room temperature for 10 minutes. Then, compound **3b** (2.0 g, 8.6 mmol) was added in portions, and the mixture was stirred at room temperature for 5 hours. The reaction was quenched with ammonium chloride solution and extracted with dichloromethane (100 mL × 2). The organic phase was washed with saturated brine (100 mL), dried over anhydrous sodium sulfate, filtered, and concentrated under reduced pressure to obtain the crude product. The crude product was purified by flash column chromatography to give the title product **3c** (1.1 g).

### Step 3

### 2-(4-cyanophenyl)-5-methyloxazolo[4,5-c]pyridin-5-ium 3d

At room temperature, methyl iodide (0.97 g, 6.8 mmol) was added to a solution of compound **3c** (1.0 g, 4.5 mmol) in DMF (10 mL). The mixture was stirred at room temperature for 5 h, and the reaction solution was concentrated to give the title product **3d** (1.0 g).

### Step 4

### 2-(4-cyanophenyl)-5-methyl-4,5,6,7-tetrahydrooxazolo[4,5-c]pyridine 3e

Under an ice bath, sodium borohydride (200 mg, 5.5 mmol) was added in portions to a solution of compound **3d** (1.0 g, 2.8 mmol) in methanol (30 mL). After the reaction solution was stirred at room temperature for 3 hours, it was quenched with water (20 mL), concentrated until the methanol was evaporated, and then extracted with ethyl acetate (30 mL×3). The combined organic phases were washed with saturated brine (50 mL), dried over anhydrous sodium sulfate, filtered, and concentrated under reduced pressure to obtain the crude product. The crude product was purified by flash column chromatography to give the title product **3e** (200 mg).

### Step 5

### 2-(4-cyanophenyl)-4,5,6,7-tetrahydrooxazolo[4,5-c]pyridine 3f

At 0 °C, under a nitrogen atmosphere, 1-chloroethyl chloroformate (250 mg, 1.8 mmol) was added to a solution of compound **3e** (200 mg, 0.9 mmol) in dichloroethane (8 mL). The reaction solution was stirred at 80°C for 2 hours. Then, the mixture was cooled to room temperature and concentrated to dryness. Methanol (6 mL) was added to the mixture and stirring was continued for 2 h. The mixture was concentrated under reduced pressure to obtain the crude product. The crude product was purified by flash column chromatography to give the title product **3f** (180 mg).

### Step 6

### (R)-4-(5-(4-((1-(hydroxymethyl)cyclobutyl)amino)-5-oxido-6,7-dihydrothieno[3,2-d]pyrimidin-2-yl)-4,5,6,7-tetrahydrooxazolo[4,5-c]pyridin-2-yl)benzonitrile 3

Diisopropylethylamine (108 mg, 0.84 mmol) was added to a solution of compound **3f** (47 mg, 0.21 mmol) and compound 1c (55 mg, 0.19 mmol) in 1,4-dioxane (4 mL). The reaction solution was stirred under microwave conditions at 120°C for 40 min. Ethyl acetate (30 mL) was added to the reaction solution. The organic phase was washed successively with saturated sodium chloride solution (20 mL) and saturated brine (20 mL), dried over anhydrous sodium sulfate, filtered, and concentrated under reduced pressure to obtain the crude product. The crude product was purified by flash column chromatography to give the title product **3** (20 mg, yield: 22.2%).

¹H NMR (400 MHz, DMSO-*d₆*): δ 8.14 (m, 4 H), 7.61 (d, *J* = 8.0 Hz, 2 H), 7.54 (s, 1 H), 4.89 (t, *J* = 4.0 Hz, 1 H), 4.83 (br. s, 2 H), 4.19 (br. s, 2 H), 3.78 (d, *J* = 4.0 Hz, 2 H), 3.43-3.53 (m, 1 H), 3.21-3.30 (m, 1 H), 2.96-3.04 (m, 1 H), 2.87-2.95 (m, 3 H), 2.30-2.45 (m, 2 H), 2.20-2.29 (m, 2 H), 1.79-1.89 (m, 2 H)
MS m/z (ESI): 477 [M+1]

### Example 4

### (R)-4-((1-(Hydroxymethyl)cyclobutyl)amino)-2-(2-(3-(2-methoxyethoxy)phenyl)-6,7-dihydrooxazolo[4,5-c]pyridin-5(4H)-yl)-6,7-dihydrothieno[3,2-d]pyrimidine 5-oxide 4

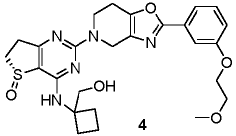

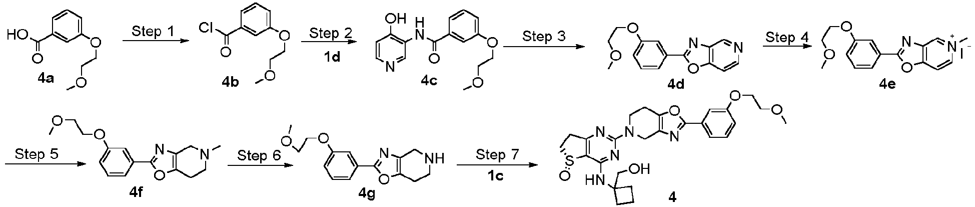

### Step 1

### 3-(2-Methoxyethoxy)benzoyl chloride 4b

Under nitrogen protection, DMF (37 mg, 0.5 mmol) was added to a solution of 3-(2-methoxyethoxy)benzoic acid **4a** (1.0 g, 5.1 mmol) in dichloromethane (10 mL). The mixture was cooled to 0 °C in an ice bath. Then, a solution of oxalyl chloride (771 mg, 6.1 mmol) in dichloromethane (3 mL) was slowly added dropwise. After stirring at room temperature for two hours, the mixture was concentrated to give the title product **4b** (1.1 g).

### Step 2

### N-(4-hydroxypyridin-3-yl)-3-(2-methoxyethoxy)benzamide 4c

The title product **4c** (700 mg) was prepared from compound **4b** (1.1 g, 5.1 mmol) according to the method described in step 3 of Example 1.

### Step 3

### 2-(3-(2-methoxyethoxy)phenyl)oxazolo[4,5-c]pyridine 4d

The title product **4d** (600 mg) was prepared from compound **4c** (700 mg, 2.4 mmol) according to the method described in Step 4 of Example 1 at room temperature.

### Step 4

### 2-(3-(2-methoxyethoxy)phenyl)-5-methyloxazolo[4,5-c]pyridin-5-ium 4e

The title product **4e** (900 mg) was prepared from compound **4d** (600 mg, 2.2 mmol) according to the method described in step 5 of Example 1 at room temperature.

### Step 5

### 2-(3-(2-methoxyethoxy)phenyl)-5-methyl-4,5,6,7-tetrahydrooxazolo[4,5-c]pyridine 4f

The title product **4f** (500 mg) was prepared from compound **4e** (900 mg, 2.2 mmol) according to the method described in Step 6 of Example 1 at 0 °C.

### Step 6

### 2-(3-(2-methoxyethoxy)phenyl)-4,5,6,7-tetrahydrooxazolo[4,5-c]pyridine 4g

The title product **4g** (400 mg) was prepared from compound **4e** (500 mg, 1.7 mmol) according to the method described in step 7 of Example 1 at room temperature.

### Step 7

### (R)-4-((1-(hydroxymethyl)cyclobutyl)amino)-2-(2-(3-(2-methoxyethoxy)phenyl)-6,7-dihydrooxazolo[4,5-c]pyridin-5(4H)-yl)-6,7-dihydrothieno[3,2-d]pyrimidine 5-oxide 4

The title product **4** (20 mg, yield: 23.8%) was prepared from compound **4g** (50 mg, 0.18 mmol) and compound **1c** (47 mg, 0.16 mmol) according to the method described in step 8 of Example 1.

¹H NMR (400 MHz, DMSO-*d₆*): δ 7.55 (m, 2 H), 7.45 (m, 2 H), 7.11 (dd, *J* = 2.0 Hz, 8.0 Hz, 1 H), 4.90 (t, *J =* 5.6 Hz, 1 H), 4.78 (br. s, 2 H), 4.18-4.21 (m, 2 H), 3.78 (d, *J* = 5.2 Hz, 2 H), 3.70 (t, *J* = 4.8 Hz, 2 H), 3.45-3.51 (m, 1 H), 3.22-3.35 (m, 4 H), 2.92-3.02 (m, 1 H), 2.88-2.89 (m, 4 H), 2.31-2.38 (m, 2 H), 2.24-2.27 (m, 2 H), 1.80-1.84 (m, 2 H)
MS m/z (ESI): 526 [M+1]

### Example 5

### (R)-2-(2-cyclohexyl-6,7-dihydrooxazolo[4,5-c]pyridin-5(4H)-yl)-4-((1-(hydroxymethyl)cyclobutyl) amino)-6,7-dihydrothieno[3,2-d]pyrimidine 5-oxide 5

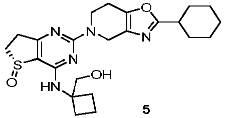

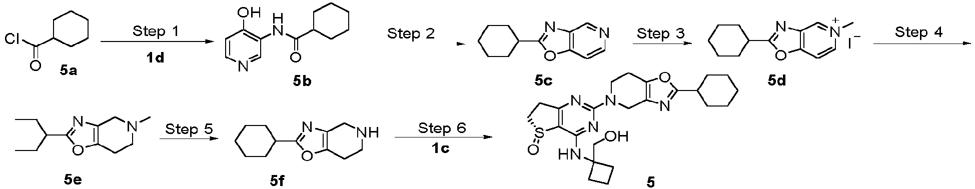

### Step 1

### N-(4-hydroxypyridin-3-yl)cyclohexanecarboxamide 5b

Under nitrogen protection, triethylamine (2.75 g, 27.3 mmol) was added to a solution of 3-aminopyridin-4-ol 1d (2.0 g, 18.2 mmol) in dichloromethane (30 mL). The mixture was cooled to 0 °C in an ice bath. Then, a solution of cyclohexanecarbonyl chloride 5a (2.8 g, 19.1 mmol) in dichloromethane (5 mL) was slowly added dropwise. After stirring at room temperature for two hours, the reaction was quenched with ammonium chloride solution and extracted with dichloromethane (100 mL×2). The organic phase was washed with saturated brine (100 mL), dried over anhydrous sodium sulfate, filtered, and concentrated under reduced pressure to obtain the crude product. The crude product was purified by flash column chromatography to give the title product **5b** (2.8 g).

### Step 2

### 2-cyclohexyloxazolo[4,5-c]pyridine 5c

At room temperature, triphenylphosphine (7.2 g, 27.3 mmol) and triethylamine (7.4 g, 72.8 mmol) were added to a solution of hexachloroethane (5.4 g, 22.5 mmol) in dichloromethane (30 mL). The mixture was stirred at room temperature for 10 minutes. Then, compound **5b** (2.0 g, 9.1 mmol) was added in portions, and the mixture was stirred at room temperature for 5 hours. The reaction was quenched with ammonium chloride solution and extracted with dichloromethane (100 mL×2). The organic phase was washed with saturated brine (100 mL), dried over anhydrous sodium sulfate, filtered, and concentrated under reduced pressure to obtain the crude product. The crude product was purified by flash column chromatography to give the title product **5c** (0.8 g).

### Step 3

### 2-cyclohexyl-5-methyloxazolo[4,5-c]pyridin-5-ium 5d

At room temperature, methyl iodide (0.85 g, 6.0 mmol) was added to a solution of compound **5c** (0.8 g, 4.0 mmol) in DMF (10 mL). The mixture was stirred at room temperature for 5 h, and the reaction solution was concentrated to obtain the title product **5d** (0.9 g).

### Step 4

### 2-cyclohexyl-5-methyl-4,5,6,7-tetrahydrooxazolo[4,5-c]pyridine 5e

Under an ice bath, sodium borohydride (190 mg, 5.2 mmol) was added in portions to a solution of compound **5d** (0.9 g, 2.6 mmol) in methanol (30 mL). After the reaction solution was stirred at room temperature for 3 hours, it was quenched with water (20 mL), concentrated until the methanol was evaporated, and extracted with ethyl acetate (30 mL×3). The combined organic phases were washed with saturated brine (50 mL), dried over anhydrous sodium sulfate, filtered, and concentrated under reduced pressure to obtain the crude product. The crude product was purified by flash column chromatography to give the title product **5e** (700 mg).

### Step 5

### 2-cyclohexyl-4,5,6,7-tetrahydrooxazolo[4,5-c]pyridine 5f

At 0 °C, under a nitrogen atmosphere, 1-chloroethyl chloroformate (910 mg, 6.4 mmol) was added to a solution of compound **5e** (700 mg, 3.2 mmol) in dichloroethane (8 mL). The reaction solution was stirred at 80°C for 2 hours. Then, the mixture was cooled to room temperature and concentrated to dryness. Methanol (6 mL) was added to the mixture and stirring was continued for 2 h. The mixture was concentrated under reduced pressure to obtain the crude product. The crude product was purified by flash column chromatography to give the title product **5f** (280 mg).

### Step 6

### (R)-2-(2-cyclohexyl-6,7-dihydrooxazolo[4,5-c]pyridin-5(4H)-yl)-4-((1-(hydroxymethyl)cyclobutyl)amino)-6,7-dihydrothieno[3,2-d]pyrimidine 5-oxide 5

Diisopropylethylamine (108 mg, 0.84 mmol) was added to a solution of compound **5f** (44 mg, 0.21 mmol) and compound **1c** (55 mg, 0.19 mmol) in 1,4-dioxane (4 mL). The reaction solution was stirred under microwave conditions at 120°C for 40 min. Ethyl acetate (30 mL) was added to the reaction solution. The organic phase was washed successively with saturated sodium chloride solution (20 mL) and saturated brine (20 mL), dried over anhydrous sodium sulfate, filtered, and concentrated under reduced pressure to obtain the crude product. The crude product was purified by flash column chromatography to give the title product **5** (16 mg, yield: 18.4%).

¹H NMR (400 MHz, DMSO-*d₆*): δ 7.49 (s, 1 H), 4.88 (t, *J* = 8.0 Hz, 1 H), 4.66 (s, 2 H), 4.11 (s, 2 H), 3.77 (d, *J* = 8.0 Hz, 2 H), 3.40-3.50 (m, 1 H), 3.19-3.29 (m, 1 H), 2.78-3.01 (m, 2 H), 2.70-2.77 (m, 2 H), 2.28-2.44 (m, 2 H), 2.18-2.26 (m, 2 H), 1.80-1.98 (m, 4 H), 1.62-1.70 (m, 1H) 1.44-1.56 (m, 2 H), 1.22-1.43 (m, 4H)
MS m/z (ESI): 458 [M+1]

### Example 6

### (R)-2-(2-(4-chlorophenyl)-6,7-dihydrothiazolo[4,5-c]pyridin-5(4H)-yl)-4-((1-(hydroxymethyl)cyclobutyl)amino)-6,7-dihydrothieno[3,2-d]pyrimidine 5-oxide 6

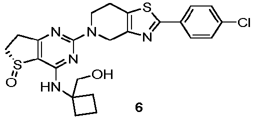

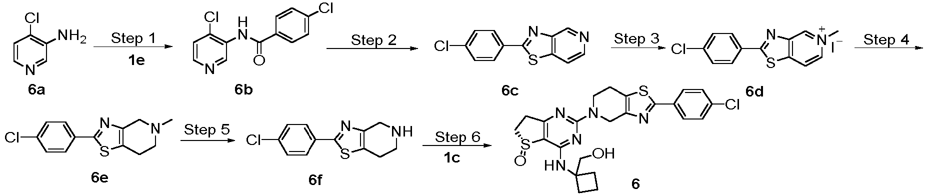

### Step 1

### 4-chloro-N-(4-chloropyridin-3-yl)benzamide 6b

Under nitrogen protection, a solution of 4-chloropyridin-3-amine 6a (2.0 g, 15.6 mmol) in dichloromethane (30 mL) was cooled to 0 °C in an ice bath. Then, a solution of 4-chlorobenzoyl chloride 1e (3.0 g, 17.2 mmol) in dichloromethane (5 mL) was slowly added dropwise. After stirring at room temperature for two hours, the reaction was quenched with ammonium chloride solution and extracted with dichloromethane (100 mL×2). The organic phase was washed with saturated brine (100 mL), dried over anhydrous sodium sulfate, filtered, and concentrated under reduced pressure to obtain the crude product. The crude product was purified by flash column chromatography to give the title product **6b** (2.1 g).

### Step 2

### 2-(4-chlorophenyl)thiazolo[4,5-c]pyridine 6c

At room temperature, compound **6b** (2.0 g, 7.5 mmol) was added to a solution of Lawesson's reagent (2.1 g, 5.3 mmol) in xylene (50 mL). The mixture was heated to 120°C and stirred for 5 hours. The solvent was removed by rotary evaporation. Sodium hypochlorite solution was added, and the mixture was extracted with dichloromethane (100 mL×2). The organic phase was washed with saturated brine (100 mL), dried over anhydrous sodium sulfate, filtered, and concentrated under reduced pressure to obtain the crude product. The crude product was purified by flash column chromatography to give the title product 6c (1.1 g).

### Step 3

### 2-(4-chlorophenyl)-5-methythiazolo[4,5-c]pyridin-5-ium 6d

At room temperature, methyl iodide (0.9 g, 6.3 mmol) was added to a solution of compound **6c** (1.1 g, 4.2 mmol) in DMF (10 mL). The mixture was stirred at room temperature for 5 h, and the reaction solution was concentrated to obtain the title product **6d** (1.2 g).

### Step 4

### 2-(4-chlorophenyl)-5-methyl-4,5,6,7-tetrahydrothiazolo[4,5-c]pyridine 6e

Under an ice bath, sodium borohydride (230 mg, 6.2 mmol) was added in portions to a solution of compound **6d** (1.2 g, 3.1 mmol) in methanol (30 mL). After the reaction solution was stirred at room temperature for 3 hours, it was quenched with water (20 mL). The solution was concentrated until the methanol was evaporated, and then extracted with ethyl acetate (30 mL×3). The combined organic phases were washed with saturated brine (50 mL), dried over anhydrous sodium sulfate, filtered, and concentrated under reduced pressure to obtain the crude product. The crude product was purified by flash column chromatography to give the title product **6e** (800 mg).

### Step 5

### 2-(4-chlorophenyl)-4,5,6,7-tetrahydrothiazolo[4,5-c]pyridine 6f

At 0°C, under a nitrogen atmosphere, 1-chloroethyl chloroformate (860 mg, 6.0 mmol) was added to a solution of compound **6e** (800 mg, 3.0 mmol) in dichloroethane (12 mL). The reaction solution was stirred at 80°C for 2 hours. Then, the mixture was cooled to room temperature and concentrated to dryness. Methanol (6 mL) was added to the mixture and stirring was continued for 2 h. The mixture was concentrated under reduced pressure to obtain the crude product. The crude product was purified by flash column chromatography to give the title product **6f** (350 mg).

### Step 6

### (R)-2-(2-(4-chlorophenyl)-6,7-dihydrothiazolo[4,5-c]pyridin-5(4H)-yl)-4-((1-(hydroxymethyl)cyclobutyl)amino)-6,7-dihydrothieno[3,2-d]pyrimidine 5-oxide 6

Diisopropylethylamine (108 mg, 0.84 mmol) was added to a solution of compound **6f** (53 mg, 0.21 mmol) and compound **1c** (55 mg, 0.19 mmol) in 1,4-dioxane (4 mL). The reaction solution was stirred under microwave conditions at 120°C for 40 min. Ethyl acetate (30 mL) was added to the reaction solution. The organic phase was washed successively with saturated sodium chloride solution (20 mL) and saturated brine (20 mL), dried over anhydrous sodium sulfate, filtered, and concentrated under reduced pressure to obtain the crude product. The crude product was purified by flash column chromatography to give the title product **6** (16 mg, yield: 16.8%).

¹H NMR (400 MHz, DMSO-*d₆*): δ 7.93 (d, *J* = 8.0 Hz, 2 H), 7.57 (d, *J* = 8.0 Hz, 2 H), 7.51 (s, 1 H), 4.98 (br. s, 2 H), 4.89 (t, *J* = 8.0 Hz, 1 H), 4.15 (br. s, 2 H), 3.78 (d, *J* = 4.0 Hz, 2 H), 3.43-3.52 (m, 1 H), 3.21-3.29 (m, 1 H), 2.86-3.05 (m, 4 H), 2.33-2.46 (m, 2 H), 2.20-2.29 (m, 2 H), 1.77-1.89 (m, 2 H)
MS m/z (ESI): 503 [M+1]

### Example 7

### (R)-2-(2-(4-chlorophenyl)-4,6-dihydro-5H-pyrrolo[3,4-d]oxazol-5-yl)-4-((1-(hydroxymethyl)cyclobutyl)amino)-6,7-dihydrothieno[3,2-d]pyrimidine 5-oxide 7

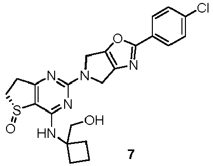

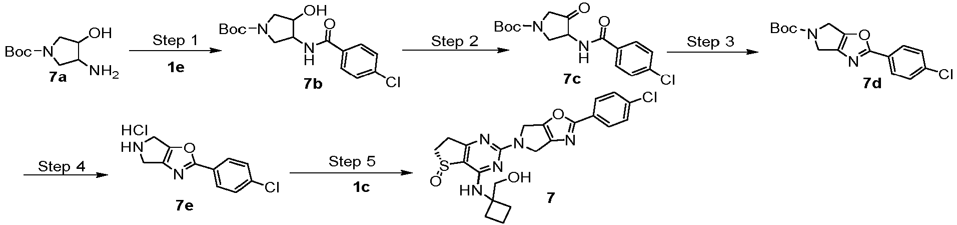

### Step 1

### tert-butyl 3-amino-4-hydroxypyrrolidine-1-carboxylate 7b

Under nitrogen protection, a solution of tert-butyl 3-amino-4-hydroxypyrrolidine-1-carboxylate 7a (0.6 g, 3.0 mmol) in dichloromethane (20 mL) was cooled to 0 °C in an ice bath. Then, a solution of 4 - chlorobenzoyl chloride (0.57 g, 3.3 mmol) in dichloromethane (3 mL) was slowly added dropwise. After stirring at room temperature for two hours, the reaction was quenched with ammonium chloride solution and extracted with dichloromethane (50 mL×2). The organic phase was washed with saturated brine (50 mL), dried over anhydrous sodium sulfate, filtered, and concentrated under reduced pressure to obtain the crude product. The crude product was purified by flash column chromatography to give the title product **7b** (0.56 g).

### Step 2

### tert-butyl 3-(4-chlorobenzamido)-4-oxopyrrolidine-1-carboxylate 7c

At 0 °C, Dess-Martin periodinane (1.4 g, 3.3 mmol) was added to a solution of compound **7b** (0.56 g, 1.6 mmol) in dichloromethane (50 mL). After stirring at room temperature for two hours, the reaction was quenched with sodium thiosulfate solution and extracted with dichloromethane (50 mL×2). The organic phase was washed with saturated brine (50 mL), dried over anhydrous sodium sulfate, filtered, and concentrated under reduced pressure to obtain the crude product. The crude product was purified by flash column chromatography to give the title product **7c** (0.41 g).

### Step 3

### tert-butyl 2 -(4 -chlorophenyl)-4,6-dihydro-5H-pyrrolo[3,4-d]oxazole-5-carboxylate 7d

At room temperature, triphenylphosphine (0.94 g, 3.6 mmol) and triethylamine (0.97 g, 9.6 mmol) were added to a solution of hexachloroethane (0.71 g, 3.0 mmol) in dichloromethane (30 mL). The mixture was stirred at room temperature for 10 minutes. Then, compound 7c (0.41 g, 1.2 mmol) was added in portions, and the mixture was stirred at room temperature for 5 hours. The reaction was quenched with ammonium chloride solution and extracted with dichloromethane (50 mL×2). The organic phase was washed with saturated brine (50 mL), dried over anhydrous sodium sulfate, filtered, and concentrated under reduced pressure to obtain the crude product. The crude product was purified by flash column chromatography to give the title product 7d (0.19 g).

### Step 4

### 2-(4-chlorophenyl)-5,6-dihydro-4H-pyrrolo[3,4-d]oxazole hydrochloride 7e

At room temperature, compound **7d** (0.19 g, 3.1 mmol) was stirred in a solution of hydrochloric acid in 1,4 -dioxane solution (5 mL) for 1 hour. The solution was concentrated until the solvent was evaporated to obtain the title product **7e** (150 mg).

### Step 5

### (R)-2-(2-(4-chlorophenyl)-4,6-dihydro-5H-pyrrolo[3,4-d]oxazol-5-yl)-4-((1-(hydroxymethyl)cyclobutyl)amino)-6,7-dihydrothieno[3,2 -d]pyrimidine 5-oxide 7

Diisopropylethylamine (108 mg, 0.84 mmol) was added to a solution of compound **7e** (54 mg, 0.21 mmol) and compound **1c** (55 mg, 0.19 mmol) in 1,4 -dioxane (4 mL). The reaction solution was stirred under microwave conditions at 120°C for 40 min. Ethyl acetate (30 mL) was added to the reaction solution. The organic phase was washed successively with saturated sodium chloride solution (20 mL) and saturated brine (20 mL), dried over anhydrous sodium sulfate, filtered, and concentrated under reduced pressure to obtain the crude product. The crude product was purified by flash column chromatography to give the title product **7** (11 mg, yield: 12.3%).

¹H NMR (400 MHz, DMSO-*d₆*): δ 8.00-8.05 (m, 2 H), 7.63-7.68 (m, 2 H), 7.51 (s, 1 H), 4.85-4.91 (m, 1 H), 4.74-4.81 (m, 2 H), 4.56-4.62 (m, 2 H), 3.79 (d, *J* = 8.0 Hz, 2 H), 3.46-3.53 (m, 1 H), 3.23-3.32 (m, 1 H), 2.98-3.08 (m, 1 H), 2.89-2.96 (m, 1 H), 2.34-2.48 (m, 2 H), 2.21-2.30 (m, 2 H), 1.76-1.89 (m, 2 H)
MS m/z (ESI): 472 [M+1]

### Example 8

### (R)-2-(4-((2-(2-(4-Chlorophenyl)-6,7-dihydrooxazolo[4,5-c]pyridin-5(4H)-yl)-5-oxido-6,7-dihydrothieno[3,2-d]pyrimidin-4-yl)amino)phenyl)acetic acid 8

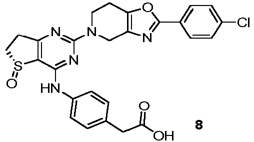

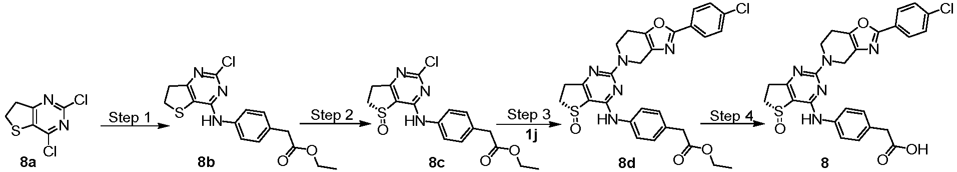

### Step 1

### Ethyl 2-(4-((2-chloro-6,7-dihydrothieno[3,2-d]pyrimidin-4-yl)amino)phenyl)acetate 8b

At room temperature, 2,4-dichloro-6,7-dihydrothieno[3,2-d]pyrimidine **8a** (1.0 g, 4.9 mmol), ethyl 2-(4-aminophenyl)acetate (0.96 g, 5.4 mmol) and triethylamine (0.99 g, 9.8 mmol) were mixed in ethanol (15 mL). The reaction solution was stirred at 80 °C for 5 hours. After the reaction was completed, the solvent was removed by rotary evaporation. Then, water (30 mL) was added, and the mixture was extracted with dichloromethane (30 mL×2). The combined organic phases were concentrated under reduced pressure to obtain the crude product. The crude product was purified by flash column chromatography to give the title product **8b** (800 mg).

### Step 2

### Ethyl (R)-2-(4-((2-chloro-5-oxido-6,7-dihydrothieno[3,2-d]pyrimidin-4-yl)amino)phenyl)acetate 8c

The title product **8c** (500 mg) was prepared from compound **8b** (800 mg, 2.3 mmol) according to the method described in step 2 of Example 1 at room temperature.

### Step 3

### Ethyl (R)-2-(4-((2-(2-(4-chlorophenyl)-6,7-dihydrooxazolo[4,5-c]pyridin-5(4H)-yl)-5-oxido-6,7-dihydrothieno[3,2-d]pyrimidin-4-yl)amino)phenyl)acetate 8d

The title product **8d** (60 mg) was prepared from compound **8c** (100 mg, 0.27 mmol) and compound **1j** (66 mg, 0.24 mmol) according to the method described in step 8 of Example 1.

### Step 4

### (R)-2-(4-((2-(2-(4-Chlorophenyl)-6,7-dihydrooxazolo[4,5-c]pyridin-5(4H)-yl)-5-oxido-6,7-dihydrothieno[3,2-d]pyrimidin-4-yl)amino)phenyl)acetic acid 8

Sodium hydroxide (18 mg, 0.44 mmol) was added to a solution of compound **8d** (60 mg, 0.11 mmol) in methanol (6 mL)/water (2 mL). The reaction solution was stirred at room temperature for 2 hours. After the methanol was removed by concentration, dilute hydrochloric acid (2 M) was added to the reaction solution to adjust the pH to 2-3. The mixture was filtered, and the filter cake was washed with ethyl acetate and dried to obtain the title product **8** (30 mg, yield: 50.8%).

¹H NMR (400 MHz, DMSO-*d₆*): δ 9.66 (s, 1 H), 7.97 (d, *J* = 8.4 Hz, 2 H), 7.61-7.65 (m, 4 H), 7.28 (d, *J=* 8.4 Hz, 2 H), 4.81 (br. s, 2 H), 4.21 (br. s, 2 H), 3.60 (s, 2 H), 3.55-3.57 (m, 1 H), 3.28-3.30 (m, 1 H), 2.99-3.14 (m, 2 H), 2.90-2.92 (m, 2 H).

MS m/z (ESI): 536 [M+1]

### Example 9

### (R)-2-(6-(5-chloropyrimidin-2-yl)-3,4-dihydroisoquinolin-2(1H)-yl)-4-((1-(hydroxymethyl)cyclobutyl)amino)-6,7-dihydrothieno[3,2-d]pyrimidine 5-oxide 9

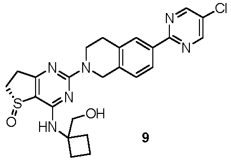

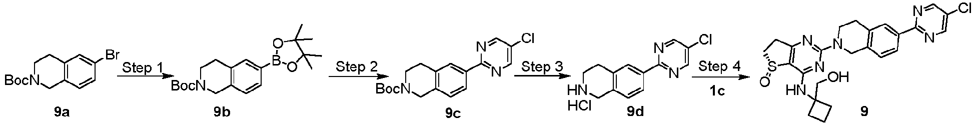

### Step 1

### tert-butyl 6-(4,4,5,5-tetramethyl-1,3,2-dioxaborolan-2-yl)-3,4-dihydroisoquinoline-2(1H)-carboxylate 9b

Under nitrogen protection, tert-butyl 6-bromo-3,4-dihydroisoquinoline-2(1H)-carboxylate **9a** (1.0 g, 3.2 mmol), bis(pinacolato)diboron (0.9 g, 3.5 mmol), potassium acetate (0.47 mg, 4.8 mmol) and [1,1'-bis(diphenylphosphino)ferrocene]palladium(II) dichloride dichloromethane complex (250 mg, 0.3 mmol) were mixed in 1,4-dioxane (15 mL). The reaction mixture was stirred at 90°C for 3 hours. After the reaction, the solvent was removed by rotary evaporation. Then, water (30 mL) was added, and the mixture was extracted with ethyl acetate (30 mL×2). The combined organic phases were concentrated under reduced pressure to obtain the crude product of the title product **9b** (1.2 g).

### Step 2

### tert-butyl 6-(5-chloropyrimidin-2-yl)-3,4-dihydroisoquinoline-2(1H)-carboxylate 9c

Compound **9a** (500 mg, 1.4 mmol), 2-bromo-5-chloropyrimidine (322 mg, 1.7 mmol), potassium carbonate (309 mg, 2.2 mmol) and [1,1'-bis(diphenylphosphino)ferrocene]palladium(II) dichloride dichloromethane complex (81 mg, 0.1 mmol) were mixed in a solution of 1,4-dioxane (15 mL)/water (3 mL) under nitrogen protection. The reaction mixture was stirred at 80 °C for 3 hours. Ethyl acetate (30 mL) was added to the reaction mixture. The organic phase was washed successively with saturated sodium chloride solution (20 mL) and saturated brine (20 mL), dried over anhydrous sodium sulfate, filtered, and concentrated under reduced pressure to obtain the crude product. The crude product was purified by flash column chromatography to give the title product **9c** (300 mg).

### Step 3

### 6-(5-chloropyrimidin-2-yl)-1,2,3,4-tetrahydroisoquinoline hydrochloride 9d

A solution of hydrogen chloride in 1,4-dioxane (4 M, 2 mL) was added to a solution of compound 9c (300 mg, 0.87 mmol) in dichloromethane. The reaction mixture was stirred at room temperature for 2 h and then concentrated to dryness to obtain the title product 9d (330 mg).

### Step 4

### (R)-2-(6-(5-chloropyrimidin-2-yl)-3,4-dihydroisoquinolin-2(1H)-yl)-4-((1-(hydroxymethyl)cyclobutyl)amino)-6,7-dihydrothieno[3,2-d]pyrimidine 5-oxide 9

The title product 9 (22 mg, yield: 27.8%) was prepared from compound **9d** (50 mg, 0.18 mmol) and compound **1c** (47 mg, 0.16 mmol) according to the method described in step 8 of Example 1.

¹H NMR (400 MHz, DMSO-*d₆*): δ 9.01 (s, 2 H), 8.20 (d, *J =* 7.2 Hz, 2 H), 7.38-7.42 (m, 2 H), 4.98 (br. s, 2 H), 4.88 (t, *J* = 5.6 Hz, 2H), 4.04 (t, *J=* 5.6 Hz, 2 H), 3.78 (d, *J=* 5.6 Hz, 2 H), 3.44-3.49 (m, 1 H), 3.22-3.26 (m, 1 H), 2.85-2.95 (m, 4 H), 2.36-2.41 (m, 2 H), 2.22-2.26 (m, 2 H), 1.80-1.87 (m, 2 H)
MS m/z (ESI): 497 [M+1]

### Example 10

### (R)-2-(6-(4-fluoropiperidin-1-yl)-3,4-dihydroisoquinolin-2(1H)-yl)-4-((1-(hydroxymethyl)cyclobutyl)amino)-6,7-dihydrothieno[3,2-d]pyrimidine 5-oxide 10

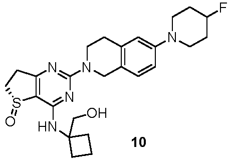

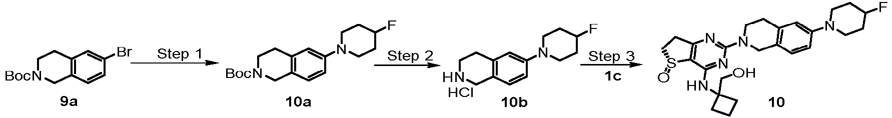

### Step 1

### tert-butyl 6-(4-fluoropiperidin-1-yl)-3,4-dihydroisoquinoline-2(1H)-carboxylate 10a

Under nitrogen protection, tert-butyl 6-bromo-3,4-dihydroisoquinoline-2(1H)-carboxylate **9a** (1.0 g, 3.2 mmol), 4-fluoropiperidine hydrochloride (0.54 g, 3.8 mmol), sodium tert-butoxide (0.70 g, 7.1 mmol), tris(dibenzylideneacetone)dipalladium(0) (293 mg, 0.32 mmol) and 1,1'-binaphthalene-2,2'-bis(diphenylphosphine) (206 mg, 0.32 mmol) were mixed in toluene (15 mL). The reaction mixture was stirred at 110 °C for 6 hours. After the reaction, the solvent was removed by rotary evaporation. Then, water (30 mL) was added, and the mixture was extracted with ethyl acetate (30 mL×2). The combined organic phases were concentrated under reduced pressure to obtain the crude product. The crude product was purified by flash column chromatography to give the title product **10a** (400 mg).

### Step 2

### 6-(4-fluoropiperidin-1-yl)-1,2,3,4-tetrahydroisoquinoline hydrochloride 10b

A solution of hydrogen chloride in 1,4-dioxane (4 M, 2 mL) was added to a solution of compound 10a (400 mg, 1.2 mmol) in dichloromethane. The reaction mixture was stirred at room temperature for 2 h and then concentrated to dryness to obtain the title product **10b** (300 mg).

### Step 3

### (R)-2-(6-(4-fluoropiperidin-1-yl)-3,4-dihydroisoquinolin-2(1H)-yl)-4-((1-(hydroxymethyl) cyclobutyl)amino)-6,7-dihydrothieno[3,2-d]pyrimidine 5-oxide 10

The title product **10** (22 mg, yield: 28.2%) was prepared from compound **10b** (50 mg, 0.18 mmol) and compound **1c** (47 mg, 0.16 mmol) according to the method described in step 8 of Example 1.

¹H NMR (400 MHz, DMSO-*d₆*): δ 7.35 (s, 1 H), 7.06 (d, *J* = 7.6 Hz, 1 H), 6.85 (dd, *J* = 2.0 Hz, 8.0 Hz, 1 H), 6.78 (s, 1 H), 4.90-4.93 (m, 2 H), 4.77-4.79 (m, 2 H), 3.94 (br. s, 2 H), 3.78 (d, *J =* 5.6 Hz, 2 H), 3.44-3.49 (m, 1 H), 3.22-3.26 (m, 1 H), 3.09-3.15 (m, 1 H), 2.85-2.95 (m, 2 H), 2.81 (t, *J* = 5.6 Hz, 2 H), 2.35-2.40 (m, 2 H), 2.22-2.33 (m, 2 H), 2.35-2.40 (m, 2 H),1.90-2.08 (m, 2 H), 1.79-1.82 (m, 4 H)
MS m/z (ESI): 486 [M+1]

### Example 11

### (R)-2-(3-(4-fluorophenyl)-1,4,6,7-tetrahydro-5H-pyrazolo[4,3-c]pyridin-5-yl)-4-((1-(hydroxymethyl)cyclobutyl)amino)-6,7-dihydrothieno[3,2-d]pyrimidine 5-oxide 11

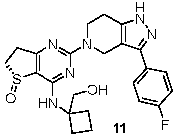

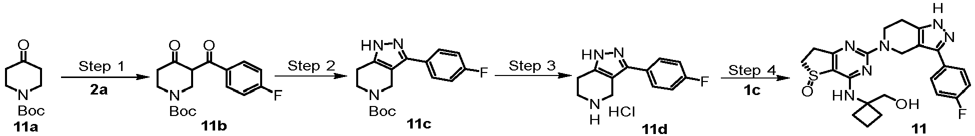

### Step 1

### tert-butyl 3-(4-fluorobenzoyl)-4-oxopiperidine-1-carboxylate 11b

Under nitrogen protection, LHMDS (10 mL, 1 M) was slowly added dropwise to a solution of tert -butyl 4-oxopiperidine-1-carboxylate (2.0 g, 10.0 mmol) in tetrahydrofuran (30 mL) while maintaining the temperature at -30 °C. The reaction mixture was stirred at this temperature for 30 min. Then, a solution of 4-fluorobenzoyl chloride (1.7 g, 10.0 mmol) in tetrahydrofuran (5 mL) was slowly added dropwise to the reaction mixture. The reaction mixture was further stirred at this temperature for 3 h. The reaction was quenched by the addition of saturated ammonium chloride solution (40 mL), and the mixture was extracted with ethyl acetate (40 mL×2). The combined organic phases were concentrated under reduced pressure to obtain the crude product of the title product **11b** (3 g).

### Step 2

### tert-butyl 3-(4-fluorophenyl)-1,4,6,7-tetrahydro-5H-pyrazolo[4,3-c]pyridine-5-carboxylate 11c

Compound 11b (3 g, 9.3 mmol) and hydrazine hydrate (0.75 g, 18.7 mmol, 80% in H₂O) were dissolved in ethanol (30 mL), and the reaction was carried out at 80 °C for 3 h. The reaction mixture was cooled to room temperature, and most of the ethanol was removed by concentration. Water (30 mL) was added, and the mixture was extracted with ethyl acetate (40 mL×2). The organic phase was washed successively with saturated brine (20 mL), dried over anhydrous sodium sulfate, filtered, and concentrated under reduced pressure to obtain the crude product. The crude product was purified by flash column chromatography to give the title product **11c** (400 mg).

### Step 3

### 3-(4-fluorophenyl)-4,5,6,7-tetrahydro-1H-pyrazolo[4,3-c]pyridine hydrochloride 11d

A solution of hydrogen chloride in 1,4-dioxane (4 M, 2 mL) was added to a solution of compound 11c (400 mg, 1.2 mmol) in dichloromethane. The reaction mixture was stirred at room temperature for 2 h and then concentrated to dryness to obtain the title product **11d** (450 mg).

### Step 4

### (R)-2-(3-(4-fluorophenyl)-1,4,6,7-tetrahydro-5H-pyrazolo[4,3-c]pyridin-5-yl)-4-((1-(hydroxymethyl)cyclobutyl)amino)-6,7-dihydrothieno[3,2-d]pyrimidine 5-oxide 11

The title product 11 (10 mg, yield: 11.9%) was prepared from compound **11d** (50 mg, 0.20 mmol) and compound **1c** (51 mg, 0.18 mmol) according to the method described in step 8 of Example 1.

¹H NMR (400 MHz, DMSO-*d₆*): δ 7.68-7.69 (m, 2 H), 7.36-7.43 (m, 2 H), 4.85-5.05 (m, 3 H), 4.1 (br. s, 2 H), 3.65-3.79 (m, 2 H), 3.44-3.49 (m, 1 H), 3.22-3.26 (m, 1 H), 2.77-2.96 (m, 4 H), 2.35-2.41 (m, 2 H), 2.22-2.33 (m, 2 H), 1.80-1.85 (m, 2 H)
MS m/z (ESI): 469 [M+1]

### Example 12

### (R)-4-((1-(hydroxymethyl)cyclobutyl)amino)-2-(1-phenyl-1,4,6,7-tetrahydro-5H-pyrazolo[4,3-c]pyridin-5-yl)-6,7-dihydrothieno[3,2-d]pyrimidine-5-oxide 12

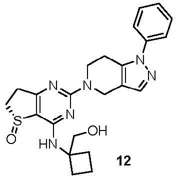

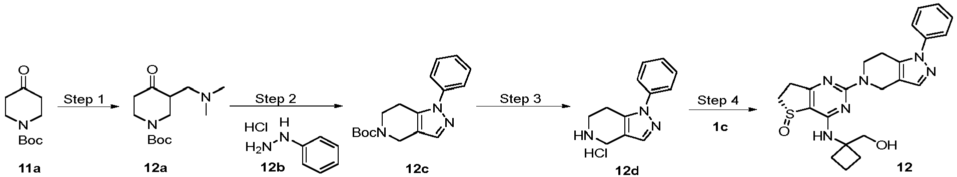

### Step 1

### tert-butyl 3-((dimethylamino)methyl)-4-oxopiperidine-1-carboxylate 12a

Under nitrogen protection, DMF-DMA (1.2 g, 10.0 mmol) was added to a solution of tert-butyl 4-oxopiperidine-1-carboxylate **11a** (2.0 g, 10.0 mmol) in DMF (20 mL). The reaction mixture was stirred at 80 °C for 4 h. Then the reaction mixture was concentrated, added with saturated ammonium chloride solution (40 mL), and extracted with ethyl acetate (40 mL×2). The combined organic phases were concentrated under reduced pressure to obtain the title product **12a** (2.2 g).

### Step 2

tert-butyl 1-phenyl-1,4,6,7-tetrahydro-5H-pyrazolo[4,3-c]pyridine-5-carboxylate **12c** Compound **12a** (2.1 g, 9.3 mmol) was dissolved in methanol (100 mL) and water (50 mL). Then sodium bicarbonate (0.53 g, 5.0 mmol) and phenylhydrazine hydrochloride **12b** (1.43 g, 9.9 mmol) were added, and finally acetic acid (1 mL) was added. The mixture was stirred at room temperature for 1 h. Then the reaction mixture was neutralized with saturated sodium bicarbonate solution and concentrated until the methanol was evaporated completely. The mixture was extracted with dichloromethane (50 mL × 2). The organic phase was washed with saturated brine (40 mL), dried over anhydrous sodium sulfate, filtered, and concentrated under reduced pressure to obtain the crude product. The crude product was purified by flash column chromatography to give the title product **12c** (1.3 g).

### Step 3

### 1-phenyl-4,5,6,7-tetrahydro-1H-pyrazolo[4,3-c]pyridine hydrochloride 12d

A solution of hydrogen chloride in 1,4-dioxane (4 M, 2 mL) was added to a solution of compound **12c** (200 mg, 1.2 mmol) in dichloromethane. The reaction mixture was stirred at room temperature for 2 h and then concentrated to dryness to obtain the title product **12d** (250 mg).

### Step 4

### (R)-4-((1-(hydroxymethyl)cyclobutyl)amino)-2-(1-phenyl-1,4,6,7-tetrahydro-5H-pyrazolo[4,3-c]pyridin-5-yl)-6,7-dihydrothieno[3,2-d]pyrimidine-5-oxide 12

The title product **12** (20 mg, yield: 24.7%) was prepared from compound **12d** (50 mg, 0.21 mmol) and compound **1c** (51 mg, 0.18 mmol) according to the method described in step 8 of Example 1.

¹H NMR (400 MHz, DMSO-*d₆*): δ 7.66 (s, 1 H), 7.60 (d, *J =* 7.6 Hz, 2 H), 7.53 (t, *J =* 8.0 Hz, 2 H), 7.50 (s, 1 H), 7.40 (d, *J=* 7.6 Hz, 1 H), 4.89 (t, *J=* 5.6 Hz, 1 H), 4.82-4.85 (m, 2 H), 4.06 (br. s, 2 H), 3.78 (d, *J=* 5.6 Hz, 2 H), 3.44-3.49 (m, 1 H), 3.22-3.27 (m, 1 H), 2.90-3.00 (m, 4 H), 2.23-2.40 (m, 2 H), 2.22-2.26 (m, 2 H), 1.80-1.88 (m, 2 H)
MS m/z (ESI): 451 [M+1]

### Example 13

### (R)-2-(2-(4-fluorophenyl)-6,7-dihydrothiazolo[5,4-c]pyridin-5(4H)-yl)-4-((1-(hydroxymethyl)cyclobutyl)amino)-6,7-dihydrothieno[3,2 - d]pyrimidine 5-oxide 13

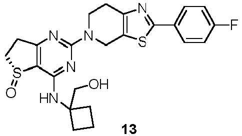

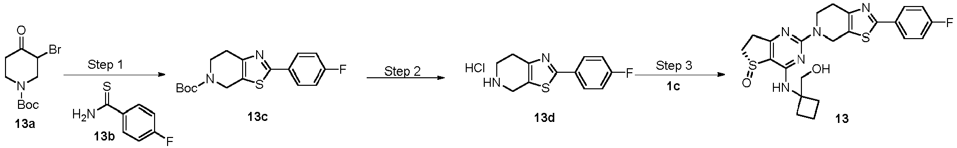

### Step 1

### tert-butyl 2-(4-fluorophenyl)-6,7-dihydrothiazolo[5,4-c]pyridine-5(4H)-carboxylate 13c

At room temperature, tert-butyl 3-bromo-4-oxopiperidine-1-carboxylate **13a** (1.0 g, 3.6 mmol) and 4-fluorobenzothioamide **13b** (0.56 g, 3.6 mmol) were mixed in isopropanol (20 mL). The mixture was stirred at 60 °C for 5 hours and then filtered. The filter cake was washed with isopropanol and dried to obtain the title product **13c** (0.54 g).

### Step 2

### 2-(4-fluorophenyl)-4,5,6,7-tetrahydrothiazolo[5,4-c]pyridine hydrochloride 13d

At room temperature, compound **13c** (0.54 g, 1.6 mmol) was stirred in a solution of hydrochloric acid in 1,4-dioxane solution (8 mL) for 1 hour. The solution was concentrated until the solvent was evaporated over to obtain the title product **13d** (0.48 g).

### Step 3

### (R)-2-(2-(4-fluorophenyl)-6,7-dihydrothiazolo[5,4-c]pyridin-5(4H)-yl)-4-((1-(hydroxymethyl)cyclobutyl)amino)-6,7-dihydrothieno[3,2 - d]pyrimidine 5-oxide 13

Diisopropylethylamine (108 mg, 0.84 mmol) was added to a solution of compound **13d** (57 mg, 0.21 mmol) and compound **1c** (55 mg, 0.19 mmol) in 1,4-dioxane (4 mL). The reaction solution was stirred under microwave conditions at 120 °C for 40 min. Ethyl acetate (30 mL) was added to the reaction solution. The organic phase was washed successively with saturated sodium chloride solution (20 mL) and saturated brine (20 mL), dried over anhydrous sodium sulfate, filtered, and concentrated under reduced pressure to obtain the crude product. The crude product was purified by flash column chromatography to give the title product **13** (30 mg, yield: 32.6%).

¹H NMR (400 MHz, DMSO-*d₆*): δ 7.96 (dd, *J* = 8.0, 2.0 Hz, 2 H), 7.56 (s, 1 H), 7.36 (t, *J* = 8.0 Hz, 2 H), 5.07 (br. s, 2 H), 4.89 (t, *J* = 4.0 Hz, 1 H), 4.14 (br. s, 2 H), 3.79 (br. *d, J* = 8.0 Hz, 2 H), 3.42-3.52 (m, 1 H), 3.20-3.29 (m, 1 H), 2.86-3.04 (m, 4 H), 2.31-2.43 (m, 2 H), 2.21-2.29 (m, 2 H), 1.76-1.89 (m, 2 H)
MS m/z (ESI): 486 [M+1]

### Example 14

### (R)-4-((1-(hydroxymethyl)cyclobutyl)amino)-2-(2-(pyridin-2-yl)-6,7-dihydrothiazolo[5,4-c]pyridin-5(4H)-yl)-6,7-dihydrothieno[3,2-d]pyrimidine 5-oxide 14

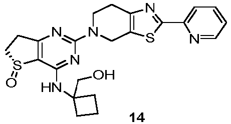

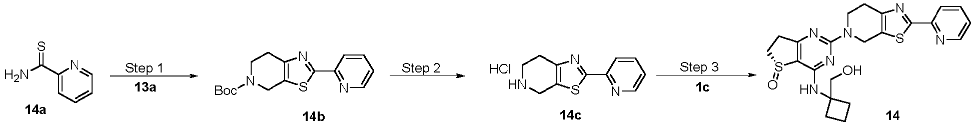

### Step 1

### tert-butyl 2-(pyridin-2-yl)-6,7-dihydrothiazolo[5,4-c]pyridine-5(4H)-carboxylate 14b

At room temperature, tert-butyl 3- bromo -4-oxopiperidine-1-carboxylate 13a (1.0 g, 3.6 mmol) and pyridine-2-thioamide **14a** (0.5 g, 3.6 mmol) were mixed in isopropanol solution (20 mL). The mixture was stirred at 60 °C for 5 hours, then filtered. The filter cake was washed with isopropanol and dried to obtain the title product **14b** (0.9 g).

### Step 2

### 2-(pyridin-2-yl)-4,5,6,7 - tetrahydrothiazolo[5,4-c]pyridine hydrochloride 14c

At room temperature, compound 14b (0.9 g, 2.8 mmol) was stirred in a solution (8 mL) of hydrochloric acid in 1,4-dioxanefor 1 hour. The solution was concentrated until the solvent was completely evaporated to obtain the title product **14c** (0.8 g).

### Step 3

### (R)-4-((1-(hydroxymethyl)cyclobutyl)amino)-2-(2-(pyridin-2-yl)-6,7-dihydrothiazolo[5,4-c]pyridin-5(4H)-yl)-6,7-dihydrothieno[3,2-d]pyrimidine 5-oxide 14

Diisopropylethylamine (108 mg, 0.84 mmol) was added to a solution of compound **14c** (53 mg, 0.21 mmol) and compound **1c** (55 mg, 0.19 mmol) in 1,4-dioxane solution (4 mL). The reaction solution was stirred at 120 °C for 40 min under microwave conditions. Ethyl acetate (30 mL) was added to the reaction solution. The organic phase was washed successively with saturated sodium chloride solution (20 mL) and saturated brine (20 mL), dried over anhydrous sodium sulfate, filtered, and concentrated under reduced pressure to obtain the crude product. The crude product was purified by flash column chromatography to give the title product **14** (35 mg, yield: 39.3%).

¹H NMR (400 MHz, DMSO-*d₆*): δ 8.63 (d, *J* = 8.0 Hz, 1 H), 8.07 (d, *J* = 8.0 Hz, 1 H), 7.96 (t, *J* = 8.0 Hz, 1 H), 7.54 (s, 1 H), 7.50 (t, *J* = 8.0 Hz, 1 H), 5.09 (br. s, 2 H), 4.89 (t, *J* = 8.0 Hz, 1 H), 4.15 (br. s, 2 H), 3.77 (d, *J* = 4.0 Hz, 2 H), 3.41-3.51 (m, 1 H), 3.20-3.30 (m, 1 H), 2.87-3.04 (m, 4 H), 2.31-2.44 (m, 2 H), 2.20-2.29 (m, 2 H), 1.76-1.89 (m, 2 H)
MS m/z (ESI): 469 [M+1]

### Example 15

### (R)-4-((1-(hydroxymethyl)cyclobutyl)amino)-2-(2-phenyl-7,8-dihydropyrimido[4,3-d]pyrimidin-6(5H)-yl)-6,7-dihydrothieno[3,2-d]pyrimidine 5-oxide 15

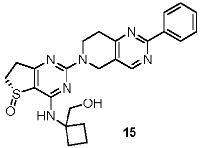

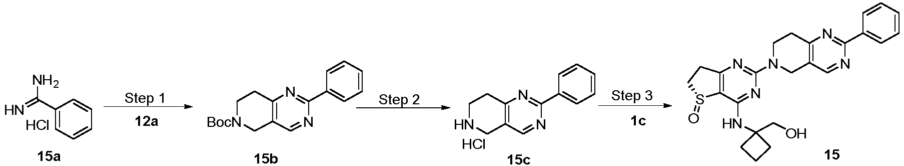

### Step 1

### tert-butyl 2-phenyl-7,8-dihydropyrimido[4,3-d]pyrimidine-6(5H)-carboxylate 15b

Under nitrogen protection, compound **12a** (1 g, 3.9 mmol), benzamidine hydrochloride **15a** (0.67 g, 4.3 mmol), and triethylamine (0.98 g, 9.7 mmol) were dissolved in ethanol (15 mL). The reaction solution was stirred at reflux temperature for 3 h. The reaction solution was concentrated, diluted with water (30 mL), and extracted with ethyl acetate (40 mL×2). The combined organic phases were concentrated under reduced pressure to obtain the crude product. The crude product was purified by flash column chromatography to give the title product 15b (700 mg).

### Step 2

### 2-phenyl-5,6,7,8-tetrahydropyrimido[4,3-d]pyrimidine hydrochloride 15c

A solution of hydrogen chloride in 1,4-dioxane (4 M, 2 mL) was added to a dichloromethane solution of compound **15b** (300 mg, 0.96 mmol). The reaction solution was stirred at room temperature for 2 h and then concentrated to dryness to obtain the title product **15c** (350 mg).

### Step 3

### (R)-4-((1-(hydroxymethyl)cyclobutyl)amino)-2-(2-phenyl-7,8-dihydropyrimido[4,3-d]pyrimidin-6(5H)-yl)-6,7-dihydrothieno[3,2-d]pyrimidine 5-oxide 15

The title product **15** (20 mg, yield: 24.1%) was prepared from compound **15c** (50 mg, 0.20 mmol) and compound **1c** (51 mg, 0.18 mmol) according to the method described in step 8 of Example 1.

¹H NMR (400 MHz, DMSO-*d₆*): δ 8.82 (s, 1 H), 8.38-8.41 (m, 2 H), 7.51-7.54 (m, 4 H), 5.01 (br. s, 2 H), 4.89 (t, *J* = 5.6 Hz, 1 H), 4.16 (t, *J* = 5.6 Hz, 2 H), 4.06 (br. s, 2 H), 3.79 (d, *J* = 5.6 Hz, 2 H), 3.44-3.49 (m, 1 H), 3.22-3.27 (m, 1 H), 2.91-3.05 (m, 4 H), 2.37-2.40 (m, 2 H), 2.28-2.34 (m, 2 H), 1.79-1.85 (m, 2 H)
MS m/z (ESI): 463 [M+1]

### Example 16

### (R)-3-(((2-(2-(4-fluorophenyl)-6,7-dihydrooxazolo[4,5-c]pyridin-5(4H)-yl)-5-oxido-6,7-dihydrothieno[3,2-d]pyrimidin-4-yl)amino)methyl)-4,6-dimethylpyridin-2(1H)-one 16

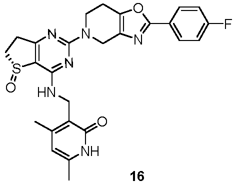

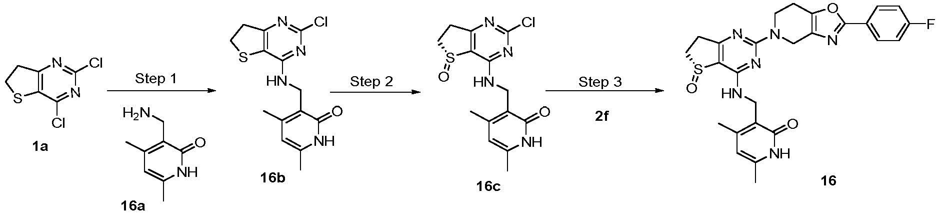

### Step 1

### 3-(((2-chloro-6,7-dihydrothieno[3,2-d]pyrimidin-4-yl)amino)methyl)-4,6-dimethylpyridin-2(1H)-one 16b

The title product **16b** (1.1 g) was prepared from compound 1a (1.0 g, 5.0 mmol) and 3-(aminomethyl)-4,6-dimethylpyridin-2(1H)-one 16a (0.75 g, 1.0 mmol) according to the method described in step 1 of Example 1.

### Step 2

### (R)-3-(((2-chloro-5-oxido-6,7-dihydrothieno[3,2-d]pyrimidin-4-yl)amino)methyl)-4,6-dimethylpyridin-2(1H)-one 16c

Under nitrogen protection, the title product 16c (0.8 g) was prepared from compound 16b (1.10 g, 3.41 mmol) according to the method described in step 2 of Example 1.

### Step 3

### (R)-3-(((2-(2-(4-fluorophenyl)-6,7-dihydrooxazolo[4,5-c]pyridin-5(4H)-yl)-5-oxido-6,7-dihydrothieno[3,2-d]pyrimidin-4-yl)amino)methyl)-4,6-dimethylpyridin-2(1H)-one 16

The title product 16 (3 mg, yield: 6%) was prepared from compound 2f (20 mg, 0.1 mmol) and compound 16c (30 mg, 0.1 mmol) according to the method described in step 8 of Example 1.

¹H NMR (400 MHz, DMSO-d6) δ ppm 11.55 (br s, 1 H), 8.03 (dd, *J* = 8.74, 5.45 Hz, 2 H), 7.61 (br s, 1 H), 7.39 (t, *J=* 8.88 Hz, 2 H), 5.88 (s, 1 H), 4.86 (br s, 2 H), 4.46-4.59 (m, 2 H), 4.26 (br s, 2 H), 3.37-3.53 (m, 1 H), 3.25-3.32 (m, 1 H), 2.94-3.06 (m, 1 H), 2.84-2.93 (m, 3 H), 2.19 (s, 3 H), 2.14 (s, 3 H)
MS m/z (ESI): 521 [M+1]

### Example 17

### (R)-3-(((2-(2-(4-fluorophenyl)-6,7-dihydrooxazolo[4,5-c]pyridin-5(4H)-yl)-5-oxido-6,7-dihydrothieno[3,2-d]pyrimidin-4-yl)amino)methyl)-6-methyl-4-(trifluoromethyl)pyridin-2(14)-one 17

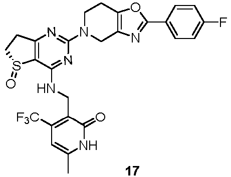

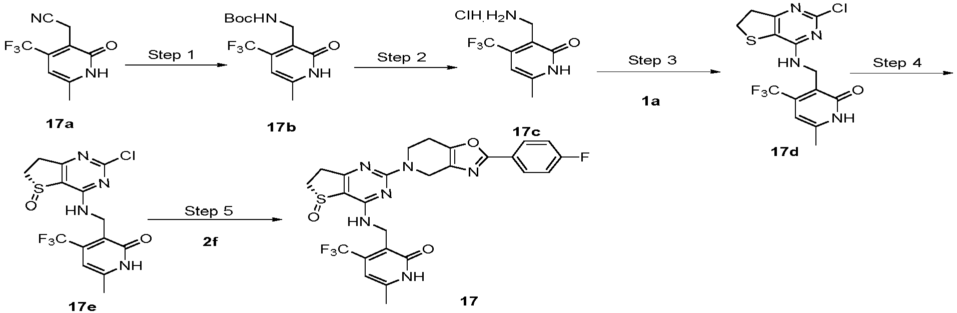

### Step 1

### tert-butyl (6-methyl-2-oxo-4-(trifluoromethyl)-1,2-dihydropyridin-3-yl)methylcarbamate 17b

Under an ice bath, 2-(6-methyl-2-oxo-4-(trifluoromethyl)-1,2-dihydropyridin-3-yl)acetonitrile **17a** (0.5 g, 2.5 mmol), nickel chloride (40 mg, 0.25 mmol) and di-tert-butyl dicarbonate (1.1 g, 5.0 mmol) were mixed in methanol (20 mL). Subsequently, sodium borohydride (0.19 g, 5.0 mmol) was added and the mixture was stirred for 2 hours. Then, sodium borohydride (0.19 g, 5.0 mmol) was added again, and the temperature was raised to 25 °C and the reaction solution was stirred for 14 hours. After the reaction was completed, water (30 mL) was added, and the mixture was extracted with ethyl acetate (40 mL×2). The combined organic phases were concentrated under reduced pressure to obtain the crude product. The crude product was purified by flash column chromatography to give the title product **17b** (0.1 g).

### Step 2

### 3-(aminomethyl)-6-methyl-4-(trifluoromethyl)pyridin-2(1H)-one hydrochloride 17c

At room temperature, compound 17b (0.1 g, 0.3 mmol) was stirred in a 1,4-dioxane solution of hydrochloric acid (4 M, 3 mL) for 1 hour. After the reaction was completed, it was concentrated under reduced pressure to obtain the title product **17c** (80 mg).

### Step 3

### 3-(((2-chloro-6,7-dihydrothieno[3,2-d]pyrimidin-4-yl)amino)methyl)-6-methyl-4-(trifluoromethyl)pyridin-2(1H)-one 17d

The title product **17d** (20 mg) was prepared from compound **1a** (70 mg, 0.3 mmol) and 3-(aminomethyl)-6-methyl-4-(trifluoromethyl)pyridin-2(1H)-one hydrochloride **17c** (80 mg, 0.3 mmol) according to the method described in step 1 of Example 1.

### Step 4

### (R)-3-(((2-chloro-5-oxido-6,7-dihydrothieno[3,2-d]pyrimidin-4-yl)amino)methyl)-6-methyl-4-(trifluoromethyl)pyridin-2(1H)-one 17e

Under nitrogen protection, the title product **17e** (15 mg) was prepared from compound **17d** (20 mg, 0.05 mmol) according to the method described in step 2 of Example 1.

### Step 5

### (R)-3-(((2-(2-(4-fluorophenyl)-6,7-dihydrooxazolo[4,5-c]pyridin-5(4H)-yl)-5-oxido-6,7-dihydrothieno[3,2 - d]pyrimidin-4-yl)amino)methyl)-6-methyl-4-(trifluoromethyl)pyridin-2(1H)-one 17

The title product 17 (2 mg, yield: 9%) was prepared from compound **2f** (9 mg, 0.04 mmol) and compound 17e (15 mg, 0.04 mmol) according to the method described in step 8 of Example 1.

¹H NMR (400 MHz, DMSO-d6) δ ppm 8.03 (dd, *J* = 8.79, 5.40 Hz, 2 H), 7.68 (br s, 1 H), 7.39 (t, *J* = 8.88 Hz, 2 H), 6.32 (s, 1 H), 4.79-4.89 (m, 2 H), 4.55-4.66 (m, 2 H), 4.20-4.29 (m, 2 H), 3.43-3.52 (m, 2 H), 3.01 (br dd, *J=* 17.03, 7.87 Hz, 1 H), 2.87-2.93 (m, 3 H), 2.29 (s, 3 H)_{∘}

MS m/z (ESI): 575 [M+1]

### Example 18

### (R)-2-(2-(4-fluorophenyl)-6,7-dihydrooxazolo[4,5-c]pyridin-5(4H)-yl)-4-(((2-methoxy-4,6-dimethylpyridin-3-yl)methyl)amino)-6,7-dihydrothieno[3,2-d]pyrimidine 5-oxide 18

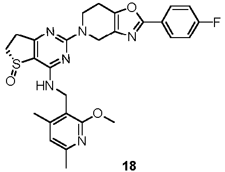

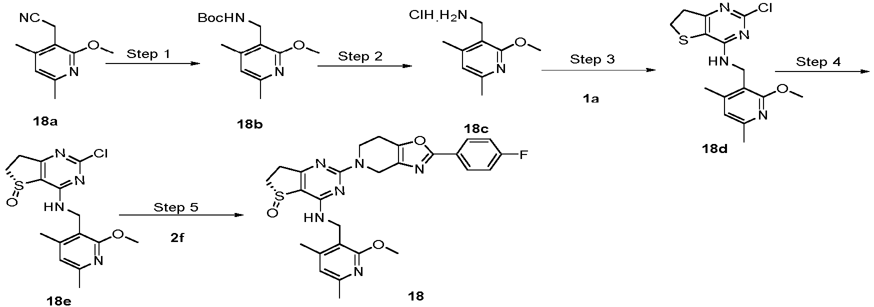

### Step 1

### tert-butyl (2-methoxy-4,6-dimethylpyridin-3-yl)methylcarbamate 18b

Under an ice bath, the title product **18b** (0.2 g) was prepared from 2-(2-methoxy-4,6-dimethylpyridin-3-yl)acetonitrile **18a** (0.5 g, 3.08 mmol) according to the method described in step 1 of Example 17.

### Step 2

### (2-methoxy-4,6-dimethylpyridin-3-yl)methanamine hydrochloride 18c

At room temperature, the title product **18c** (15 mg) was prepared from compound **18b** (0.2 g, 0.75 mmol) according to the method described in step 2 of Example 17.

### Step 3

### 2-chloro-N-((2-methoxy-4,6-dimethyl-1,2-dihydropyridin-3-yl)methyl)-6,7-dihydrothieno[3,2-d]pyrimidin-4-amine 18d

The title product **18d** (0.23 g) was prepared from compound **1a** (0.15 g, 0.75 mmol) and (2-methoxy-4,6-dimethylpyridin-3-yl)methanamine hydrochloride **18c** (0.15 g, 0.75 mmol) according to the method described in step 1 of Example 1.

### Step 4

### (R)-2-Chloro-4-(((2-methoxy-4,6-dimethylpyridin-3-yl)methyl)amino)-6,7-dihydrothieno[3,2-d]pyrimidine 5-oxide 18e

Under nitrogen protection, the title product **18e** (0.23 g) was prepared from compound **18d** (0.23 g, 0.68 mmol) according to the method described in step 2 of Example 1.

### Step 5

### (R)-2-(2-(4-fluorophenyl)-6,7-dihydrooxazolo[4,5-c]pyridin-5(4H)-yl)-4-(((2-methoxy-4,6-dimethylpyridin-3-yl)methyl)amino)-6,7-dihydrothieno[3,2-d]pyrimidine 5-oxide 18

The title product **18** (5 mg, yield: 9%) was prepared from compound **2f** (22 mg, 0.1 mmol) and compound **18e** (35 mg, 0.1 mmol) according to the method described in step 8 of Example 1.

¹H NMR (400 MHz, DMSO-d6) δ ppm 8.03 (dd, *J =* 8.79, 5.40 Hz, 2 H), 7.85 (br s, 1 H), 7.40 (t, *J* = 8.83 Hz, 2 H), 6.69 (s, 1 H), 4.83 (s, 2 H), 4.70 (dd, *J=* 14.14, 5.08 Hz, 1 H), 4.56 (dd, *J=* 14.14, 4.53 Hz, 1 H), 4.23 (br s, 2 H), 3.85-3.93 (m, 3 H), 3.41 -3.51 (m, 1 H), 3.27 (dt, *J* = 13.73, 8.51 Hz, 1 H), 3.01 (br dd, *J=* 17.58, 7.51 Hz, 1 H), 2.84-2.93 (m, 3 H), 2.35 (s, 3 H), 2.28 (s, 3 H)_{∘}

MS m/z (ESI): 535 [M+1]

### Example 19

### (R)-4-(benz[d]oxazol-6-ylamino)-2-(2-(pyridin-2-yl)-6,7-dihydrothiazolo[5,4-c]pyridin-5(4H)-yl)-6,7-dihydrothieno[3,2-d]pyrimidine 5-oxide 19

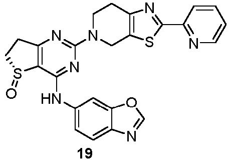

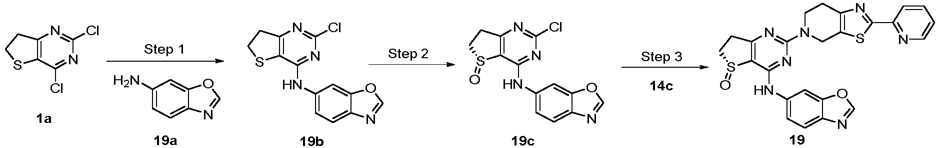

### Step 1

### N-(2-chloro-6,7-dihydrothieno[3,2-d]pyrimidin-4-yl)benzo[d]oxazol-6-amine 19b

The title product **19b** (0.72 g) was prepared from compound **1a** (1.0 g, 4.83 mmol) and benzo[d]oxazol-6-amine **19a** (0.65 g, 4.83 mmol) according to the method described in step 1 of Example 1.

### Step 2

### (R)-4-(benzo[d]oxazol-6-amino)-2-chloro-6,7-dihydrothieno[3,2-d]pyrimidine 5-oxide 19c

Under nitrogen protection, the title product **19c** (0.58 g) was prepared from compound **19b** (0.72 g, 2.36 mmol) according to the method described in step 2 of Example 1.

### Step 3

### (R)-4-(benzo[d]oxazol-6-amino)-2-(2-(pyridin-2-yl)-6,7-dihydrothiazolo[5,4-c]pyridin-5(4H)-yl)-6,7-dihydrothieno[3,2-d]pyrimidine 5-oxide 19

The title product **19** (12 mg, yield: 12%) was prepared from compound **14c** (50 mg, 0.2 mmol) and compound **19c** (63 mg, 0.2 mmol) according to the method described in step 8 of Example 1.

¹H NMR (400 MHz, DMSO-d6) δ ppm 9.93 (s, 1 H), 8.75 (s, 1 H), 8.65 (d, *J=* 4.30 Hz, 1 H), 8.18 (br s, 1 H), 8.10 (d, *J=* 7.87 Hz, 1 H), 7.96 (td, *J=* 7.71, 1.60 Hz, 1 H), 7.81 (d, *J=* 8.61 Hz, 1 H), 7.69 (br d, *J* = 8.15 Hz, 1 H), 7.50 (dd, *J=* 6.91, 5.36 Hz, 1 H), 5.13 (br s, 2 H), 4.18 (br s, 2 H), 3.54-3.65 (m, 1 H), 3.26-3.33 (m, 1 H), 3.15 (br dd, *J=* 16.80, 8.01 Hz, 1 H), 2.95-3.07 (m, 3 H)
MS m/z (ESI): 502 [M+1]

### Example 20

### (R)-2-(2-(4-fluorophenyl)-6,7-dihydrooxazolo[4,5-c]pyridin-5(4H)-yl)-4-(((1S,4S)-4-hydroxycyclohexyl)amino)-6,7-dihydrothieno[3,2 - d]pyrimidine 5-oxide 20

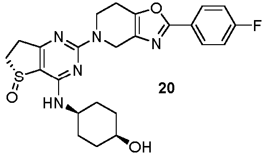

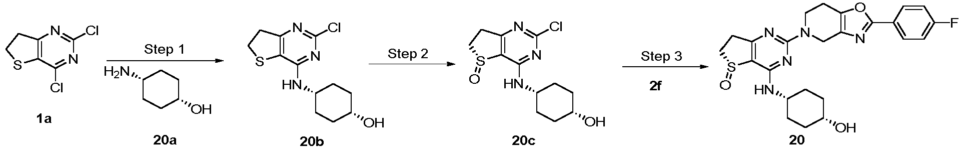

### Step 1

### (1S,4S)-4-((2-Chloro-6,7-dihydrothieno[3,2-d]pyrimidin-4-yl)amino)cyclohexan-1-ol 20b

The title product **20b** (300 mg) was prepared from compound **1a** (300 mg, 1.45 mmol) and compound **20a** (167 mg, 1.45 mmol) according to the method described in step 1 of Example 1.

### Step 2

### (R)-2-chloro-4-(((1S,4S)-4-hydroxycyclohexyl)amino)-6,7-dihydrothieno[3,2-d]pyrimidine 5-oxide 20c

Under nitrogen protection, the title product **20c** (150 mg) was prepared from compound **20b** (200 mg, 0.70 mmol) according to the method described in step 2 of Example 1.

### Step 3

### (R)-2-(2-(4-fluorophenyl)-6,7-dihydrooxazolo[4,5-c]pyridin-5(4H)-yl)-4-(((1S,4S)-4-hydroxycyclohexyl)amino)-6,7-dihydrothieno[3,2 - d]pyrimidine 5-oxide 20

The title product **20** (12 mg, yield: 12%) was prepared from compound **20c** (30 mg, 0.1 mmol) and compound **2f** (21 mg, 0.1 mmol) according to the method described in step 8 of Example 1.

¹H NMR (400 MHz, DMSO-*d*₆) δ ppm 8.02 (m, 2 H), 7.59 (d, *J*=7.23 Hz, 1 H), 7.39 (t, *J*=8.83 Hz, 2 H), 4.81 (br s, 2 H), 4.38 (d, *J*=2.75 Hz, 1 H), 4.21 (br s, 2 H), 4.01 (br s, 1 H), 3.83 (br s, 1 H), 3.41 - 3.54 (m, 1 H), 3.24 (m, 1 H), 2.86- 3.05 (m, 4 H), 1.77 - 1.90 (m, 2 H), 1.73 (m, 2 H), 1.51 - 1.68 (m, 4 H)
MS m/z (ESI): 484 [M+1]

### Example 21

### (R)-2-(2-(4-chlorophenyl)-6,7-dihydrooxazolo[5,4-c]pyridin-5(4H)-yl)-4-((1-(hydroxymethyl)cyclobutyl)amino)-6,7-dihydrothieno[3,2-d]pyrimidine 5-oxide 21

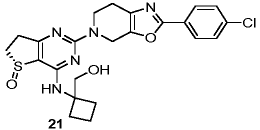

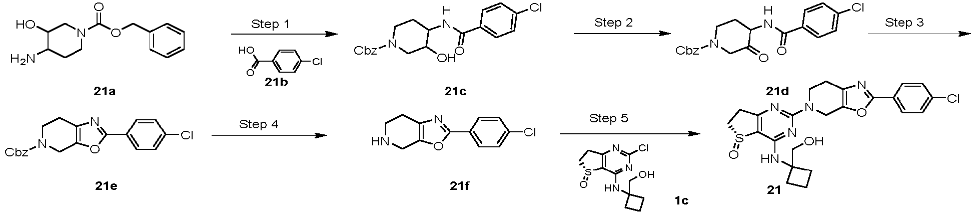

### Step 1

### benzyl 4-((4-chlorobenzoyl)amino)-3-hydroxypiperidine-1-carboxylate 21c

Compound **21b** (312 mg, 2.00 mmol), EDCI (766 mg, 4.00 mmol), and HOBT (540 mg, 4.00 mmol) were dissolved in dichloromethane (10 mL). After stirring at 25 °C for 1 hour, compound 21a (500 mg, 2.00 mmol) and TEA (404 mg, 4.00 mmol) were added, and the mixture was stirred at room temperature for 18 hours. After the reaction was completed , the reaction was quenched with water, extracted with DCM (20 mL×3), washed with saturated NaHCO₃ solution, dried over sodium sulfate, and concentrated to obtain the crude product. The crude product was purified by flash column chromatography to give the title product 21c (700 mg).

### Step 2

### benzyl 4-((4-chlorobenzoyl)amino)-3-oxopiperidine-1-carboxylate 21d

Compound 21c (400 mg, 1.03 mmol) was dissolved in dichloromethane. Under nitrogen protection, Dess-Martin (1309 mg, 3.09 mmol) was added at 0 °C, and the mixture was stirred at room temperature for 18 h. After the reaction was completed, water (10 mL) was added to the mixture to quench the reaction. The mixture was extracted with dichloromethane (20 mL)for three times. The organic phase was washed with saturated brine, dried over anhydrous sodium sulfate, and concentrated to obtain the crude product. The crude product was purified by flash chromatography to give the title product **21d** (350 mg).

### Step 3

### benzyl 2-(4-chlorophenyl)-6,7-dihydrooxazolo[5,4]pyridine-5(4H)-carboxylate 21e

Compound **21d** (300 mg, 0.77 mmol) was dissolved in phosphorus oxychloride (10 mL), and the mixture was stirred at 100°C for 3 hours. After the reaction was completed, most of the solvent was removed by evaporation. Ice water was added to quench the reaction. The mixture was extracted with DCM (20 mL×3), washed with saturated NaHCO₃ solution, dried over sodium sulfate, and rotary-evaporated to dryness to obtain the crude product. The crude product was purified by flash chromatography to give the title product **21e** (150 mg).

### Step 4

### 2-(4-chlorophenyl)-4,5,6,7-tetrahydrooxazolo[5,4-c]pyridine 21f

Compound **21e** (100 mg, 0.27 mmol) was dissolved in acetonitrile. Under nitrogen protection, TMSI (163 mg, 0.81 mmol) was added at 0 °C, and the mixture was stirred at 25 °C for 18 h. After the reaction was completed, the mixture was added with water (5 mL) to quench the reaction, extracted with dichloromethane (10 mL) three times. The organic phase was washed with saturated brine, dried over anhydrous sodium sulfate, and rotary-evaporated to dryness to obtain the crude product. The crude product was purified by flash chromatography to give the title product **21f** (50 mg).

### Step 5

### (R)-2-(2-(4-chlorophenyl)-6,7-dihydrooxazolo[5,4-c]pyridin-5(4H)-yl)-4-((1-(hydroxymethyl)cyclobutyl)amino)-6,7-dihydrothieno[3,2-d]pyrimidine 5-oxide 21

The title product **21** (10 mg, yield: 11%) was prepared from compound **1c** (50 mg, 0.17 mmol) and compound **21f** (49 mg, 0.21 mmol) according to the method described in step 8 of Example 1.

¹H NMR (400 MHz, DMSO) δ 7.97-7.95 (d, *J* = 8.4 Hz, 2H), 7.60-7.57 (m, 3H), 4.94(s, 2H), 4.88-4.86 (m, 1H), 4.09(s, 2H), 3.75-3.74 (d, *J=* 5.2 Hz, 2H), 3.41-3.39 (m, 1H), 3.19-3.17 (m, 1H), 2.85-2.84 (m, 2H), 2.67(s, 2H), 2.22-2.21 (m, 4H), 1.81-1.77 (m, 2H)
MS m/z (ESI): 486 [M+1]

### Example 22

### (R)-2-(6-(pyrimidin-2-yl)-3,4-dihydroisoquinolin-2(1H)-yl)-4-((tetrahydro-2H -pyran-4-yl)amino)-6,7-dihydrothieno[3,2-d]pyrimidine 5-oxide 22

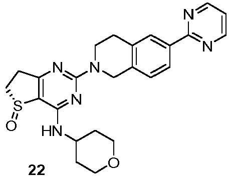

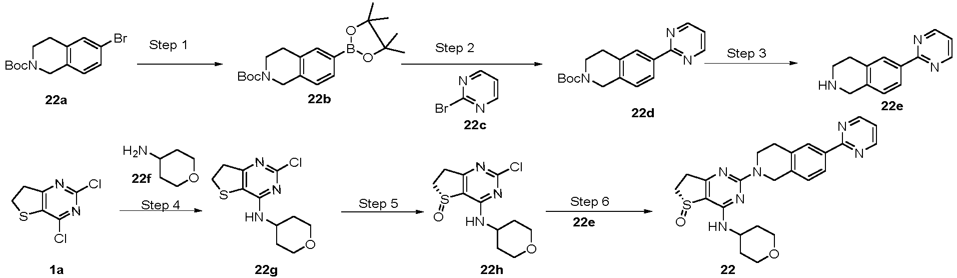

### Step 1

### tert-butyl 6-(4,4,5,5- tetramethyl-1,3,2- dioxaborolan-2-yl)-3,4-dihydroisoquinoline-2(1H)-carboxylate 22b

Compound **22a** (1 g, 3.2 mmol), bis(pinacolato)diboron (1.1 g, 4.2 mmol), potassium acetate (0.41 g, 4.2 mmol) and [1,1'-bis(diphenylphosphino)ferrocene]dichloropalladium(II) dichloromethane complex (150 mg, 0.2 mmol) were mixed in 1,4-dioxane solution (15 mL) under nitrogen protection. The reaction mixture was stirred at 90 °C for 3 hours. After the reaction was completed, the mixture was concentrated under reduced pressure to remove solvent, added with water (30 mL) and extracted with ethyl acetate (30 mL×2). The organic phases were combined and concentrated under reduced pressure to obtain the crude title product **22b** (1.2 g).

### Step 2

### tert-butyl 6-(pyrimidin-2-yl)-3,4-dihydroisoquinoline-2(1H)- carboxylate 22d

Compound **22b** (1.2 g, 3.3 mmol), compound **22c** (349 mg, 2.2 mmol), potassium carbonate (455 mg, 3.3 mmol) and [1,1'-bis(diphenylphosphino)ferrocene]dichloropalladium(II) dichloromethane complex (146 mg, 0.2 mmol) were mixed in a solution of 1,4-dioxane (10 mL)/water (2 mL) under nitrogen protection. The reaction mixture was stirred at 80 °C for 3 hours. Ethyl acetate (30 mL) was added to the reaction mixture. The organic phase was washed successively with saturated ammonium chloride solution (20 mL) and saturated brine (20 mL), dried over anhydrous sodium sulfate, filtered and concentrated under reduced pressure to obtain the crude product. The crude product was purified by flash column chromatography to obtain the title product **22d** (0.97 g).

### Step 3

### 6- (pyrimidin-2-yl)-1,2,3,4-tetrahydroisoquinoline 22e

Trifluoroacetic acid (4 mL) was added to a solution of compound **22d** (0.97 g, 3.1 mmol) in dichloromethane (8 mL). The reaction mixture was stirred at room temperature for 1 hour and then concentrated to dryness to obtain the title product **22e** (654 mg).

### Step 4

### 2-chloro-N-(tetrahydro-2H-pyran-4-yl)-6,7-dihydrothieno[3,2-d]pyrimidin-4-amine 22g

Under nitrogen protection, the title product **22g** (180 mg) was obtained from compound **1a** (200 mg, 0.97 mmol) and compound **22f** (101 mg, 0.97 mmol) according to the method in step 1 of Example 1.

### Step 5

### (R)-2-chloro-4-((tetrahydro-2H-pyran-4-yl)amino)-6,7- dihydrothieno[3,2-d]pyrimidine 5-oxide 22h

At room temperature, the title product 22h (150 mg) was parapared from compound **22g** (180 mg, 0.66 mmol) according to the method in the second step of Example 1.

### Step 6

### (R)-2-(6-(pyrimidin-2-yl)-3,4-dihydroisoquinolin-2(1H)-yl)-4-((tetrahydro-2H-pyran-4-yl)amino)-6,7-dihydrothieno[3,2-d]pyrimidine 5-oxide 22

The title product 22 (27 mg, yield: 15%) was obtained from compound 22e (80 mg, 0.37 mmol) and compound 22h (106 mg, 0.37 mmol) under microwave conditions and at 120 °C according to the method in step 8 of Example 1.

¹H NMR (400 MHz, DMSO-*d*₆) δ ppm 8.89 (d, *J=*4.85 Hz, 2 H), 8.21 - 8.24 (m, 2 H), 7.61 (d, *J*=7.42 Hz, 1 H), 7.37 - 7.45 (m, 2 H), 4.97 (s, 2 H), 4.24-4.28 (m, 1 H), 4.05 (t, *J=*5.86 Hz, 2 H), 3.87 - 3.94 (m, 2 H), 3.40 - 3.50 (m, 3 H), 3.18-3.27 (m, 1 H), 2.97 - 3.01 (m, 4 H), 1.78-1.84 (m, 2 H), 1.58-1.71 (m, 2 H)
MS m/z (ESI): 463.55 [M+1]

### Example 23

### (R)-2-(2-(4-fluorophenyl)-6,7-dihydrooxazolo[4,5-c]pyridin-5(4H)-yl)-4-((((2-methoxy-4,6-dimethylpyridin-3-yl)methyl)amino)-6,7-dihydrothieno[3,2-d]pyrimidine 5-oxide 23

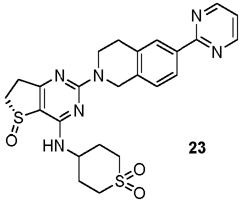

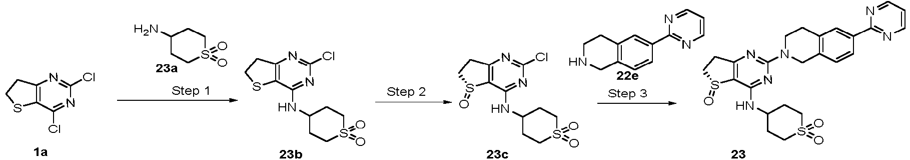

### Step 1

### 4-((2-chloro-6,7-dihydrothieno[3,2-d]pyrimidin-4-yl)amino)tetrahydro-2H-thiopyran 1,1-dioxide 23b

Under nitrogen protection, the title product 23b (180 mg) was obtained from compound **1a** (200 mg, 0.97 mmol) and compound **23a** (145 mg, 0.97 mmol) according to the method in step 1 of Example 1.

### Step 2

### (R)-4-((2-chloro-5-oxido-6,7-dihydrothieno[3,2-d]pyrimidin-4-yl)amino)tetrahydro-2H-thiopyran 1,1-dioxide 23c

At room temperature, the title product 23c (150 mg) was obtained from compound **23b** (180 mg, 0.56 mmol) according to the method in step 2 of Example 1.

### Step 3

### (R)-2-(2-(4-fluorophenyl)-6,7-dihydrooxazolo[4,5-c]pyridin-5(4H)-yl)-4-(((2-methoxy-4,6-dimethylpyridin-3-yl)methyl)amino)-6,7-dihydrothieno[3,2-d]pyrimidine 5-oxide 23

The title product 23 (15 mg, yield: 33%) was obtained from compound **23c** (30 mg, 0.09 mmol) and compound **22e** (19 mg, 0.09 mmol) according to the method in step 8 of Example 1.

¹H NMR (400 MHz, DMSO-*d*₆) δ ppm 8.92 (d, *J*=4.85 Hz, 2 H), 8.22 - 8.30 (m, 2 H), 7.86 (br d, J=7.42 Hz, 1 H), 7.46 (br t, *J*=4.81 Hz, 2 H), 5.02 (s, 2 H), 4.39 - 4.54 (m, 1 H), 4.11 (br t, *J*=5.40 Hz, 2 H), 3.42 - 3.54 (m, 3 H), 3.15- 3.28 (m, 3 H), 2.89 - 3.08 (m, 4 H), 2.20 (br s, 4 H)
MS m/z (ESI): 511 [M+1]

### Example 24

### (R)-4-((3-(hydroxymethyl)bicyclo[1.1.1]pentan-1-yl)amino)-2-(6-(pyrimidin-2-yl)-3,4-dihydroisoquinolin-2(1H)-yl)-6,7-dihydrothieno[3,2-d]pyrimidine 5-oxide 24

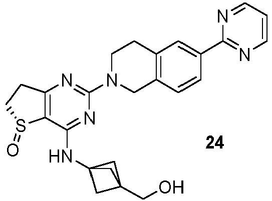

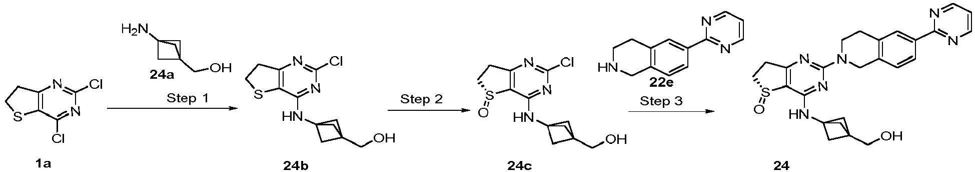

### Step 1

### (3-((2-chloro-6,7-dihydrothieno[3,2-d]pyrimidin-4-yl)amino)bicyclo[1.1.1]pentan-1-yl)methanol 24b

The title product 24b (180 mg) was obtained from compound **1a** (200 mg, 0.97 mmol) and compound **24a** (109 mg, 0.97 mmol) under nitrogen protection according to the method in step 1 of Example 1.

### Step 2

### (R)-2-chloro-4-((3-(hydroxymethyl)bicyclo[1.1.1]pentan-1-yl)atnino)-6,7-dihydrothieno[3,2-d]pyrimidine 5-oxide 24c

The title product 24c (140 mg) was obtained from compound **24b** (180 mg, 0.64 mmol) at room temperature according to the method in step 2 of Example 1.

### Step 3

### (R)-4-((3-(hydroxymethyl)bicyclo[1.1.1]pentan-1-yl)amino)-2-(6-(pyrimidin-2-yl)-3,4-dihydroisoquinolin-2(1H)-yl)-6,7-dihydrothieno[3,2-d]pyrimidine 5-oxide 24

The title product 24 (15 mg, yield: 32%) was obtained from compound **24c** (30 mg, 0.1 mmol) and compound **22e** (21 mg, 0.1 mmol) according to the method in step 8 of Example 1.

¹H NMR (400 MHz, DMSO-*d*₆) δ ppm 8.92 (d, *J*=4.85 Hz, 2 H), 8.35 (s, 1 H), 8.26 (s, 1 H), 8.25 (d, *J*=7.35 Hz, 1 H), 7.39 - 7.48 (m, 2 H), 5.02 (s, 2 H), 4.58 (t, *J*=5.63 Hz, 1 H), 4.09 (t, *J*=5.86 Hz, 2 H), 3.46- 3.59 (m, 3 H), 3.19 - 3.27 (m, 1 H), 2.98-3.06 (m, 3 H), 2.90 (m, 1 H), 2.06 (s, 6 H)

MS m/z (ESI): 475 [M+1]

### Example 25

### 2-(4-((2-(6,7-dihydrothieno[3,2-c]pyridin-5(4H)-yl)-5,5-dioxido-7,8-dihydro-6H-thiopyrano[3,2-d]pyrimidin-4-yl)amino)phenyl) acetic acid 25

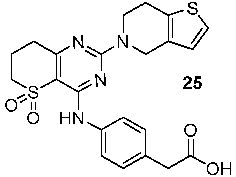

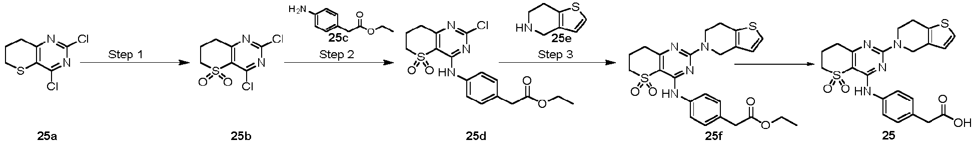

### Step 1

### 2,4-dichloro-7,8-dihydro-6H-thiopyrano[3,2-d]pyrimidine5,5 -dioxide 25b

At room temperature,2,4-dichloro-7,8-dihydro-6H-thiopyrano[3,2-d]pyrimidine25a (1.1 g, 4.98 mmol) was dissolved in dichloromethane (25 mL). Then m-chloroperoxybenzoic acid (2.57 g, 14.94 mmol) was added to the reaction solution. The reaction solution was stirred at 25 °C for 3 hours. After the reaction was completed, the solvent was removed by concentration under reduced pressure. The resulting crude product was purified by flash column chromatography to obtain the title product **25b** (0.85 g).

### Step 2

### ethyl 2-(4-((2-chloro-5,5-dioxido-7,8-dihydro-6H-thiopyrano[3,2-d]pyrimidin-4-yl)amino)phenyl)acetate 25d

At room temperature, 2,4-dichloro-7,8-dihydro-6H-thiopyrano[3,2-d]pyrimidine 5,5- dioxide **25b** (100 mg, 0.4 mmol), compound **25c** (88 mg, 0.54 mmol) and N,N - diisopropylethylamine (0.24 mL, 1.35 mmol) were mixed in ethanol (10 mL). The reaction solution was stirred at 60 °C for 12 hours. After the reaction was completed, the solvent was removed by concentration under reduced pressure. Then water (20 mL) was added, and the mixture was extracted with dichloromethane (30 mL×2). The organic phases were combined and concentrated under reduced pressure to obtain the crude product. The crude product was purified by flash column chromatography to obtain the title product 25d (100 mg).

### Step 3

### ethyl 2-(4-((2-(6,7-dihydrothieno[3,2-c]pyridin-5(4H)-yl)-5,5-dioxido-7,8-dihydro-6H-thiopyrano[3,2-d]pyrimidin-4-yl)amino)phenyl) acetate 25f

At room temperature, compound **25d** (100 mg, 0.25 mmol), compound **25e** (35 mg, 0.25 mmol), N,N-diisopropylethylamine (65 mg, 0.5 mmol) and dioxane (3 mL) were added to a microwave reaction tube. The microwave tube was placed in a microwave reactor and reacted at 120 °C for 1 hour. After the reaction was completed, the solvent was removed by concentration under reduced pressure. The resulting crude product was purified by flash column chromatography to obtain the title product **25f** (60 mg).

### Step 4

### 2-(4-((2-(6,7-dihydrothieno[3,2-c]pyridin-5(4H)-yl)-5,5-dioxido-7,8-dihydro-6H-thiopyrano[3,2-d]pyrimidin-4-yl)amino)phenyl) acetic acid 25

At room temperature, compound **25f** (60 mg, 0.12 mmol) and lithium hydroxide (15 mg, 0.36 mmol) were dissolved in a mixed solution of THF/MeOH/H₂O (3 mL/1 mL/1 mL). The reaction solution was reacted at room temperature for 1 hour. Then the pH was adjusted to 2-3 with dilute hydrochloric acid, and the mixture was extracted with dichloromethane (20 mL×2). The organic phases were combined and concentrated under reduced pressure to obtain the crude product. The crude product was purified by flash column chromatography to obtain the title product **25** (20 mg, yield 36%).

¹H NMR (400 MHz, DMSO-*d*₆) δ ppm 12.37 (br s, 1 H), 8.62 (s, 1 H), 7.55 (m, 2 H), 7.39 (d, *J*=5.13 Hz, 1 H), 7.32 (m, 2 H), 6.98 (br d, *J*=4.85 Hz, 1 H), 4.72 - 4.91 (m, 2 H), 4.05 (br s, 2 H), 3.55- 3.65 (m, 4 H), 2.83- 2.92 (m, 4 H), 2.28-2.36 (m, 2 H)
MS m/z (ESI): 471 [M+1]

### Example 26

### 2-(4-((2-(3,4-dihydroisoquinolin-2(1H)-yl)-5,5-dioxido-7,8-dihydro-6H-thiopyrano[3,2 - d]pyrimidin-4-yl)amino)-2-fluorophenyl)acetic acid 26

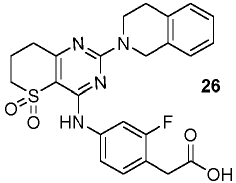

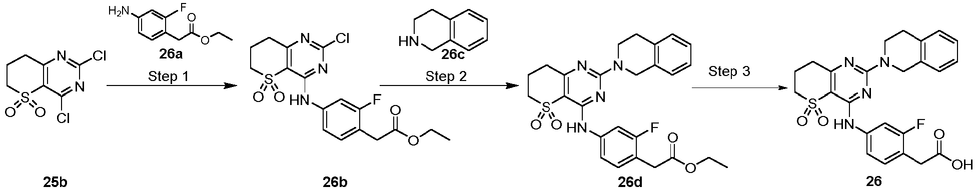

### Step 1

### ethyl 2-(4-((2-chloro-5,5-dioxido-7,8-dihydro-6H-thiopyrano[3,2-d]pyrimidin-4-yl)amino)-2-fluorophenyl) acetate 26b

Under nitrogen protection, the title product **26b** (180 mg) was obtained from compound **25b** (200 mg, 0.79 mmol) and compound **26a** (156 mg, 0.79 mmol) according to the method in step 2 of Example 25.

### Step 2

### ethyl 2-(4-((2-(3,4-dihydroisoquinolin-2(1H)-yl)-5,5-dioxido-7,8-dihydro-6H-thiopyrano[3,2-d]pyrimidin-4-yl)amino)-2-fluorophenyl)acetate 26d

The title product 26d (120 mg) was obtained from compound **26c** (58 mg, 0.43 mmol) and compound 26b (180 mg, 0.43 mmol) according to the method in the third step of Example 25.

### Step 3

### 2-(4-((2-(3,4-dihydroisoquinolin-2(1H)-yl)-5,5-dioxido-7,8-dihydro-6H-thiopyrano[3,2-d]pyrimidin-4-yl)amino)-2-fluorophenyl)acetic acid 26

The title product **26** (20 mg, yield: 36%) was obtained from compound **26d** (60 mg, 0.12 mmol) at room temperature according to the method in step 4 of Example 25.

¹H NMR (400 MHz, DMSO-*d*₆) δ ppm 8.74 (s, 1 H), 7.73 (br s, 1 H), 7.37 (t, *J*=8.05 Hz, 1 H), 7.20 - 7.32 (m, 5 H), 4.82 - 5.00 (m, 2 H), 3.93- 4.07 (m, 2 H), 3.57 - 3.68 (m, 4 H), 2.85- 2.95 (m, 4 H), 2.30-2.36 (m, 2 H)
MS m/z (ESI): 483 [M+1]

### Biological Evaluation

### Test Example 1: Inhibitory effect of Compounds in present disclosure on PDE4 Enzyme

PDE4 enzyme can hydrolyze cAMP to produce AMP. The synthetic PDE4 enzyme inhibitor act together with PDE4 enzyme and the substrate cAMP for a certain period of time, and then the AMP-Glo^{™} Assay kit was used for detection. The detection is divided into two steps: first, converting the AMP produced in the reaction into ADP, and second, converting ADP into ATP. ATP binds to the reagent and emits fluorescence. The fluorescence value is detected using an Echo 550. IC₅₀ value of the PDE4 enzyme inhibitor was calculated by plotting a graph with the fluorescence values. The specific details are as follows:

### 1.1 Reagents and Instruments

### 1.1.1 Reagents

| Material | Manufacturer | Item Number |
|---|---|---|
| PDE4B2 | BPS bioscience | 60042 |
| Cyclic-3',5'-AMP | Sigma | A6885 |
| AMP-Glo^{™} Assay kit | Promega | V5011 |
| Tris | amresco | T0497 |
| BSA | Perkin Elmer | CR84-100 |
| Magnesium chloride | Sigma | M1028 |
| Tween-20 | Solarbio | T8220 |
| DTT | Invitrogen | P2325 |
| DMSO | Sigma | D8418 |

### 1.1.2 Instruments

| Instrument | Manufacturer | Model |
|---|---|---|
| 96- well plate | Nunc | 249944 |
| 384 - well plate | Greiner | 784075 |
| Microplate shaker | QILINBEIER | QB-9002 |
| Centrifuge | Eppendorf | 5810R |
| Envision multimode plate reader | PerkinElmer | Envision 2105 |
| Vortex mixer | IKA | MS3 digital |
| Echo | Labcyte | 550 |

### 1.2 Experimental Procedures

### 1.2.1 Compound Preparation and Handling

a) Preparation of Compound DMSO Stock Solutions: All compounds were dissolved in DMSO to prepare stock solutions with a concentration of 10 mM.
b) Compound Storage: All compounds dissolved in DMSO were stored in a desiccator at room temperature for short-term storage.

### 1.2.2 Preparation of Working Stock Solutions

a) The positive control AKEX0834 (the compound of Example 2 in WO2013026797A1) solution was subjected to 3-fold gradient dilution with DMSO, serial dilutions from 100 µM for a total of 10 concentrations.
b) The test compounds were subjected to 3-fold gradient dilution with DMSO, serial dilutions from 100 µM for a total of 10 concentrations.
c) 200X positive control (1 mM AKEX0834) and 200X solvent control (100% DMSO) were prepared. The compound plate was centrifuged at 1000 rpm for 1 minute.

### 1.2.3 Compound Screening

a) 20 nL of the compound dilution was transferred to each well of the detection plate using an Echo 550.
b) The detection plate was sealed and the compound plate was centrifuged at 1000 rpm for 1 minute.
c) 2X PDE4B2 or PDE4D2 was prepared in frozen PDE assay buffer.
d) 2 µL of 2X PDE4B2 or PDE4D2 was added to a single well of the detection plate (prepared in step b).
e) The detection plate was sealed and equilibrated at room temperature for 10 minutes.
f) 2X Cyclic-3',5'-AMP was prepared in PDE detection buffer.
g) 2 µL of 2X Cyclic-3',5'-AMP (prepared in step f) was added to each well of the detection plate (prepared in step e) for starting action, which was incubated at room temperature for 60 minutes.
h) 4 µL of AMP-Glo Reagent I was added and an incubation was performed at room temperature for 60 minutes.
i) 8 µL of AMP detection solution was added and an incubation was performed at room temperature for 60 minutes.
j) The RLU signal on an Envision 2105 plate reader was read.

### 1.3 Data Analysis

The calculation formula is as follows: % Inhibition ={ 1-(Average RLU of the compound-Average RLU of the positive control of the whole plate) / (Average RLU of the negative control of the whole plate-Average RLU of the positive control of the whole plate)}

The IC50 is calculated by fitting the % inhibition values and the logarithm of the compound concentration to a non-linear regression (dose response-variable slope) using Graphpad 8.0.

**Table 1 Inhibitory effect of Compounds in present disclosure on PDE4 Enzyme**

| Example No. | IC₅₀(nM) | |
|---|---|---|
| | PDE4B2 | PDE4D2 |
| 1 | 0.21 | 0.67 |
| 2 | 0.19 | 0.38 |
| 3 | 0.23 | 0.42 |
| 4 | 0.08 | 0.21 |
| 5 | 0.93 | - |
| 6 | 1.20 | 2.1 |
| 7 | 2.58 | 4.64 |
| 8 | 0.07 | 0.09 |
| 9 | 0.24 | 0.74 |
| 10 | 0.17 | 0.23 |
| 11 | 0.46 | 1.90 |
| 12 | 0.63 | - |
| 13 | 0.28 | 0.31 |
| 14 | 0.16 | 0.31 |
| 15 | 0.60 | 0.86 |
| 16 | 0.2 | 3.4 |
| 17 | 8.9 | 26.3 |
| 18 | 0.5 | 0.8 |
| 19 | 0.023 | 0.066 |
| 20 | 0.4 | 0.4 |
| 21 | 0.42 | 1.29 |
| 22 | 0.071 | 0.09 |
| 23 | 0.063 | 0.106 |
| 24 | <0.1 | - |
| 25 | 2.5 | 19.7 |
| 26 | 0.45 | 27.4 |

Conclusion: The compounds disclosed in present disclosure exhibit significant inhibitory effects on PDE4 enzymes.

## Claims

1. A compound of formula I, or a pharmaceutically acceptable salt thereof: 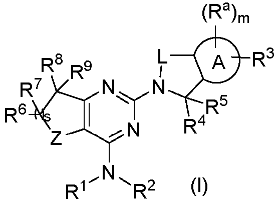 wherein:
ring A is selected from heteroaryl, aryl, heterocyclyl, and cycloalkyl;
L is -(CR^{4a} R^{4b} )ₚ(CR^{5a}R^{5b})_{q}-;
Z is selected from -S-, -S(O)-, -S(O)₂-, and -O-;
R¹ and R² are identical or different, and are each independently selected from a hydrogen atom, a deuterium atom, alkyl, alkenyl, alkynyl, cycloalkyl, heterocyclyl, aryl, and heteroaryl; wherein the alkyl, alkenyl, alkynyl, cycloalkyl, heterocyclyl, aryl, and heteroaryl are each independently optionally substituted by one or more substituents selected from a group consisting of a deuterium atom, alkyl, halogen, oxo, haloalkyl, -OR^{c1} , cyano, -(CH₂)ᵤ₁NR^{d1}R^{d2} , -C(O)NR^{d3}R^{d4} , -S(O)₂NR^{d3}R^{d4} , - (CH₂)ᵥ₁NR^{d5}C(O)R^{e1} , -(CH₂)ᵥ₁NR^{d5}S(O)₂R^{e1}, -C(O)R^{e2}, -OC(O)R^{e'3}, -S(O)ᵣR^{e4}, -(CH₂)_{w1}C(O)ORC², - (CH₂)ᵤR, hydroxyalkyl, cycloalkyl, heterocyclyl, aryl, and heteroaryl;
R³ is selected from a group consisting of a hydrogen atom, a deuterium atom, alkyl, alkenyl, alkynyl, halogen, cyano, alkylcyano, oxo, -OR^{c3}, -(CH₂)ᵤ₂NR^{d6}R^{d7}, -C(O)NR^{d8}R^{d9}, -(CH₂)ᵥ₂NR^{d10}C(O)R^{e5} - C(O)R^{e6}, -OC(O)R^{e7}, -S(O)ₜR^{e8}, -(CH₂)_{w2}C(O)OR^{c4} , and 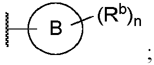 wherein the alkyl, alkenyl, and alkynyl are each independently optionally substituted by one or more substituents selected from a deuterium atom, halogen, haloalkyl, -OR^{c1},cyano,-(CH₂)ᵤ₁NR^{d1}R^{d2}, hydroxyalkyl, cycloalkyl, heterocyclyl, aryl, and heteroaryl;
ring B is selected from heteroaryl, aryl, heterocyclyl, and cycloalkyl;
each R^{b} is identical or different, and is each independently selected from a group consisting of a deuterium atom, halogen, cyano, alkylcyano, oxo, alkyl, haloalkyl, -OR^{c3}, -(CH₂)ᵤ₂NR^{d6}R^{d7}, - C(O)NR^{d8}R^{d9}, -(CH₂)ᵥ₂NR^{d10}C(O)R^{e5}, -C(O)R^{e6}, -OC(O)R^{e7}, -S(O)ᵣR^{e8}, -(CH₂)_{w2}C(O)OR^{c4} , hydroxyalkyl, cycloalkyl, heterocyclyl, aryl, and heteroaryl;
R⁴, R^{4a}, R^{4b}, R⁵, R^{5a} and R^{5b} are identical or different, and are each independently selected from a hydrogen atom, a deuterium atom, alkyl, halogen, alkenyl, alkynyl, cyano, alkylcyano, hydroxyalkyl, haloalkyl, -OR^{c3}, -(CH₂)ᵤ₂NR^{d6}R^{d7}, -C(O)NR^{d8}R^{d9}, -(CH₂)v₂NR^{d10}C(O)R^{e5}, -C(O)R^{e6}, -OC(O)R^{e7}, - S(O)ₜR^{e8}, -(CH₂)_{w2}C(O)OR^{c4} , cycloalkyl, heterocyclyl, aryl, and heteroaryl; or each of R⁴ and R⁵ , R^{4a} and R^{4b} , R^{5a} and R^{5b} together with the carbon atom to which they are attached form an oxo;
R⁶, R⁷, R⁸ and R⁹ are identical or different, and are each independently selected from a hydrogen atom, a deuterium atom, alkyl, alkenyl, alkynyl, halogen, cyano, alkylcyano, haloalkyl, hydroxyalkyl, - OR^{c3}, -(CH₂)ᵤ₂NR^{d6}R^{d7}, cycloalkyl, heterocyclyl, aryl, and heteroaryl; wherein the alkyl, alkenyl, alkynyl, cycloalkyl, heterocyclyl, aryl, and heteroaryl are each independently optionally substituted by one or more substituents selected from a group consisting of halogen, oxo, alkyl, haloalkyl, -OR^{c1}, cyano, -(CH₂)ᵤ₁NR^{d1}R^{d2}, hydroxyalkyl, cycloalkyl, heterocyclyl, aryl, and heteroaryl;
each R^{a} is identical or different, and is each independently selected from a deuterium atom, alkyl, halogen, oxo, alkenyl, alkynyl, cyano, alkylcyano, hydroxyalkyl, haloalkyl, -OR^{c3}, -(CH₂)ᵤ₂NR^{d6}R^{d7}, cycloalkyl, heterocyclyl, aryl, and heteroaryl;
R is selected from a group consisting of cycloalkyl, heterocyclyl, aryl, and heteroaryl; wherein the cycloalkyl, heterocyclyl, aryl, and heteroaryl are each independently optionally substituted by one or more substituents selected from a group consisting of a deuterium atom, halogen, oxo, alkyl, haloalkyl, -OR^{c1}, cyano, alkylcyano, -(CH₂)ᵤ₁NR^{d1}R^{d2}, hydroxyalkyl, cycloalkyl, heterocyclyl, aryl, and heteroaryl;
at each occurrence, R^{c1}, R^{c2}, R^{c3}, and R^{c4} are identical or different, and are each independently selected from a group consisting of a hydrogen atom, a deuterium atom, alkyl, hydroxyalkyl, cycloalkyl, heterocyclyl, aryl, and heteroaryl, wherein the alkyl, cycloalkyl, heterocyclyl, aryl, and heteroaryl are each independently optionally substituted by one or more substituents selected from a deuterium atom, halogen, oxo, alkyl, haloalkyl, alkoxy, haloalkoxy, cyano, alkylcyano, -(CH₂)ᵤ₁NR^{d1}R^{d2}, hydroxy, hydroxyalkyl, cycloalkyl, heterocyclyl, aryl, and heteroaryl;
at each occurrence, R^{d1}, R^{d2}, R^{d3}, R^{d4}, R^{d5}, R^{d6}, R^{d7}, R^{d8}, R^{d9} and R^{d10} are identical or different, and are each independently selected from a hydrogen atom, a deuterium atom, alkyl, haloalkyl, hydroxyalkyl, cycloalkyl, heterocyclyl, aryl, and heteroaryl;
or each of R^{d1} and R^{d2} , R^{d3} and R^{d4}, R^{d6} and R^{d7}, and R^{d8} and R^{d9} forms a heterocyclyl together with the nitrogen atom to which they are attached, wherein the heterocyclyl is each independently optionally substituted by one or more substituents selected from a deuterium atom, halogen, oxo, alkyl, alkoxy, haloalkyl, haloalkoxy, cyano, alkylcyano, amino, alkylamino, hydroxy, hydroxyalkyl, cycloalkyl, heterocyclyl, aryl, and heteroaryl;
at each occurrence, R^{e1}, R^{e2}, R^{e3}, R^{e4}, R^{e5}, R^{e6}, R^{e7}, and R^{e8} are identical or different, and are each independently selected from alkyl, hydroxyalkyl, cycloalkyl, heterocyclyl, aryl, and heteroaryl, wherein the alkyl, cycloalkyl, heterocyclyl, aryl, and heteroaryl are each independently optionally substituted by one or more substituents selected from a deuterium atom, halogen, oxo, alkyl, haloalkyl, alkoxy, haloalkoxy, cyano, alkylcyano, -(CH₂)ᵤ₁NR^{d1}R^{d2}, hydroxy, hydroxyalkyl, cycloalkyl, heterocyclyl, aryl, and heteroaryl;
m is 0, 1, 2, or 3;
n is 0, 1, 2, 3, 4, 5, or 6;
p is 0, 1, or 2;
q is 0, 1, or 2;
r is 0, 1, or 2;
s is 1 or 2;
t is 0, 1, or 2;
u is 0, 1, 2, or 3;
u1 is 0, 1, 2, or 3;
u2 is 0, 1, 2, or 3;
v1 is 0, 1, 2, or 3;
v2 is 0, 1, 2, or 3;
w1 is 0, 1, 2, or 3; and
w2 is 0, 1, 2, or 3.

2. The compound of formula I according to claim 1, or a pharmaceutically acceptable salt thereof, wherein: R³ is 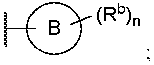 ring B, R^{b} and n are as defined in claim 1.

3. The compound of formula I according to claim 1 or 2, or a pharmaceutically acceptable salt thereof, wherein: Z is selected from -S-, -S(O)- and -S(O)₂-; preferably, Z is -S(O)-.

4. The compound of formula (I) according to any one of claims 1 to 3 or a pharmaceutically acceptable salt thereof, which is a compound of formula (II) or a pharmaceutically acceptable salt thereof: 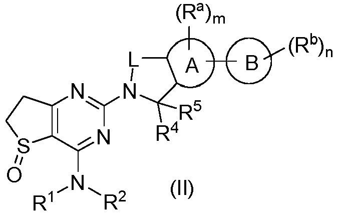 wherein: ring A, ring B, L, R^{a}, R^{b}, R¹, R², R⁴, R⁵, n and m are as defined in claim 1.

5. The compound of formula (I) according to any one of claims 1 to 4 or a pharmaceutically acceptable salt thereof, which is a compound of formula (II-1) or formula (II-2) or a pharmaceutically acceptable salt thereof: 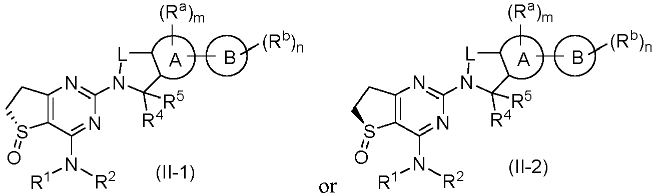 wherein: ring A, ring B, L, R^{a}, R^{b}, R¹, R², R⁴, R⁵, n and m are as defined in claim 1.

6. The compound of formula (I) according to any one of claims 1 to 4 or a pharmaceutically acceptable salt thereof, which is a compound of formula (III) or a pharmaceutically acceptable salt thereof: 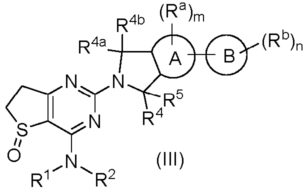 wherein: ring A, ring B, R^{a}, R^{b}, R¹, R², R⁴, R⁵, R^{4a}, R^{4b}, n and m are as defined in claim 1.

7. The compound of formula (I) according to any one of claims 1 to 6 or a pharmaceutically acceptable salt thereof, which is a compound of formula (III-1) or formula (III-2) or a pharmaceutically acceptable salt thereof: 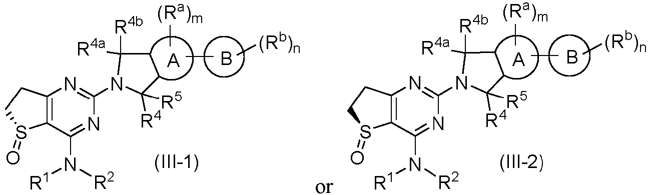 wherein: ring A, ring B, R^{a}, R^{b}, R¹, R², R⁴, R⁵, R^{4a}, R^{4b}, n and m are as defined in claim 1.

8. The compound of formula (I) according to any one of claims 1 to 4 or a pharmaceutically acceptable salt thereof, which is a compound of formula (IV) or a pharmaceutically acceptable salt thereof: 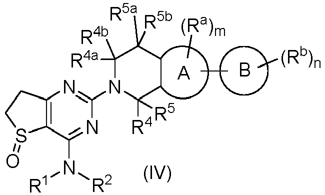 wherein: ring A, ring B, R^{a}, R^{b}, R¹, R², R⁴, R⁵, R^{4a}, R^{4b}, R^{5a}, R^{5b}, n and m are as defined in claim 1.

9. The compound of formula (I) according to any one of claims 1 to 5 and 8 or a pharmaceutically acceptable salt thereof, which is a compound of formula (IV-1) or formula (IV-2) or a pharmaceutically acceptable salt thereof: 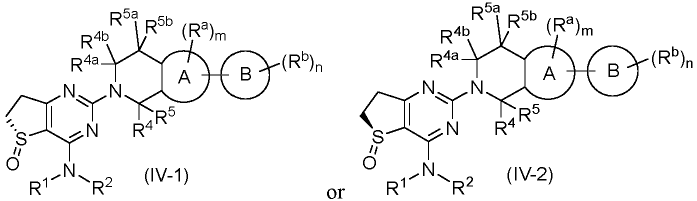 wherein: ring A, ring B, R^{a}, R^{b}, R¹, R², R⁴, R⁵, R^{4a}, R^{4b}, R^{5a}, R^{5b}, n and m are as defined in claim 1.

10. The compound of formula (I) according to any one of claims 1 to 9 or a pharmaceutically acceptable salt thereof, wherein: ring A is selected from 5- to 10-membered heteroaryl, 6- to 10-membered aryl, 3- to 8-membered heterocyclyl and 3- to 8-membered cycloalkyl; preferably, ring A is 5- to 6-membered heteroaryl or phenyl.

11. The compound of formula (I) according to any one of claims 1 to 10 or a pharmaceutically acceptable salt thereof, wherein: ring B is selected from 5- to 10-membered heteroaryl, 6- to 10-membered aryl, 3-to 8-membered heterocyclyl and 3- to 8-membered cycloalkyl; preferably, ring B is selected from 5- to 6-membered heteroaryl, phenyl, 3- to 6-membered heterocyclyl and 3- to 6-membered cycloalkyl.

12. The compound of formula (I) according to any one of claims 1 to 11 or a pharmaceutically acceptable salt thereof, wherein: n is 0, 1, 2 or 3; and each R^{b} is identical or different and is independently selected from deuterium atom, halogen, C₁₋₆ alkyl, cyano, C₁₋₆ alkoxy, amino, C₁₋₆ alkylamino, C₁₋₆ alkoxy-C₁₋₆ alkoxy, halogenated C₁₋₆ alkyl and halogenated C₁₋₆ alkoxy.

13. The compound of formula (I) according to any one of claims 1 to 12 or a pharmaceutically acceptable salt thereof, wherein: m is 0.

14. The compound of formula (I) according to any one of claims 1 to 13 or a pharmaceutically acceptable salt thereof, wherein: R¹ and R² are identical or different and each is independently selected from hydrogen atom, deuterium atom, C₁₋₆ alkyl, 5- to 10-membered heteroaryl, 6- to 10-membered aryl, 3-to 8-membered cycloalkyl and 3- to 8-membered heterocyclyl; wherein the C₁₋₆ alkyl, 5- to 10-membered heteroaryl, 6- to 10-membered aryl, 3- to 8-membered cycloalkyl and 3- to 8-membered heterocyclyl are each independently optionally substituted by one or more substituents selected from deuterium atom, halogen, oxo, C₁₋₆ alkyl, halogenated ₁₋₆ alkyl, hydroxyl group, C₁₋₆ alkoxy, -(CH₂)ᵤ₁NR^{d1}R^{d2}, - (CH₂)_{w1}C(O)OR^{c2}, -(CH₂)ᵤR, hydroxy- C₁₋₆ alkyl, 3- to 8-membered cycloalkyl, 3- to 8-membered heterocyclyl, 6- to 10-membered aryl and 5- to 10-membered heteroaryl; R^{c2}, R^{d1}, R^{d2}, R, u, u1 and w1 are as defined in claim 1.

15. The compound of formula (I) according to any one of claims 1 to 14 or a pharmaceutically acceptable salt thereof, wherein: R⁴, R^{4a}, R^{4b} and R⁵ are all hydrogen atoms.

16. The compound of formula (I) according to any one of claims 1 to 5, 8 to 15 or a pharmaceutically acceptable salt thereof, wherein: R^{5a} and R^{5b} are both hydrogen atoms.

17. The compound of formula (I) according to any one of claims 1 to 16 or a pharmaceutically acceptable salt thereof, which is selected from following compounds:

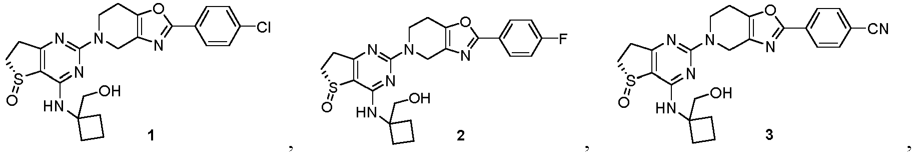

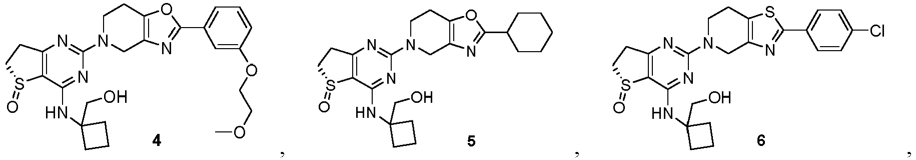

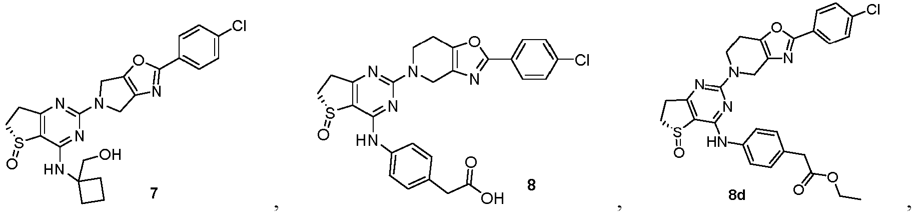

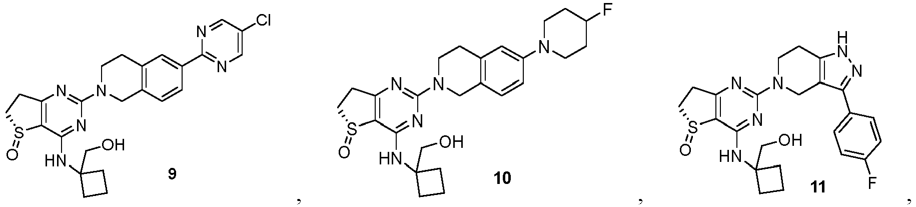

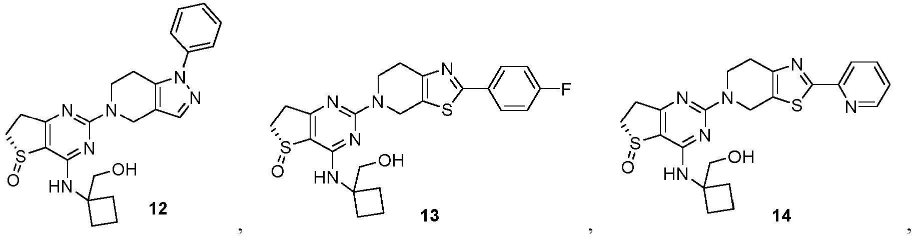

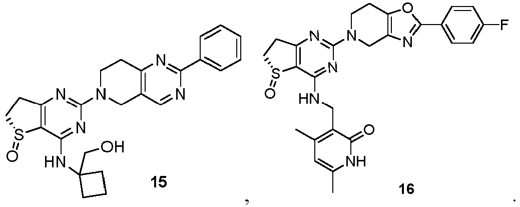

18. The compound of formula (I) according to any one of claims 1 to 16 or a pharmaceutically acceptable salt thereof, which is selected from following compounds:

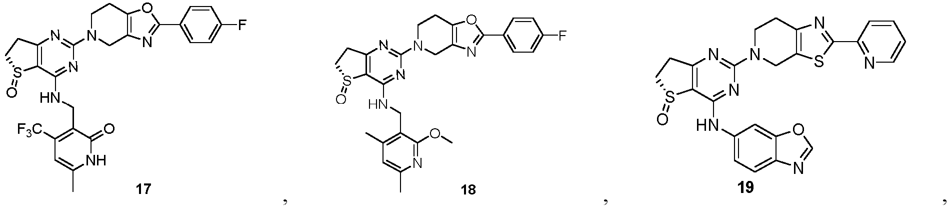

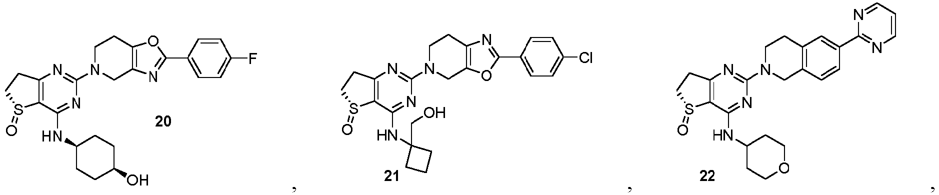

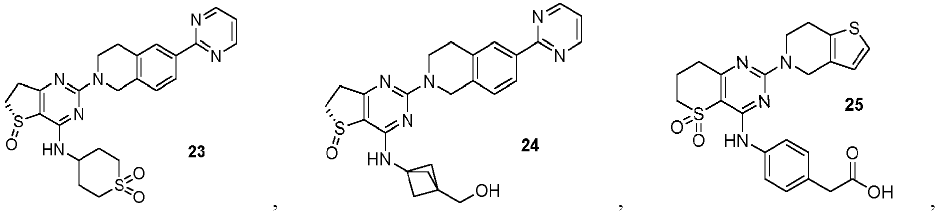

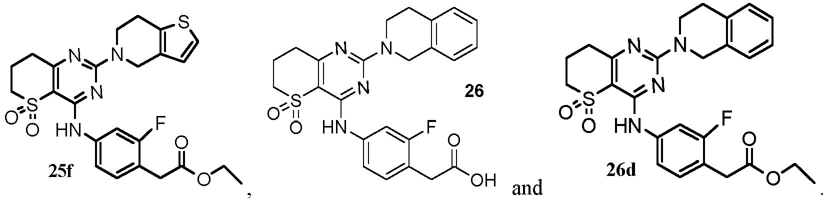

19. The compound selected from the following:

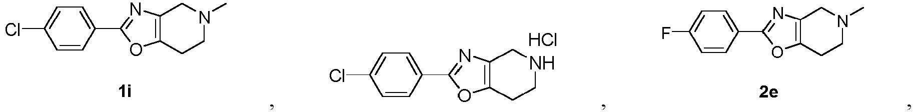

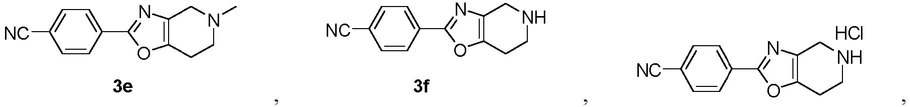

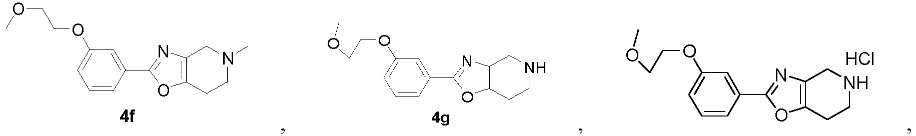

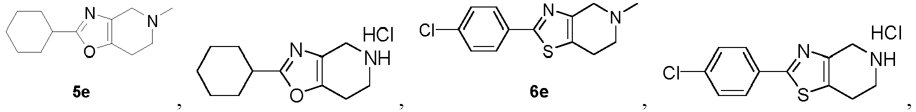

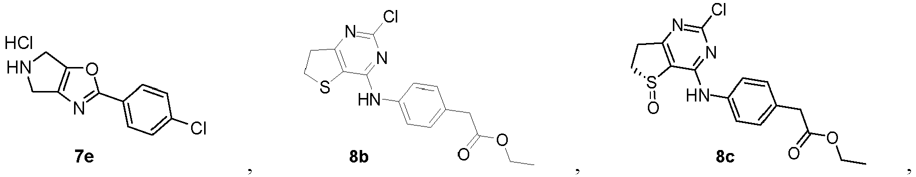

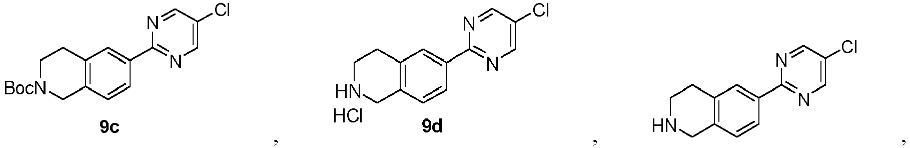

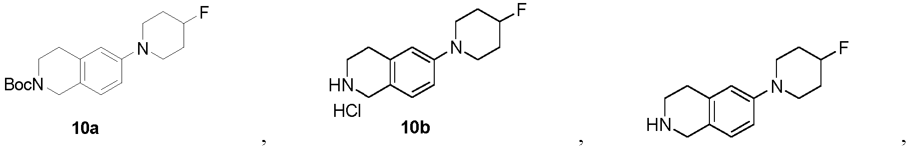

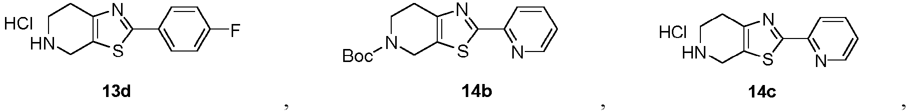

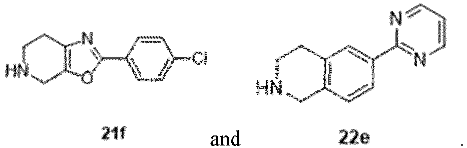

20. A method for preparing a compound of formula (I) or a pharmaceutically acceptable salt thereof, comprising following steps: 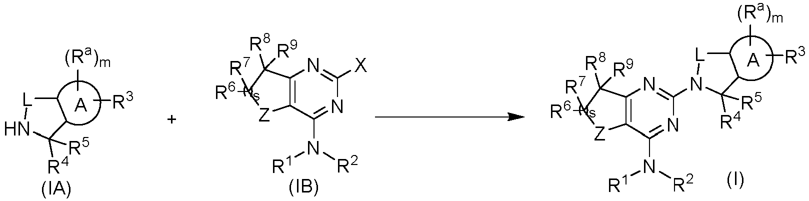
Subjecting a compound of formula (IA) or a salt thereof to a nucleophilic substitution reaction with a compound of formula (IB) or a salt thereof to obtain a compound of formula (I) or a pharmaceutically acceptable salt thereof;
wherein: X is a halogen, preferably Cl;
ring A, L, Z, R^{a}, R¹ to R⁹, m and s are as defined in claim 1.

21. A pharmaceutical composition comprising a therapeutically effective amount of the compound of Formula (I) according to any one of claims 1 to 18 or a pharmaceutically acceptable salt thereof, and one or more pharmaceutically acceptable carriers, diluents or excipients.

22. The compound of Formula (I) according to any one of claims 1 to 18 or a pharmaceutically acceptable salt thereof, or the pharmaceutical composition according to claim 20, for use in the preparation of a medicament for inhibiting PDEs enzymes, preferably PDE4 enzymes, more preferably PDE4B enzyme.

23. The compound of Formula (I) according to any one of claims 1 to 18 or a pharmaceutically acceptable salt thereof, or the pharmaceutical composition according to claim 20, for use in the preparation of a medicament for treating and/or preventing diseases or disorders mediated by PDEs enzymes, preferably PDE4 enzymes, more preferably PDE4B enzyme.

24. The use according to claim 23, wherein the disease or disorder is selected from respiratory diseases, pulmonary diseases, gastrointestinal diseases, inflammatory diseases, cancers and peripheral or central nervous system diseases.

25. The use according to claim 24, wherein the respiratory and pulmonary diseases are selected from respiratory and pulmonary diseases, obstructive pulmonary diseases and airway diseases, which are accompanied by increased mucus production, preferably selected from COPD, asthma, interstitial lung disease, pulmonary fibrosis, idiopathic pulmonary fibrosis, α1-antitrypsin deficiency, chronic sinusitis, chronic bronchitis and pulmonary arterial hypertension.

26. The use according to claim 24, wherein the gastrointestinal disease is selected from regional enteritis, ulcerative colitis and Crohn's disease.

27. The use according to claim 24, wherein the inflammatory disease is selected from hyperproliferative and inflammatory skin diseases, arthritic diseases and ocular inflammatory diseases; wherein the inflammatory skin diseases are preferably selected from atopic dermatitis, seborrheic dermatitis, contact dermatitis, epidermal inflammation, alopecia, alopecia areata, rosacea, SAPHO syndrome, skin atrophy, skin photoaging, acne vulgaris, hidradenitis suppurativa, urticaria, pruritus, eczema hand dermatitis and psoriasis, wherein the arthritic diseases are preferably selected from rheumatoid arthritis, psoriatic arthritis and spondyloarthritis, and the ocular inflammatory disease is preferably glaucoma or dry eye syndrome.

28. The use according to claim 24, wherein the peripheral or central nervous system disease is selected from Alzheimer's disease, age-associated memory impairment (AAMI), age-associated cognitive decline, vascular dementia, delirium, Parkinson's disease, Huntington's disease, Pick's disease, mental retardation, cerebrovascular diseases, depression, schizophrenia, stroke, neurasthenic disorders, attention deficit disorder, subdural hematoma, normal pressure hydrocephalus, brain tumors, cerebral apoplexy, cognitive impairment caused by sleep deprivation, intellectual and developmental disabilities and multiple sclerosis.

29. The use according to claim 24, wherein the cancer is selected from leukemia, lymphoma, macroglobulinemia, heavy chain disease, sarcoma, carcinoma, pancreatic cancer, breast cancer, ovarian cancer, prostate cancer, squamous cell carcinoma, sweat gland carcinoma, sebaceous gland carcinoma, papillary carcinoma, cystadenocarcinoma, medullary carcinoma, bronchogenic carcinoma, liver cancer, cholangiocarcinoma, choriocarcinoma, seminoma, embryonal carcinoma, Wilms' tumor, cervical cancer, endometrial cancer, testicular cancer, lung cancer, bladder cancer, glioma, medulloblastoma, craniopharyngioma, ependymoma, pinealoma, hemangioblastoma, acoustic neuroma, neurilemmoma, neurofibroma, retinoblastoma, melanoma, skin cancer, kidney cancer, nasopharyngeal cancer, stomach cancer, esophageal cancer, head and neck cancer, colorectal cancer, small intestine cancer, gallbladder cancer, pediatric tumors, urothelial cancer, ureteral tumors, thyroid cancer, osteoma, neuroblastoma, brain tumors and myeloma.
